(19) Europäisches Patentamt
European Patent Office
Office européen des brevets

(11) EP 4 067 481 A1

(12) EUROPEAN PATENT APPLICATION
published in accordance with Art. 153(4) EPC

(43) Date of publication:
**05.10.2022 Bulletin 2022/40**

(21) Application number: **20894278.9**

(22) Date of filing: **26.11.2020**

(51) International Patent Classification (IPC):
***C12N 5/0783*** *(2010.01)* ***C12N 15/63*** *(2006.01)*
***C07K 14/55*** *(2006.01)* ***C07K 14/705*** *(2006.01)*
***A61K 35/17*** *(2015.01)* ***A61P 35/00*** *(2006.01)*
***A61K 38/00*** *(2006.01)* ***A61K 38/20*** *(2006.01)*
***A61K 38/17*** *(2006.01)*

(52) Cooperative Patent Classification (CPC):
**A61K 35/17; A61K 38/00; A61K 38/17; A61K 38/20; A61P 35/00; C07K 14/55; C07K 14/705; C12N 5/06; C12N 15/63**

(86) International application number:
**PCT/KR2020/016949**

(87) International publication number:
**WO 2021/107635 (03.06.2021 Gazette 2021/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2019 KR 20190154631**
**10.02.2020 KR 20200015802**

(71) Applicant: **GI Cell, Inc.**
**Seongnam-si, Gyeonggi-do 13201 (KR)**

(72) Inventors:
- **JANG, Myoung Ho**
  **Seoul 05849 (KR)**
- **HONG, Chun-Pyo**
  **Seongnam-si, Gyeonggi-do 13643 (KR)**
- **YANG, Zung Yoon**
  **Incheon 22009 (KR)**
- **KOH, Young Jun**
  **Seoul 06800 (KR)**
- **LEE, June Sub**
  **Gwangju-si, Gyeonggi-do 12795 (KR)**
- **CHOI, Young Joo**
  **Hwaseong-si, Gyeonggi-do 18479 (KR)**

(74) Representative: **Mewburn Ellis LLP**
**Aurora Building**
**Counterslip**
**Bristol BS1 6BX (GB)**

(54) **COMPOSITION FOR ANTICANCER TREATMENT, COMPRISING NK CELLS AND FUSION PROTEIN WHICH COMPRISES IL-2 PROTEIN AND CD80 PROTEIN**

(57) Provided is an anticancer agent, comprising, as active ingredients, NK cells and a fusion protein which comprises an IL-2 protein and CD80 protein. In one specific embodiment, a fusion protein comprising a CD80 fragment, an immunoglobulin Fc and an IL-2 variant can activate immunocytes such as natural killer cells. In addition, since cancer can be effectively inhibited when coadministering with natural killer cells, the pharmaceutical composition increases the immune activity in the body so as to be effectively usable for cancer, there by having high industrial applicability.

FIG.29

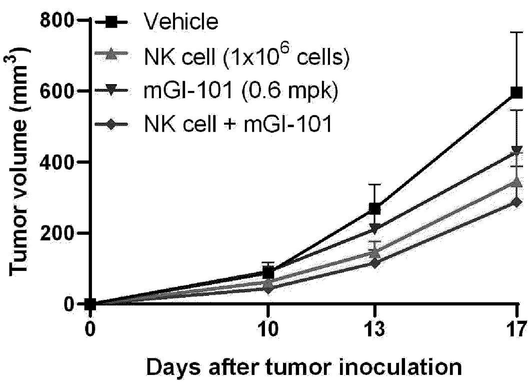


EP 4 067 481 A1

<110> GI Cell, Inc.

<120> PHARMACEUTICAL COMPOSITION FOR TREATING CANCER COMPRISING FUSION PROTEIN COMPRISING IL-2 PROTEIN AND CD80 PROTEIN AND NATURAL KILLER CELLS

<130> SPO20-034-PCT-GIC

<150> KR 10-2019-0154631
<151> 2019-11-27

<150> KR 10-2020-0015802
<151> 2020-02-10

<160> 47

<170> KopatentIn 3.0

<210> 1
<211> 25
<212> PRT
<213> Artificial Sequence

<220>
<223> signal peptide (TPA)

<400> 1
```
Met Asp Ala Met Leu Arg Gly Leu Cys Cys Val Leu Leu Leu Cys Gly
  1               5                  10                  15

Ala Val Phe Val Ser Pro Ser His Ala
             20                  25
```

<210> 2
<211> 208
<212> PRT
<213> Artificial Sequence

<220>
<223> hB7-1:35-242

<400> 2
```
Val Ile His Val Thr Lys Glu Val Lys Glu Val Ala Thr Leu Ser Cys
  1               5                  10                  15

Gly His Asn Val Ser Val Glu Glu Leu Ala Gln Thr Arg Ile Tyr Trp
             20                  25                  30

Gln Lys Glu Lys Lys Met Val Leu Thr Met Met Ser Gly Asp Met Asn
         35                  40                  45

Ile Trp Pro Glu Tyr Lys Asn Arg Thr Ile Phe Asp Ile Thr Asn Asn
     50                  55                  60

Leu Ser Ile Val Ile Leu Ala Leu Arg Pro Ser Asp Glu Gly Thr Tyr
 65                  70                  75                  80

Glu Cys Val Val Leu Lys Tyr Glu Lys Asp Ala Phe Lys Arg Glu His
                 85                  90                  95

Leu Ala Glu Val Thr Leu Ser Val Lys Ala Asp Phe Pro Thr Pro Ser
```

```
            100                 105                 110

Ile Ser Asp Phe Glu Ile Pro Thr Ser Asn Ile Arg Arg Ile Ile Cys
        115                 120                 125

Ser Thr Ser Gly Gly Phe Pro Glu Pro His Leu Ser Trp Leu Glu Asn
    130                 135                 140

Gly Glu Glu Leu Asn Ala Ile Asn Thr Thr Val Ser Gln Asp Pro Glu
145                 150                 155                 160

Thr Glu Leu Tyr Ala Val Ser Ser Lys Leu Asp Phe Asn Met Thr Thr
                165                 170                 175

Asn His Ser Phe Met Cys Leu Ile Lys Tyr Gly His Leu Arg Val Asn
            180                 185                 190

Gln Thr Phe Asn Trp Asn Thr Thr Lys Gln Glu His Phe Pro Asp Asn
        195                 200                 205



<210>  3
<211>  30
<212>  PRT
<213>  Artificial Sequence

<220>
<223>  hinge with linker


<400>  3
Gly Ser Gly Gly Gly Gly Ser Gly Gly Gly Gly Ser Gly Gly Gly Gly
1               5                   10                  15

Ser Ala Glu Ser Lys Tyr Gly Pro Pro Cys Pro Pro Cys Pro
            20                  25                  30


<210>  4
<211>  216
<212>  PRT
<213>  Artificial Sequence

<220>
<223>  immunoglobulin fc


<400>  4
Ala Pro Glu Ala Ala Gly Gly Pro Ser Val Phe Leu Phe Pro Pro Lys
1               5                   10                  15

Pro Lys Asp Gln Leu Met Ile Ser Arg Thr Pro Glu Val Thr Cys Val
            20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
```

65 70 75 80

Gln Asp Trp Leu Asn Gly Lys Glu Tyr Lys Cys Lys Val Ser Asn Lys
85 90 95

Gly Leu Pro Ser Ser Ile Glu Lys Thr Ile Ser Lys Ala Lys Gly Gln
100 105 110

Pro Arg Glu Pro Gln Val Tyr Thr Leu Pro Pro Ser Gln Glu Glu Met
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Arg Leu Thr Val Asp Lys Ser Arg Trp Gln Glu Gly Asn Val
180 185 190

Phe Ser Cys Ser Val Leu His Glu Ala Leu His Asn His Tyr Thr Gln
195 200 205

Lys Ser Leu Ser Leu Ser Leu Gly
210 215

<210> 5
<211> 5
<212> PRT
<213> Artificial Sequence

<220>
<223> linker

<400> 5
Gly Gly Gly Gly Ser
1 5

<210> 6
<211> 133
<212> PRT
<213> Artificial Sequence

<220>
<223> hIL-2M

<400> 6
Ala Pro Thr Ser Ser Ser Thr Lys Lys Thr Gln Leu Gln Leu Glu His
1 5 10 15

Leu Leu Leu Asp Leu Gln Met Ile Leu Asn Gly Ile Asn Asn Tyr Lys
20 25 30

Asn Pro Lys Leu Thr Ala Met Leu Thr Ala Lys Phe Tyr Met Pro Lys
35 40 45

```
Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Cys Gln Ser Ile
        115                 120                 125

Ile Ser Thr Leu Thr
    130


<210>   7
<211>   617
<212>   PRT
<213>   Artificial Sequence

<220>
<223>   fusion protein comprising variants of IL-2 and fragments of CD80


<400>   7
Met Asp Ala Met Leu Arg Gly Leu Cys Cys Val Leu Leu Leu Cys Gly
1               5                   10                  15

Ala Val Phe Val Ser Pro Ser His Ala Val Ile His Val Thr Lys Glu
            20                  25                  30

Val Lys Glu Val Ala Thr Leu Ser Cys Gly His Asn Val Ser Val Glu
        35                  40                  45

Glu Leu Ala Gln Thr Arg Ile Tyr Trp Gln Lys Glu Lys Lys Met Val
    50                  55                  60

Leu Thr Met Met Ser Gly Asp Met Asn Ile Trp Pro Glu Tyr Lys Asn
65                  70                  75                  80

Arg Thr Ile Phe Asp Ile Thr Asn Asn Leu Ser Ile Val Ile Leu Ala
                85                  90                  95

Leu Arg Pro Ser Asp Glu Gly Thr Tyr Glu Cys Val Val Leu Lys Tyr
            100                 105                 110

Glu Lys Asp Ala Phe Lys Arg Glu His Leu Ala Glu Val Thr Leu Ser
        115                 120                 125

Val Lys Ala Asp Phe Pro Thr Pro Ser Ile Ser Asp Phe Glu Ile Pro
    130                 135                 140

Thr Ser Asn Ile Arg Arg Ile Ile Cys Ser Thr Ser Gly Gly Phe Pro
145                 150                 155                 160

Glu Pro His Leu Ser Trp Leu Glu Asn Gly Glu Glu Leu Asn Ala Ile
                165                 170                 175

Asn Thr Thr Val Ser Gln Asp Pro Glu Thr Glu Leu Tyr Ala Val Ser
```

180 185 190

Ser Lys Leu Asp Phe Asn Met Thr Thr Asn His Ser Phe Met Cys Leu
195 200 205

Ile Lys Tyr Gly His Leu Arg Val Asn Gln Thr Phe Asn Trp Asn Thr
210 215 220

Thr Lys Gln Glu His Phe Pro Asp Asn Gly Ser Gly Gly Gly Gly Ser
225 230 235 240

Gly Gly Gly Gly Ser Gly Gly Gly Gly Ser Ala Glu Ser Lys Tyr Gly
245 250 255

Pro Pro Cys Pro Pro Cys Pro Ala Pro Glu Ala Ala Gly Gly Pro Ser
260 265 270

Val Phe Leu Phe Pro Pro Lys Pro Lys Asp Gln Leu Met Ile Ser Arg
275 280 285

Thr Pro Glu Val Thr Cys Val Val Val Asp Val Ser Gln Glu Asp Pro
290 295 300

Glu Val Gln Phe Asn Trp Tyr Val Asp Gly Val Glu Val His Asn Ala
305 310 315 320

Lys Thr Lys Pro Arg Glu Glu Gln Phe Asn Ser Thr Tyr Arg Val Val
325 330 335

Ser Val Leu Thr Val Leu His Gln Asp Trp Leu Asn Gly Lys Glu Tyr
340 345 350

Lys Cys Lys Val Ser Asn Lys Gly Leu Pro Ser Ser Ile Glu Lys Thr
355 360 365

Ile Ser Lys Ala Lys Gly Gln Pro Arg Glu Pro Gln Val Tyr Thr Leu
370 375 380

Pro Pro Ser Gln Glu Glu Met Thr Lys Asn Gln Val Ser Leu Thr Cys
385 390 395 400

Leu Val Lys Gly Phe Tyr Pro Ser Asp Ile Ala Val Glu Trp Glu Ser
405 410 415

Asn Gly Gln Pro Glu Asn Asn Tyr Lys Thr Thr Pro Pro Val Leu Asp
420 425 430

Ser Asp Gly Ser Phe Phe Leu Tyr Ser Arg Leu Thr Val Asp Lys Ser
435 440 445

Arg Trp Gln Glu Gly Asn Val Phe Ser Cys Ser Val Leu His Glu Ala
450 455 460

Leu His Asn His Tyr Thr Gln Lys Ser Leu Ser Leu Ser Leu Gly Gly
465 470 475 480

Gly Gly Gly Ser Ala Pro Thr Ser Ser Ser Thr Lys Lys Thr Gln Leu
485 490 495

Gln Leu Glu His Leu Leu Leu Asp Leu Gln Met Ile Leu Asn Gly Ile
500 505 510

Asn Asn Tyr Lys Asn Pro Lys Leu Thr Ala Met Leu Thr Ala Lys Phe

515 520 525

Tyr Met Pro Lys Lys Ala Thr Glu Leu Lys His Leu Gln Cys Leu Glu
530 535 540

Glu Glu Leu Lys Pro Leu Glu Glu Val Leu Asn Leu Ala Gln Ser Lys
545 550 555 560

Asn Phe His Leu Arg Pro Arg Asp Leu Ile Ser Asn Ile Asn Val Ile
565 570 575

Val Leu Glu Leu Lys Gly Ser Glu Thr Thr Phe Met Cys Glu Tyr Ala
580 585 590

Asp Glu Thr Ala Thr Ile Val Glu Phe Leu Asn Arg Trp Ile Thr Phe
595 600 605

Cys Gln Ser Ile Ile Ser Thr Leu Thr
610 615

<210> 8
<211> 1857
<212> DNA
<213> Artificial Sequence

<220>
<223> nucleotiedes coding fusion protein (GI101)

<400> 8
atggatgcta tgctgagagg cctgtgttgc gtgctgctgc tgtgtggcgc tgtgttcgtg 60

tctccttctc acgctgtgat ccacgtgacc aaagaagtga aagaggtcgc cacactgtcc 120

tgcggccaca acgtttcagt ggaagaactg gcccagacca ggatctactg gcagaaagaa 180

aagaaaatgg tgctgaccat gatgtccggc gacatgaaca tctggcctga gtacaagaac 240

cggaccatct tcgacatcac caacaacctg tccatcgtga ttctggccct gaggccttct 300

gatgagggca cctatgagtg cgtggtgctg aagtacgaga aggacgcctt caagcgcgag 360

cacctggctg aagtgacact gtccgtgaag gccgactttc ccacaccttc catctccgac 420

ttcgagatcc ctacctccaa catccggcgg atcatctgtt ctacctctgg cggctttcct 480

gagcctcacc tgtcttggct ggaaaacggc gaggaactga acgccatcaa caccaccgtg 540

tctcaggacc ccgaaaccga gctgtacgct gtgtcctcca agctggactt caacatgacc 600

accaaccaca gcttcatgtg cctgattaag tacggccacc tgagagtgaa ccagaccttc 660

aactggaaca ccaccaagca agagcacttc cctgacaatg gatctggcgg cggaggttct 720

ggcggaggtg gaagcggagg cggaggatct gctgagtcta agtatggccc tccttgtcct 780

ccatgtcctg ctccagaagc tgctggcgga ccctctgtgt tcctgtttcc tccaaagcct 840

aaggaccagc tcatgatctc tcggacaccc gaagtgacct gcgtggtggt ggatgtgtct 900

caagaggacc ctgaggtgca gttcaattgg tacgtggacg gcgtggaagt gcacaacgcc 960

aagaccaagc ctagagagga acagttcaac tccacctaca gagtggtgtc cgtgctgacc 1020

gtgctgcacc aggattggct gaacggcaaa gagtacaagt gcaaggtgtc caacaagggc 1080

ctgccttcca gcatcgaaaa gaccatctcc aaggctaagg gccagcctag ggaaccccag 1140

gtttacaccc tgcctccaag ccaagaggaa atgaccaaga accaggtgtc cctgacctgc 1200

ctggtcaagg gcttctaccc ttccgacatt gccgtggaat gggagtccaa tggccagcct 1260

gagaacaact acaagaccac acctcctgtg ctggactccg acggctcctt ctttctgtac 1320

tctcgcctga ccgtggacaa gtctagatgg caagagggca acgtgttctc ctgctctgtg 1380

ctgcacgagg ccctgcacaa tcactacacc cagaagtccc tgtctctgtc tcttggaggt 1440

ggtggcggtt ctgcccctac cagctcctct accaagaaaa cccagctcca gttggagcat 1500

ctgctgctgg acctccagat gattctgaac gggatcaaca actataagaa ccccaagctg 1560

accgccatgc tgaccgctaa gttctacatg cccaagaagg ccaccgagct gaagcacctc 1620

cagtgcctgg aagaagaact gaagcccctg gaagaggtgc tgaatctggc ccagtccaag 1680

aacttccacc tgaggccacg ggacctgatc agcaacatca acgtgatcgt gctggaactg 1740

aagggctccg agacaacctt tatgtgcgag tacgccgacg agacagccac catcgtggaa 1800

tttctgaacc ggtggatcac cttctgccag agcatcatct ccacactgac ctgatga 1857

<210> 9
<211> 592
<212> PRT
<213> Artificial Sequence

<220>
<223> fusion protein (GI101)

<400> 9

Val Ile His Val Thr Lys Glu Val Lys Glu Val Ala Thr Leu Ser Cys
1 5 10 15

Gly His Asn Val Ser Val Glu Glu Leu Ala Gln Thr Arg Ile Tyr Trp
20 25 30

Gln Lys Glu Lys Lys Met Val Leu Thr Met Met Ser Gly Asp Met Asn
35 40 45

Ile Trp Pro Glu Tyr Lys Asn Arg Thr Ile Phe Asp Ile Thr Asn Asn
50 55 60

Leu Ser Ile Val Ile Leu Ala Leu Arg Pro Ser Asp Glu Gly Thr Tyr
65 70 75 80

Glu Cys Val Val Leu Lys Tyr Glu Lys Asp Ala Phe Lys Arg Glu His
85 90 95

Leu Ala Glu Val Thr Leu Ser Val Lys Ala Asp Phe Pro Thr Pro Ser
100 105 110

Ile Ser Asp Phe Glu Ile Pro Thr Ser Asn Ile Arg Arg Ile Ile Cys

115 120 125

Ser Thr Ser Gly Gly Phe Pro Glu Pro His Leu Ser Trp Leu Glu Asn
130 135 140

Gly Glu Glu Leu Asn Ala Ile Asn Thr Thr Val Ser Gln Asp Pro Glu
145 150 155 160

Thr Glu Leu Tyr Ala Val Ser Ser Lys Leu Asp Phe Asn Met Thr Thr
165 170 175

Asn His Ser Phe Met Cys Leu Ile Lys Tyr Gly His Leu Arg Val Asn
180 185 190

Gln Thr Phe Asn Trp Asn Thr Thr Lys Gln Glu His Phe Pro Asp Asn
195 200 205

Gly Ser Gly Gly Gly Gly Ser Gly Gly Gly Gly Ser Gly Gly Gly Gly
210 215 220

Ser Ala Glu Ser Lys Tyr Gly Pro Pro Cys Pro Pro Cys Pro Ala Pro
225 230 235 240

Glu Ala Ala Gly Gly Pro Ser Val Phe Leu Phe Pro Pro Lys Pro Lys
245 250 255

Asp Gln Leu Met Ile Ser Arg Thr Pro Glu Val Thr Cys Val Val Val
260 265 270

Asp Val Ser Gln Glu Asp Pro Glu Val Gln Phe Asn Trp Tyr Val Asp
275 280 285

Gly Val Glu Val His Asn Ala Lys Thr Lys Pro Arg Glu Glu Gln Phe
290 295 300

Asn Ser Thr Tyr Arg Val Val Ser Val Leu Thr Val Leu His Gln Asp
305 310 315 320

Trp Leu Asn Gly Lys Glu Tyr Lys Cys Lys Val Ser Asn Lys Gly Leu
325 330 335

Pro Ser Ser Ile Glu Lys Thr Ile Ser Lys Ala Lys Gly Gln Pro Arg
340 345 350

Glu Pro Gln Val Tyr Thr Leu Pro Pro Ser Gln Glu Glu Met Thr Lys
355 360 365

Asn Gln Val Ser Leu Thr Cys Leu Val Lys Gly Phe Tyr Pro Ser Asp
370 375 380

Ile Ala Val Glu Trp Glu Ser Asn Gly Gln Pro Glu Asn Asn Tyr Lys
385 390 395 400

Thr Thr Pro Pro Val Leu Asp Ser Asp Gly Ser Phe Phe Leu Tyr Ser
405 410 415

Arg Leu Thr Val Asp Lys Ser Arg Trp Gln Glu Gly Asn Val Phe Ser
420 425 430

Cys Ser Val Leu His Glu Ala Leu His Asn His Tyr Thr Gln Lys Ser
435 440 445

Leu Ser Leu Ser Leu Gly Gly Gly Gly Gly Ser Ala Pro Thr Ser Ser

450 455 460

Ser Thr Lys Lys Thr Gln Leu Gln Leu Glu His Leu Leu Leu Asp Leu
465 470 475 480

Gln Met Ile Leu Asn Gly Ile Asn Asn Tyr Lys Asn Pro Lys Leu Thr
485 490 495

Ala Met Leu Thr Ala Lys Phe Tyr Met Pro Lys Lys Ala Thr Glu Leu
500 505 510

Lys His Leu Gln Cys Leu Glu Glu Glu Leu Lys Pro Leu Glu Glu Val
515 520 525

Leu Asn Leu Ala Gln Ser Lys Asn Phe His Leu Arg Pro Arg Asp Leu
530 535 540

Ile Ser Asn Ile Asn Val Ile Val Leu Glu Leu Lys Gly Ser Glu Thr
545 550 555 560

Thr Phe Met Cys Glu Tyr Ala Asp Glu Thr Ala Thr Ile Val Glu Phe
565 570 575

Leu Asn Arg Trp Ile Thr Phe Cys Gln Ser Ile Ile Ser Thr Leu Thr
580 585 590

<210> 10
<211> 133
<212> PRT
<213> Artificial Sequence

<220>
<223> hIL-2

<400> 10
Ala Pro Thr Ser Ser Ser Thr Lys Lys Thr Gln Leu Gln Leu Glu His
1 5 10 15

Leu Leu Leu Asp Leu Gln Met Ile Leu Asn Gly Ile Asn Asn Tyr Lys
20 25 30

Asn Pro Lys Leu Thr Arg Met Leu Thr Phe Lys Phe Tyr Met Pro Lys
35 40 45

Lys Ala Thr Glu Leu Lys His Leu Gln Cys Leu Glu Glu Glu Leu Lys
50 55 60

Pro Leu Glu Glu Val Leu Asn Leu Ala Gln Ser Lys Asn Phe His Leu
65 70 75 80

Arg Pro Arg Asp Leu Ile Ser Asn Ile Asn Val Ile Val Leu Glu Leu
85 90 95

Lys Gly Ser Glu Thr Thr Phe Met Cys Glu Tyr Ala Asp Glu Thr Ala
100 105 110

Thr Ile Val Glu Phe Leu Asn Arg Trp Ile Thr Phe Cys Gln Ser Ile
115 120 125

Ile Ser Thr Leu Thr
130

<210> 11
<211> 288
<212> PRT
<213> Artificial Sequence

<220>
<223> CD80

<400> 11
Met Gly His Thr Arg Arg Gln Gly Thr Ser Pro Ser Lys Cys Pro Tyr
1 5 10 15

Leu Asn Phe Phe Gln Leu Leu Val Leu Ala Gly Leu Ser His Phe Cys
20 25 30

Ser Gly Val Ile His Val Thr Lys Glu Val Lys Glu Val Ala Thr Leu
35 40 45

Ser Cys Gly His Asn Val Ser Val Glu Glu Leu Ala Gln Thr Arg Ile
50 55 60

Tyr Trp Gln Lys Glu Lys Lys Met Val Leu Thr Met Met Ser Gly Asp
65 70 75 80

Met Asn Ile Trp Pro Glu Tyr Lys Asn Arg Thr Ile Phe Asp Ile Thr
85 90 95

Asn Asn Leu Ser Ile Val Ile Leu Ala Leu Arg Pro Ser Asp Glu Gly
100 105 110

Thr Tyr Glu Cys Val Val Leu Lys Tyr Glu Lys Asp Ala Phe Lys Arg
115 120 125

Glu His Leu Ala Glu Val Thr Leu Ser Val Lys Ala Asp Phe Pro Thr
130 135 140

Pro Ser Ile Ser Asp Phe Glu Ile Pro Thr Ser Asn Ile Arg Arg Ile
145 150 155 160

Ile Cys Ser Thr Ser Gly Gly Phe Pro Glu Pro His Leu Ser Trp Leu
165 170 175

Glu Asn Gly Glu Glu Leu Asn Ala Ile Asn Thr Thr Val Ser Gln Asp
180 185 190

Pro Glu Thr Glu Leu Tyr Ala Val Ser Ser Lys Leu Asp Phe Asn Met
195 200 205

Thr Thr Asn His Ser Phe Met Cys Leu Ile Lys Tyr Gly His Leu Arg
210 215 220

Val Asn Gln Thr Phe Asn Trp Asn Thr Thr Lys Gln Glu His Phe Pro
225 230 235 240

Asp Asn Leu Leu Pro Ser Trp Ala Ile Thr Leu Ile Ser Val Asn Gly
245 250 255

Ile Phe Val Ile Cys Cys Leu Thr Tyr Cys Phe Ala Pro Arg Cys Arg

```
            260                 265                 270

Glu Arg Arg Arg Asn Glu Arg Leu Arg Arg Glu Ser Val Arg Pro Val
        275                 280                 285




<210>   12
<211>   215
<212>   PRT
<213>   Artificial Sequence

<220>
<223>   modified Fc


<400>   12
Ser His Thr Gln Pro Leu Gly Val Phe Leu Phe Pro Pro Lys Pro Lys
  1               5                  10                  15

Asp Thr Leu Met Ile Ser Arg Thr Pro Glu Val Thr Cys Val Val Val
            20                  25                  30

Asp Val Ser Gln Glu Asp Pro Glu Val Gln Phe Asn Trp Tyr Val Asp
        35                  40                  45

Gly Val Glu Val His Asn Ala Lys Thr Lys Pro Arg Glu Glu Gln Phe
    50                  55                  60

Asn Ser Thr Tyr Arg Val Val Ser Val Leu Thr Val Leu His Gln Asp
 65                  70                  75                  80

Trp Leu Asn Gly Lys Glu Tyr Lys Cys Lys Val Ser Asn Lys Gly Leu
                85                  90                  95

Pro Ser Ser Ile Glu Lys Thr Ile Ser Lys Ala Lys Gly Gln Pro Arg
            100                 105                 110

Glu Pro Gln Val Tyr Thr Leu Pro Pro Ser Gln Glu Glu Met Thr Lys
        115                 120                 125

Asn Gln Val Ser Leu Thr Cys Leu Val Lys Gly Phe Tyr Pro Ser Asp
    130                 135                 140

Ile Ala Val Glu Trp Glu Ser Asn Gly Gln Pro Glu Asn Asn Tyr Lys
145                 150                 155                 160

Thr Thr Pro Pro Val Leu Asp Ser Asp Gly Ser Phe Phe Leu Tyr Ser
                165                 170                 175

Arg Leu Thr Val Asp Lys Ser Arg Trp Gln Glu Gly Asn Val Phe Ser
            180                 185                 190

Cys Ser Val Met His Glu Ala Leu His Asn His Tyr Thr Gln Lys Ser
        195                 200                 205

Leu Ser Leu Ser Leu Gly Lys
    210                 215


<210>   13
<211>   306
```

<212> PRT
<213> Artificial Sequence

<220>
<223> mCD80

<400> 13
```
Met Ala Cys Asn Cys Gln Leu Met Gln Asp Thr Pro Leu Leu Lys Phe
1               5                   10                  15

Pro Cys Pro Arg Leu Ile Leu Leu Phe Val Leu Leu Ile Arg Leu Ser
            20                  25                  30

Gln Val Ser Ser Asp Val Asp Glu Gln Leu Ser Lys Ser Val Lys Asp
        35                  40                  45

Lys Val Leu Leu Pro Cys Arg Tyr Asn Ser Pro His Glu Asp Glu Ser
    50                  55                  60

Glu Asp Arg Ile Tyr Trp Gln Lys His Asp Lys Val Val Leu Ser Val
65                  70                  75                  80

Ile Ala Gly Lys Leu Lys Val Trp Pro Glu Tyr Lys Asn Arg Thr Leu
                85                  90                  95

Tyr Asp Asn Thr Thr Tyr Ser Leu Ile Ile Leu Gly Leu Val Leu Ser
            100                 105                 110

Asp Arg Gly Thr Tyr Ser Cys Val Val Gln Lys Lys Glu Arg Gly Thr
        115                 120                 125

Tyr Glu Val Lys His Leu Ala Leu Val Lys Leu Ser Ile Lys Ala Asp
    130                 135                 140

Phe Ser Thr Pro Asn Ile Thr Glu Ser Gly Asn Pro Ser Ala Asp Thr
145                 150                 155                 160

Lys Arg Ile Thr Cys Phe Ala Ser Gly Gly Phe Pro Lys Pro Arg Phe
                165                 170                 175

Ser Trp Leu Glu Asn Gly Arg Glu Leu Pro Gly Ile Asn Thr Thr Ile
            180                 185                 190

Ser Gln Asp Pro Glu Ser Glu Leu Tyr Thr Ile Ser Ser Gln Leu Asp
        195                 200                 205

Phe Asn Thr Thr Arg Asn His Thr Ile Lys Cys Leu Ile Lys Tyr Gly
    210                 215                 220

Asp Ala His Val Ser Glu Asp Phe Thr Trp Glu Lys Pro Pro Glu Asp
225                 230                 235                 240

Pro Pro Asp Ser Lys Asn Thr Leu Val Leu Phe Gly Ala Gly Phe Gly
                245                 250                 255

Ala Val Ile Thr Val Val Val Ile Val Val Ile Ile Lys Cys Phe Cys
            260                 265                 270

Lys His Arg Ser Cys Phe Arg Arg Asn Glu Ala Ser Arg Glu Thr Asn
        275                 280                 285

Asn Ser Leu Thr Phe Gly Pro Glu Glu Ala Leu Ala Glu Gln Thr Val
```

290 295 300

Phe Leu
305

<210> 14
<211> 1848
<212> DNA
<213> Artificial Sequence

<220>
<223> nucleotiedes coding fusion protein (mGI101)

<400> 14
atggatgcta tgctgagagg cctgtgttgc gtgctgctgc tgtgtggcgc tgtgttcgtg 60

tctccttctc acgctgtgga cgagcagctc tccaagtccg tgaaggataa ggtcctgctg 120

ccttgccggt acaactctcc tcacgaggac gagtctgagg accggatcta ctggcagaaa 180

cacgacaagg tggtgctgtc cgtgatcgcc ggaaagctga aagtgtggcc tgagtacaag 240

aacaggaccc tgtacgacaa caccacctac agcctgatca tcctgggcct cgtgctgagc 300

gatagaggca cctattcttg cgtggtgcag aagaaagagc ggggcaccta cgaagtgaag 360

cacctggctc tggtcaagct gtccatcaag gccgacttca gcacccctaa catcaccgag 420

tctggcaacc cttccgccga caccaagaga atcacctgtt tcgcctctgg cggcttccct 480

aagcctcggt tctcttggct ggaaaacggc agagagctgc ccggcatcaa taccaccatt 540

tctcaggacc cagagtccga gctgtacacc atctccagcc agctcgactt taacaccacc 600

agaaaccaca ccatcaagtg cctgattaag tacggcgacg cccacgtgtc cgaggacttt 660

acttgggaga aacctcctga ggaccctcct gactctggat ctggcggcgg aggttctggc 720

ggaggtggaa gcggaggcgg aggatctgct gagtctaagt atggccctcc ttgtcctcca 780

tgtcctgctc cagaagctgc tggcggaccc tctgtgttcc tgtttcctcc aaagcctaag 840

gaccagctca tgatctctcg gacccctgaa gtgacctgcg tggtggtgga tgtgtctcaa 900

gaggaccctg aggtgcagtt caattggtac gtggacggcg tggaagtgca caacgccaag 960

accaagccta gagaggaaca gttcaactcc acctatagag tggtgtccgt gctgaccgtg 1020

ctgcaccagg attggctgaa cggcaaagag tacaagtgca aggtgtccaa caagggcctg 1080

ccttccagca tcgaaaagac catcagcaag gctaagggcc agcctaggga accccaggtt 1140

tacaccctgc ctccaagcca agaggaaatg accaagaacc aggtgtccct gacctgcctg 1200

gtcaagggct tctacccttc cgacattgcc gtggaatggg agtccaatgg ccagcctgag 1260

aacaactaca agaccacacc tcctgtgctg gactccgacg gctccttctt tctgtactct 1320

cgcctgaccg tggacaagtc taggtggcaa gagggcaacg tgttctcctg ctctgtgctg 1380

cacgaggctc tgcacaacca ctacacccag aagtccctgt ctctgtctct tggaggtggt 1440

ggcggttctg cccctacctc cagctctacc aagaaaaccc agctccagtt ggagcatctg 1500

ctgctggacc tccagatgat cctgaatggc atcaacaatt acaagaaccc caagctgacc 1560

gccatgctga ccgctaagtt ctacatgccc aagaaggcca ccgagctgaa gcacttgcag 1620

tgcctggaag aggaactgaa gcccctggaa gaagtgctga atctggccca gtccaagaac 1680

ttccacctga ggcctaggga cctgatctcc aacatcaacg tgatcgtgct ggaactgaaa 1740

ggctccgaga caaccttcat gtgcgagtac gccgacgaga cagccaccat cgtggaattt 1800

ctgaaccggt ggatcacctt ctgccagagc atcatctcca cactgacc 1848

<210> 15
<211> 616
<212> PRT
<213> Artificial Sequence

<220>
<223> fusion protein (mGI101) with signal peptide

<400> 15
Met Asp Ala Met Leu Arg Gly Leu Cys Cys Val Leu Leu Leu Cys Gly
1 5 10 15

Ala Val Phe Val Ser Pro Ser His Ala Val Asp Glu Gln Leu Ser Lys
20 25 30

Ser Val Lys Asp Lys Val Leu Leu Pro Cys Arg Tyr Asn Ser Pro His
35 40 45

Glu Asp Glu Ser Glu Asp Arg Ile Tyr Trp Gln Lys His Asp Lys Val
50 55 60

Val Leu Ser Val Ile Ala Gly Lys Leu Lys Val Trp Pro Glu Tyr Lys
65 70 75 80

Asn Arg Thr Leu Tyr Asp Asn Thr Thr Tyr Ser Leu Ile Ile Leu Gly
85 90 95

Leu Val Leu Ser Asp Arg Gly Thr Tyr Ser Cys Val Val Gln Lys Lys
100 105 110

Glu Arg Gly Thr Tyr Glu Val Lys His Leu Ala Leu Val Lys Leu Ser
115 120 125

Ile Lys Ala Asp Phe Ser Thr Pro Asn Ile Thr Glu Ser Gly Asn Pro
130 135 140

Ser Ala Asp Thr Lys Arg Ile Thr Cys Phe Ala Ser Gly Gly Phe Pro
145 150 155 160

Lys Pro Arg Phe Ser Trp Leu Glu Asn Gly Arg Glu Leu Pro Gly Ile
165 170 175

Asn Thr Thr Ile Ser Gln Asp Pro Glu Ser Glu Leu Tyr Thr Ile Ser
180 185 190

Ser Gln Leu Asp Phe Asn Thr Thr Arg Asn His Thr Ile Lys Cys Leu

195 200 205

Ile Lys Tyr Gly Asp Ala His Val Ser Glu Asp Phe Thr Trp Glu Lys
210 215 220

Pro Pro Glu Asp Pro Pro Asp Ser Gly Ser Gly Gly Gly Gly Ser Gly
225 230 235 240

Gly Gly Gly Ser Gly Gly Gly Gly Ser Ala Glu Ser Lys Tyr Gly Pro
245 250 255

Pro Cys Pro Pro Cys Pro Ala Pro Glu Ala Ala Gly Gly Pro Ser Val
260 265 270

Phe Leu Phe Pro Pro Lys Pro Lys Asp Gln Leu Met Ile Ser Arg Thr
275 280 285

Pro Glu Val Thr Cys Val Val Val Asp Val Ser Gln Glu Asp Pro Glu
290 295 300

Val Gln Phe Asn Trp Tyr Val Asp Gly Val Glu Val His Asn Ala Lys
305 310 315 320

Thr Lys Pro Arg Glu Glu Gln Phe Asn Ser Thr Tyr Arg Val Val Ser
325 330 335

Val Leu Thr Val Leu His Gln Asp Trp Leu Asn Gly Lys Glu Tyr Lys
340 345 350

Cys Lys Val Ser Asn Lys Gly Leu Pro Ser Ser Ile Glu Lys Thr Ile
355 360 365

Ser Lys Ala Lys Gly Gln Pro Arg Glu Pro Gln Val Tyr Thr Leu Pro
370 375 380

Pro Ser Gln Glu Glu Met Thr Lys Asn Gln Val Ser Leu Thr Cys Leu
385 390 395 400

Val Lys Gly Phe Tyr Pro Ser Asp Ile Ala Val Glu Trp Glu Ser Asn
405 410 415

Gly Gln Pro Glu Asn Asn Tyr Lys Thr Thr Pro Pro Val Leu Asp Ser
420 425 430

Asp Gly Ser Phe Phe Leu Tyr Ser Arg Leu Thr Val Asp Lys Ser Arg
435 440 445

Trp Gln Glu Gly Asn Val Phe Ser Cys Ser Val Leu His Glu Ala Leu
450 455 460

His Asn His Tyr Thr Gln Lys Ser Leu Ser Leu Ser Leu Gly Gly Gly
465 470 475 480

Gly Gly Ser Ala Pro Thr Ser Ser Ser Thr Lys Lys Thr Gln Leu Gln
485 490 495

Leu Glu His Leu Leu Leu Asp Leu Gln Met Ile Leu Asn Gly Ile Asn
500 505 510

Asn Tyr Lys Asn Pro Lys Leu Thr Ala Met Leu Thr Ala Lys Phe Tyr
515 520 525

Met Pro Lys Lys Ala Thr Glu Leu Lys His Leu Gln Cys Leu Glu Glu

530 535 540

Glu Leu Lys Pro Leu Glu Glu Val Leu Asn Leu Ala Gln Ser Lys Asn
545 550 555 560

Phe His Leu Arg Pro Arg Asp Leu Ile Ser Asn Ile Asn Val Ile Val
565 570 575

Leu Glu Leu Lys Gly Ser Glu Thr Thr Phe Met Cys Glu Tyr Ala Asp
580 585 590

Glu Thr Ala Thr Ile Val Glu Phe Leu Asn Arg Trp Ile Thr Phe Cys
595 600 605

Gln Ser Ile Ile Ser Thr Leu Thr
610 615

<210> 16
<211> 1437
<212> DNA
<213> Artificial Sequence

<220>
<223> nucleotiedes coding fusion protein (GI101C1)

<400> 16
atggatgcta tgctgagagg cctgtgttgc gtgctgctgc tgtgtggcgc tgtgttcgtg 60

tctccttctc acgctgtgat ccacgtgacc aaagaagtga aagaggtcgc cacactgtcc 120

tgcggccaca acgtttcagt ggaagaactg gcccagacca ggatctactg gcagaaagaa 180

aagaaaatgg tgctgaccat gatgtccggc gacatgaaca tctggcctga gtacaagaac 240

cggaccatct tcgacatcac caacaacctg tccatcgtga ttctggccct gaggccttct 300

gatgagggca cctatgagtg cgtggtgctg aagtacgaga aggacgcctt caagcgcgag 360

cacctggctg aagtgacact gtccgtgaag gccgactttc ccacaccttc catctccgac 420

ttcgagatcc ctacctccaa catccggcgg atcatctgtt ctacctctgg cggctttcct 480

gagcctcacc tgtcttggct ggaaaacggc gaggaactga acgccatcaa caccaccgtg 540

tctcaggacc ccgaaaccga gctgtacgct gtgtcctcca agctggactt caacatgacc 600

accaaccaca gcttcatgtg cctgattaag tacggccacc tgagagtgaa ccagaccttc 660

aactggaaca ccaccaagca agagcacttc cctgacaatg gatctggcgg cggaggttct 720

ggcggaggtg gaagcggagg cggaggatct gctgagtcta agtatggccc tccttgtcct 780

ccatgtcctg ctccagaagc tgctggcgga ccctctgtgt tcctgtttcc tccaaagcct 840

aaggaccagc tcatgatctc tcggacaccc gaagtgacct gcgtggtggt ggatgtgtct 900

caagaggacc ctgaggtgca gttcaattgg tacgtggacg gcgtggaagt gcacaacgcc 960

aagaccaagc ctagagagga acagttcaac tccacctaca gagtggtgtc cgtgctgacc 1020

gtgctgcacc aggattggct gaacggcaaa gagtacaagt gcaaggtgtc caacaagggc 1080

ctgccttcca gcatcgaaaa gaccatctcc aaggctaagg gccagcctag ggaaccccag 1140

gtttacaccc tgcctccaag ccaagaggaa atgaccaaga accaggtgtc cctgacctgc 1200

ctggtcaagg gcttctaccc ttccgacatt gccgtggaat gggagtccaa tggccagcct 1260

gagaacaact acaagaccac acctcctgtg ctggactccg acggctcctt ctttctgtac 1320

tctcgcctga ccgtggacaa gtctaggtgg caagagggca acgtgttctc ctgctctgtg 1380

ctgcacgagg ccctgcacaa tcactacacc cagaagtccc tgtctctgtc cctgggc 1437

<210> 17
<211> 454
<212> PRT
<213> Artificial Sequence

<220>
<223> fusion protein (GI101C1)

<400> 17

```
Val Ile His Val Thr Lys Glu Val Lys Glu Val Ala Thr Leu Ser Cys
1               5                   10                  15

Gly His Asn Val Ser Val Glu Glu Leu Ala Gln Thr Arg Ile Tyr Trp
            20                  25                  30

Gln Lys Glu Lys Lys Met Val Leu Thr Met Met Ser Gly Asp Met Asn
        35                  40                  45

Ile Trp Pro Glu Tyr Lys Asn Arg Thr Ile Phe Asp Ile Thr Asn Asn
    50                  55                  60

Leu Ser Ile Val Ile Leu Ala Leu Arg Pro Ser Asp Glu Gly Thr Tyr
65                  70                  75                  80

Glu Cys Val Val Leu Lys Tyr Glu Lys Asp Ala Phe Lys Arg Glu His
                85                  90                  95

Leu Ala Glu Val Thr Leu Ser Val Lys Ala Asp Phe Pro Thr Pro Ser
            100                 105                 110

Ile Ser Asp Phe Glu Ile Pro Thr Ser Asn Ile Arg Arg Ile Ile Cys
        115                 120                 125

Ser Thr Ser Gly Gly Phe Pro Glu Pro His Leu Ser Trp Leu Glu Asn
    130                 135                 140

Gly Glu Glu Leu Asn Ala Ile Asn Thr Thr Val Ser Gln Asp Pro Glu
145                 150                 155                 160

Thr Glu Leu Tyr Ala Val Ser Ser Lys Leu Asp Phe Asn Met Thr Thr
                165                 170                 175

Asn His Ser Phe Met Cys Leu Ile Lys Tyr Gly His Leu Arg Val Asn
            180                 185                 190

Gln Thr Phe Asn Trp Asn Thr Thr Lys Gln Glu His Phe Pro Asp Asn
        195                 200                 205
```

Gly Ser Gly Gly Gly Gly Ser Gly Gly Gly Gly Ser Gly Gly Gly Gly
    210                 215                 220

Ser Ala Glu Ser Lys Tyr Gly Pro Pro Cys Pro Pro Cys Pro Ala Pro
225                 230                 235                 240

Glu Ala Ala Gly Gly Pro Ser Val Phe Leu Phe Pro Pro Lys Pro Lys
                245                 250                 255

Asp Gln Leu Met Ile Ser Arg Thr Pro Glu Val Thr Cys Val Val Val
            260                 265                 270

Asp Val Ser Gln Glu Asp Pro Glu Val Gln Phe Asn Trp Tyr Val Asp
        275                 280                 285

Gly Val Glu Val His Asn Ala Lys Thr Lys Pro Arg Glu Glu Gln Phe
    290                 295                 300

Asn Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Gly Leu
                325                 330                 335

Pro Ser Ser Ile Glu Lys Thr Ile Ser Lys Ala Lys Gly Gln Pro Arg
            340                 345                 350

Glu Pro Gln Val Tyr Thr Leu Pro Pro Ser Gln Glu Glu Met Thr Lys
        355                 360                 365

Asn Gln Val Ser Leu Thr Cys Leu Val Lys Gly Phe Tyr Pro Ser Asp
    370                 375                 380

Ile Ala Val Glu Trp Glu Ser Asn Gly Gln Pro Glu Asn Asn Tyr Lys
385                 390                 395                 400

Thr Thr Pro Pro Val Leu Asp Ser Asp Gly Ser Phe Phe Leu Tyr Ser
                405                 410                 415

Arg Leu Thr Val Asp Lys Ser Arg Trp Gln Glu Gly Asn Val Phe Ser
            420                 425                 430

Cys Ser Val Leu His Glu Ala Leu His Asn His Tyr Thr Gln Lys Ser
        435                 440                 445

Leu Ser Leu Ser Leu Gly
    450

<210> 18
<211> 1176
<212> DNA
<213> Artificial Sequence

<220>
<223> nucleotiedes coding fusion protein (GI101C2)

<400> 18
atggatgcta tgctgagagg cctgtgttgc gtgctgctgc tgtgtggcgc tgtgttcgtg 60

tctccatctc acgccgctga gtctaagtac ggccctcctt gtcctccatg tcctgctcca 120

```
gaagctgctg gcggaccctc tgtgttcctg tttcctccaa agcctaagga ccagctcatg        180
atctctcgga cccctgaagt gacctgcgtg gtggtggatg tgtctcaaga ggaccctgag        240
gtgcagttca attggtacgt ggacggcgtg gaagtgcaca acgccaagac caagcctaga        300
gaggaacagt tcaactccac ctacagagtg gtgtccgtgc tgaccgtgct gcaccaggat        360
tggctgaacg gcaaagagta caagtgcaag gtgtccaaca agggcctgcc ttccagcatc        420
gaaaagacca tctccaaggc taagggccag cctagggaac cccaggttta caccctgcct        480
ccaagccaag aggaaatgac caagaaccag gtgtccctga cctgcctggt caagggcttc        540
tacccttccg acattgccgt ggaatgggag tccaatggcc agcctgagaa caactacaag        600
accacacctc ctgtgctgga ctccgacggc tccttctttc tgtactctcg cctgaccgtg        660
gacaagtcta ggtggcaaga gggcaacgtg ttctcctgct ctgtgctgca cgaggccctg        720
cacaatcact acacccagaa gtccctgtct ctgtctcttg gcggaggcgg aggatctgct        780
cctacctcca gctccaccaa gaaaacccag ctccagttgg agcatctgct gctggacctc        840
cagatgatcc tgaatggcat caacaattac aagaacccca agctgaccgc catgctgacc        900
gctaagttct acatgcccaa gaaggccacc gagctgaagc acctccagtg cctggaagag        960
gaactgaagc ccctggaaga agtgctgaat ctggcccagt ccaagaactt ccacctgagg       1020
cctagggacc tgatctccaa catcaacgtg atcgtgctgg aactgaaagg ctccgagaca       1080
accttcatgt gcgagtacgc cgacgagaca gccaccatcg tggaatttct gaaccggtgg       1140
atcaccttct gccagtccat catctccaca ctgacc                                 1176


<210>   19
<211>   367
<212>   PRT
<213>   Artificial Sequence

<220>
<223>   fusion protein (GI101C2)


<400>   19
Ala Glu Ser Lys Tyr Gly Pro Pro Cys Pro Pro Cys Pro Ala Pro Glu
1               5                   10                  15

Ala Ala Gly Gly Pro Ser Val Phe Leu Phe Pro Pro Lys Pro Lys Asp
            20                  25                  30

Gln Leu Met Ile Ser Arg Thr Pro Glu Val Thr Cys Val Val Val Asp
        35                  40                  45

Val Ser Gln Glu Asp Pro Glu Val Gln Phe Asn Trp Tyr Val Asp Gly
    50                  55                  60

Val Glu Val His Asn Ala Lys Thr Lys Pro Arg Glu Glu Gln Phe Asn
65                  70                  75                  80

Ser Thr Tyr Arg Val Val Ser Val Leu Thr Val Leu His Gln Asp Trp
```

85 90 95

Leu Asn Gly Lys Glu Tyr Lys Cys Lys Val Ser Asn Lys Gly Leu Pro
100 105 110

Ser Ser Ile Glu Lys Thr Ile Ser Lys Ala Lys Gly Gln Pro Arg Glu
115 120 125

Pro Gln Val Tyr Thr Leu Pro Pro Ser Gln Glu Glu Met Thr Lys Asn
130 135 140

Gln Val Ser Leu Thr Cys Leu Val Lys Gly Phe Tyr Pro Ser Asp Ile
145 150 155 160

Ala Val Glu Trp Glu Ser Asn Gly Gln Pro Glu Asn Asn Tyr Lys Thr
165 170 175

Thr Pro Pro Val Leu Asp Ser Asp Gly Ser Phe Phe Leu Tyr Ser Arg
180 185 190

Leu Thr Val Asp Lys Ser Arg Trp Gln Glu Gly Asn Val Phe Ser Cys
195 200 205

Ser Val Leu His Glu Ala Leu His Asn His Tyr Thr Gln Lys Ser Leu
210 215 220

Ser Leu Ser Leu Gly Gly Gly Gly Gly Ser Ala Pro Thr Ser Ser Ser
225 230 235 240

Thr Lys Lys Thr Gln Leu Gln Leu Glu His Leu Leu Leu Asp Leu Gln
245 250 255

Met Ile Leu Asn Gly Ile Asn Asn Tyr Lys Asn Pro Lys Leu Thr Ala
260 265 270

Met Leu Thr Ala Lys Phe Tyr Met Pro Lys Lys Ala Thr Glu Leu Lys
275 280 285

His Leu Gln Cys Leu Glu Glu Glu Leu Lys Pro Leu Glu Glu Val Leu
290 295 300

Asn Leu Ala Gln Ser Lys Asn Phe His Leu Arg Pro Arg Asp Leu Ile
305 310 315 320

Ser Asn Ile Asn Val Ile Val Leu Glu Leu Lys Gly Ser Glu Thr Thr
325 330 335

Phe Met Cys Glu Tyr Ala Asp Glu Thr Ala Thr Ile Val Glu Phe Leu
340 345 350

Asn Arg Trp Ile Thr Phe Cys Gln Ser Ile Ile Ser Thr Leu Thr
355 360 365

<210> 20
<211> 1434
<212> DNA
<213> Artificial Sequence

<220>
<223> nucleotiedes coding fusion protein (mGI101C1)

<400> 20
atggatgcta tgctgagagg cctgtgttgc gtgctgctgc tgtgtggcgc tgtgttcgtg 60

tctccttctc acgctgtgga cgagcagctc tccaagtccg tgaaggataa ggtcctgctg 120

ccttgccggt acaactctcc tcacgaggac gagtctgagg accggatcta ctggcagaaa 180

cacgacaagg tggtgctgtc cgtgatcgcc ggaaagctga aagtgtggcc tgagtacaag 240

aacaggaccc tgtacgacaa caccacctac agcctgatca tcctgggcct cgtgctgagc 300

gatagaggca cctattcttg cgtggtgcag aagaaagagc ggggcaccta cgaagtgaag 360

cacctggctc tggtcaagct gtccatcaag gccgacttca gcacccctaa catcaccgag 420

tctggcaacc cttccgccga caccaagaga atcacctgtt tcgcctctgg cggcttccct 480

aagcctcggt tctcttggct ggaaaacggc agagagctgc ccggcatcaa taccaccatt 540

tctcaggacc cagagtccga gctgtacacc atctccagcc agctcgactt taacaccacc 600

agaaaccaca ccatcaagtg cctgattaag tacggcgacg cccacgtgtc cgaggacttt 660

acttgggaga aacctcctga ggaccctcct gactctggat ctggcggcgg aggttctggc 720

ggaggtggaa gcggaggcgg aggatctgct gagtctaagt atggccctcc ttgtcctcca 780

tgtcctgctc cagaagctgc tggcggaccc tctgtgttcc tgtttcctcc aaagcctaag 840

gaccagctca tgatctctcg gacccctgaa gtgacctgcg tggtggtgga tgtgtctcaa 900

gaggaccctg aggtgcagtt caattggtac gtggacggcg tggaagtgca caacgccaag 960

accaagccta gagaggaaca gttcaactcc acctatagag tggtgtccgt gctgaccgtg 1020

ctgcaccagg attggctgaa cggcaaagag tacaagtgca aggtgtccaa caagggcctg 1080

ccttccagca tcgaaaagac catcagcaag gctaagggcc agcctaggga accccaggtt 1140

tacaccctgc ctccaagcca agaggaaatg accaagaacc aggtgtccct gacctgcctg 1200

gtcaagggct tctacccttc cgacattgcc gtggaatggg agtccaatgg ccagcctgag 1260

aacaactaca agaccacacc tcctgtgctg gactccgacg gctccttctt tctgtactct 1320

cgcctgaccg tggacaagtc taggtggcaa gagggcaacg tgttctcctg ctctgtgctg 1380

cacgaggctc tgcacaacca ctacacccag aagtccctgt ctctgtccct gggc 1434

<210> 21
<211> 478
<212> PRT
<213> Artificial Sequence

<220>
<223> fusion protein (mGI101C1)

<400> 21
Met Asp Ala Met Leu Arg Gly Leu Cys Cys Val Leu Leu Leu Cys Gly

1 5 10 15

Ala Val Phe Val Ser Pro Ser His Ala Val Asp Glu Gln Leu Ser Lys
20 25 30

Ser Val Lys Asp Lys Val Leu Leu Pro Cys Arg Tyr Asn Ser Pro His
35 40 45

Glu Asp Glu Ser Glu Asp Arg Ile Tyr Trp Gln Lys His Asp Lys Val
50 55 60

Val Leu Ser Val Ile Ala Gly Lys Leu Lys Val Trp Pro Glu Tyr Lys
65 70 75 80

Asn Arg Thr Leu Tyr Asp Asn Thr Thr Tyr Ser Leu Ile Ile Leu Gly
85 90 95

Leu Val Leu Ser Asp Arg Gly Thr Tyr Ser Cys Val Val Gln Lys Lys
100 105 110

Glu Arg Gly Thr Tyr Glu Val Lys His Leu Ala Leu Val Lys Leu Ser
115 120 125

Ile Lys Ala Asp Phe Ser Thr Pro Asn Ile Thr Glu Ser Gly Asn Pro
130 135 140

Ser Ala Asp Thr Lys Arg Ile Thr Cys Phe Ala Ser Gly Gly Phe Pro
145 150 155 160

Lys Pro Arg Phe Ser Trp Leu Glu Asn Gly Arg Glu Leu Pro Gly Ile
165 170 175

Asn Thr Thr Ile Ser Gln Asp Pro Glu Ser Glu Leu Tyr Thr Ile Ser
180 185 190

Ser Gln Leu Asp Phe Asn Thr Thr Arg Asn His Thr Ile Lys Cys Leu
195 200 205

Ile Lys Tyr Gly Asp Ala His Val Ser Glu Asp Phe Thr Trp Glu Lys
210 215 220

Pro Pro Glu Asp Pro Pro Asp Ser Gly Ser Gly Gly Gly Gly Ser Gly
225 230 235 240

Gly Gly Gly Ser Gly Gly Gly Gly Ser Ala Glu Ser Lys Tyr Gly Pro
245 250 255

Pro Cys Pro Pro Cys Pro Ala Pro Glu Ala Ala Gly Gly Pro Ser Val
260 265 270

Phe Leu Phe Pro Pro Lys Pro Lys Asp Gln Leu Met Ile Ser Arg Thr
275 280 285

Pro Glu Val Thr Cys Val Val Val Asp Val Ser Gln Glu Asp Pro Glu
290 295 300

Val Gln Phe Asn Trp Tyr Val Asp Gly Val Glu Val His Asn Ala Lys
305 310 315 320

Thr Lys Pro Arg Glu Glu Gln Phe Asn Ser Thr Tyr Arg Val Val Ser
325 330 335

Val Leu Thr Val Leu His Gln Asp Trp Leu Asn Gly Lys Glu Tyr Lys

340 345 350

Cys Lys Val Ser Asn Lys Gly Leu Pro Ser Ser Ile Glu Lys Thr Ile
355 360 365

Ser Lys Ala Lys Gly Gln Pro Arg Glu Pro Gln Val Tyr Thr Leu Pro
370 375 380

Pro Ser Gln Glu Glu Met Thr Lys Asn Gln Val Ser Leu Thr Cys Leu
385 390 395 400

Val Lys Gly Phe Tyr Pro Ser Asp Ile Ala Val Glu Trp Glu Ser Asn
405 410 415

Gly Gln Pro Glu Asn Asn Tyr Lys Thr Thr Pro Pro Val Leu Asp Ser
420 425 430

Asp Gly Ser Phe Phe Leu Tyr Ser Arg Leu Thr Val Asp Lys Ser Arg
435 440 445

Trp Gln Glu Gly Asn Val Phe Ser Cys Ser Val Leu His Glu Ala Leu
450 455 460

His Asn His Tyr Thr Gln Lys Ser Leu Ser Leu Ser Leu Gly
465 470 475

<210> 22
<211> 133
<212> PRT
<213> Artificial Sequence

<220>
<223> variants of IL-2 (3M, M45)

<400> 22
Ala Pro Thr Ser Ser Ser Thr Lys Lys Thr Gln Leu Gln Leu Glu His
1 5 10 15

Leu Leu Leu Asp Leu Gln Met Ile Leu Asn Gly Ile Asn Asn Tyr Lys
20 25 30

Asn Pro Lys Leu Thr Ala Met Leu Thr Ala Lys Phe Ala Met Pro Lys
35 40 45

Lys Ala Thr Glu Leu Lys His Leu Gln Cys Leu Glu Glu Glu Leu Lys
50 55 60

Pro Leu Glu Glu Val Leu Asn Leu Ala Gln Ser Lys Asn Phe His Leu
65 70 75 80

Arg Pro Arg Asp Leu Ile Ser Asn Ile Asn Val Ile Val Leu Glu Leu
85 90 95

Lys Gly Ser Glu Thr Thr Phe Met Cys Glu Tyr Ala Asp Glu Thr Ala
100 105 110

Thr Ile Val Glu Phe Leu Asn Arg Trp Ile Thr Phe Cys Gln Ser Ile
115 120 125

Ile Ser Thr Leu Thr
130

<210> 23
<211> 133
<212> PRT
<213> Artificial Sequence

<220>
<223> variants of IL-2 (3M, M61)

<400> 23
Ala Pro Thr Ser Ser Ser Thr Lys Lys Thr Gln Leu Gln Leu Glu His
1 5 10 15

Leu Leu Leu Asp Leu Gln Met Ile Leu Asn Gly Ile Asn Asn Tyr Lys
20 25 30

Asn Pro Lys Leu Thr Ala Met Leu Thr Ala Lys Phe Tyr Met Pro Lys
35 40 45

Lys Ala Thr Glu Leu Lys His Leu Gln Cys Leu Glu Arg Glu Leu Lys
50 55 60

Pro Leu Glu Glu Val Leu Asn Leu Ala Gln Ser Lys Asn Phe His Leu
65 70 75 80

Arg Pro Arg Asp Leu Ile Ser Asn Ile Asn Val Ile Val Leu Glu Leu
85 90 95

Lys Gly Ser Glu Thr Thr Phe Met Cys Glu Tyr Ala Asp Glu Thr Ala
100 105 110

Thr Ile Val Glu Phe Leu Asn Arg Trp Ile Thr Phe Cys Gln Ser Ile
115 120 125

Ile Ser Thr Leu Thr
130

<210> 24
<211> 133
<212> PRT
<213> Artificial Sequence

<220>
<223> variants of IL-2 (3M, M72)

<400> 24
Ala Pro Thr Ser Ser Ser Thr Lys Lys Thr Gln Leu Gln Leu Glu His
1 5 10 15

Leu Leu Leu Asp Leu Gln Met Ile Leu Asn Gly Ile Asn Asn Tyr Lys
20 25 30

Asn Pro Lys Leu Thr Ala Met Leu Thr Ala Lys Phe Tyr Met Pro Lys
35 40 45

Lys Ala Thr Glu Leu Lys His Leu Gln Cys Leu Glu Glu Glu Leu Lys
50 55 60

Pro Leu Glu Glu Val Leu Asn Gly Ala Gln Ser Lys Asn Phe His Leu

65 70 75 80

Arg Pro Arg Asp Leu Ile Ser Asn Ile Asn Val Ile Val Leu Glu Leu
85 90 95

Lys Gly Ser Glu Thr Thr Phe Met Cys Glu Tyr Ala Asp Glu Thr Ala
100 105 110

Thr Ile Val Glu Phe Leu Asn Arg Trp Ile Thr Phe Cys Gln Ser Ile
115 120 125

Ile Ser Thr Leu Thr
130

<210> 25
<211> 1851
<212> DNA
<213> Artificial Sequence

<220>
<223> nucleotiedes coding fusion protein (GI102-M45)

<400> 25
atggatgcta tgctgagagg cctgtgttgc gtgctgctgc tgtgtggcgc tgtgttcgtg 60

tctccttctc acgctgtgat ccacgtgacc aaagaagtga aagaggtcgc cacactgtcc 120

tgcggccaca acgtttcagt ggaagaactg gcccagacca ggatctactg gcagaaagaa 180

aagaaaatgg tgctgaccat gatgtccggc gacatgaaca tctggcctga gtacaagaac 240

cggaccatct tcgacatcac caacaacctg tccatcgtga ttctggccct gaggccttct 300

gatgagggca cctatgagtg cgtggtgctg aagtacgaga aggacgcctt caagcgcgag 360

cacctggctg aagtgacact gtccgtgaag gccgactttc ccacaccttc catctccgac 420

ttcgagatcc ctacctccaa catccggcgg atcatctgtt ctacctctgg cggctttcct 480

gagcctcacc tgtcttggct ggaaaacggc gaggaactga acgccatcaa caccaccgtg 540

tctcaggacc ccgaaaccga gctgtacgct gtgtcctcca agctggactt caacatgacc 600

accaaccaca gcttcatgtg cctgattaag tacggccacc tgagagtgaa ccagaccttc 660

aactggaaca ccaccaagca agagcacttc cctgacaatg gatctggcgg cggaggttct 720

ggcggaggtg gaagcggagg cggaggatct gctgagtcta agtatggccc tccttgtcct 780

ccatgtcctg ctccagaagc tgctggcgga ccctctgtgt tcctgtttcc tccaaagcct 840

aaggaccagc tcatgatctc tcggacaccc gaagtgacct gcgtggtggt ggatgtgtct 900

caagaggacc ctgaggtgca gttcaattgg tacgtggacg gcgtggaagt gcacaacgcc 960

aagaccaagc ctagagagga acagttcaac tccacctaca gagtggtgtc cgtgctgacc 1020

gtgctgcacc aggattggct gaacggcaaa gagtacaagt gcaaggtgtc caacaagggc 1080

ctgccttcca gcatcgaaaa gaccatctcc aaggctaagg gccagcctag ggaaccccag 1140

gtttacaccc tgcctccaag ccaagaggaa atgaccaaga accaggtgtc cctgacctgc 1200

ctggtcaagg gcttctaccc ttccgacatt gccgtggaat gggagtccaa tggccagcct 1260

gagaacaact acaagaccac acctcctgtg ctggactccg acggctcctt ctttctgtac 1320

tctcgcctga ccgtggacaa gtctagatgg caagagggca acgtgttctc ctgctctgtg 1380

ctgcacgagg ccctgcacaa tcactacacc cagaagtccc tgtctctgtc tcttggaggt 1440

ggtggcggtt ctgcccctac cagctcctct accaagaaaa cccagctcca gttggagcat 1500

ctgctgctgg acctccagat gattctgaac gggatcaaca actataagaa ccccaagctg 1560

accgccatgc tgaccgctaa gttcgccatg cccaagaagg ccaccgagct gaagcacctc 1620

cagtgcctgg aagaagaact gaagcccctg gaagaggtgc tgaatctggc ccagtccaag 1680

aacttccacc tgaggccacg ggacctgatc agcaacatca acgtgatcgt gctggaactg 1740

aagggctccg agacaacctt tatgtgcgag tacgccgacg agacagccac catcgtggaa 1800

tttctgaacc ggtggatcac cttctgccag agcatcatct ccacactgac c 1851

<210> 26
<211> 592
<212> PRT
<213> Artificial Sequence

<220>
<223> fusion protein (GI102-M45)

<400> 26

Val Ile His Val Thr Lys Glu Val Lys Glu Val Ala Thr Leu Ser Cys
1 5 10 15

Gly His Asn Val Ser Val Glu Glu Leu Ala Gln Thr Arg Ile Tyr Trp
20 25 30

Gln Lys Glu Lys Lys Met Val Leu Thr Met Met Ser Gly Asp Met Asn
35 40 45

Ile Trp Pro Glu Tyr Lys Asn Arg Thr Ile Phe Asp Ile Thr Asn Asn
50 55 60

Leu Ser Ile Val Ile Leu Ala Leu Arg Pro Ser Asp Glu Gly Thr Tyr
65 70 75 80

Glu Cys Val Val Leu Lys Tyr Glu Lys Asp Ala Phe Lys Arg Glu His
85 90 95

Leu Ala Glu Val Thr Leu Ser Val Lys Ala Asp Phe Pro Thr Pro Ser
100 105 110

Ile Ser Asp Phe Glu Ile Pro Thr Ser Asn Ile Arg Arg Ile Ile Cys
115 120 125

Ser Thr Ser Gly Gly Phe Pro Glu Pro His Leu Ser Trp Leu Glu Asn
130 135 140

Gly Glu Glu Leu Asn Ala Ile Asn Thr Thr Val Ser Gln Asp Pro Glu

145 150 155 160

Thr Glu Leu Tyr Ala Val Ser Ser Lys Leu Asp Phe Asn Met Thr Thr
165 170 175

Asn His Ser Phe Met Cys Leu Ile Lys Tyr Gly His Leu Arg Val Asn
180 185 190

Gln Thr Phe Asn Trp Asn Thr Thr Lys Gln Glu His Phe Pro Asp Asn
195 200 205

Gly Ser Gly Gly Gly Gly Ser Gly Gly Gly Gly Ser Gly Gly Gly Gly
210 215 220

Ser Ala Glu Ser Lys Tyr Gly Pro Pro Cys Pro Pro Cys Pro Ala Pro
225 230 235 240

Glu Ala Ala Gly Gly Pro Ser Val Phe Leu Phe Pro Pro Lys Pro Lys
245 250 255

Asp Gln Leu Met Ile Ser Arg Thr Pro Glu Val Thr Cys Val Val Val
260 265 270

Asp Val Ser Gln Glu Asp Pro Glu Val Gln Phe Asn Trp Tyr Val Asp
275 280 285

Gly Val Glu Val His Asn Ala Lys Thr Lys Pro Arg Glu Glu Gln Phe
290 295 300

Asn Ser Thr Tyr Arg Val Val Ser Val Leu Thr Val Leu His Gln Asp
305 310 315 320

Trp Leu Asn Gly Lys Glu Tyr Lys Cys Lys Val Ser Asn Lys Gly Leu
325 330 335

Pro Ser Ser Ile Glu Lys Thr Ile Ser Lys Ala Lys Gly Gln Pro Arg
340 345 350

Glu Pro Gln Val Tyr Thr Leu Pro Pro Ser Gln Glu Glu Met Thr Lys
355 360 365

Asn Gln Val Ser Leu Thr Cys Leu Val Lys Gly Phe Tyr Pro Ser Asp
370 375 380

Ile Ala Val Glu Trp Glu Ser Asn Gly Gln Pro Glu Asn Asn Tyr Lys
385 390 395 400

Thr Thr Pro Pro Val Leu Asp Ser Asp Gly Ser Phe Phe Leu Tyr Ser
405 410 415

Arg Leu Thr Val Asp Lys Ser Arg Trp Gln Glu Gly Asn Val Phe Ser
420 425 430

Cys Ser Val Leu His Glu Ala Leu His Asn His Tyr Thr Gln Lys Ser
435 440 445

Leu Ser Leu Ser Leu Gly Gly Gly Gly Gly Ser Ala Pro Thr Ser Ser
450 455 460

Ser Thr Lys Lys Thr Gln Leu Gln Leu Glu His Leu Leu Leu Asp Leu
465 470 475 480

Gln Met Ile Leu Asn Gly Ile Asn Asn Tyr Lys Asn Pro Lys Leu Thr

485 490 495

Ala Met Leu Thr Ala Lys Phe Ala Met Pro Lys Lys Ala Thr Glu Leu
500 505 510

Lys His Leu Gln Cys Leu Glu Glu Glu Leu Lys Pro Leu Glu Glu Val
515 520 525

Leu Asn Leu Ala Gln Ser Lys Asn Phe His Leu Arg Pro Arg Asp Leu
530 535 540

Ile Ser Asn Ile Asn Val Ile Val Leu Glu Leu Lys Gly Ser Glu Thr
545 550 555 560

Thr Phe Met Cys Glu Tyr Ala Asp Glu Thr Ala Thr Ile Val Glu Phe
565 570 575

Leu Asn Arg Trp Ile Thr Phe Cys Gln Ser Ile Ile Ser Thr Leu Thr
580 585 590

<210> 27
<211> 1851
<212> DNA
<213> Artificial Sequence

<220>
<223> nucleotiedes coding fusion protein (GI102-M61)

<400> 27
atggatgcta tgctgagagg cctgtgttgc gtgctgctgc tgtgtggcgc tgtgttcgtg 60

tctccttctc acgctgtgat ccacgtgacc aaagaagtga aagaggtcgc cacactgtcc 120

tgcggccaca acgtttcagt ggaagaactg gcccagacca ggatctactg gcagaaagaa 180

aagaaaatgg tgctgaccat gatgtccggc gacatgaaca tctggcctga gtacaagaac 240

cggaccatct tcgacatcac caacaacctg tccatcgtga ttctggccct gaggccttct 300

gatgagggca cctatgagtg cgtggtgctg aagtacgaga aggacgcctt caagcgcgag 360

cacctggctg aagtgacact gtccgtgaag gccgactttc ccacaccttc catctccgac 420

ttcgagatcc ctacctccaa catccggcgg atcatctgtt ctacctctgg cggctttcct 480

gagcctcacc tgtcttggct ggaaaacggc gaggaactga acgccatcaa caccaccgtg 540

tctcaggacc ccgaaaccga gctgtacgct gtgtcctcca agctggactt caacatgacc 600

accaaccaca gcttcatgtg cctgattaag tacggccacc tgagagtgaa ccagaccttc 660

aactggaaca ccaccaagca agagcacttc cctgacaatg gatctggcgg cggaggttct 720

ggcggaggtg gaagcggagg cggaggatct gctgagtcta agtatggccc tccttgtcct 780

ccatgtcctg ctccagaagc tgctggcgga ccctctgtgt tcctgtttcc tccaaagcct 840

aaggaccagc tcatgatctc tcggacaccc gaagtgacct gcgtggtggt ggatgtgtct 900

caagaggacc ctgaggtgca gttcaattgg tacgtggacg gcgtggaagt gcacaacgcc 960

aagaccaagc ctagagagga acagttcaac tccacctaca gagtggtgtc cgtgctgacc 1020

gtgctgcacc aggattggct gaacggcaaa gagtacaagt gcaaggtgtc caacaagggc 1080

ctgccttcca gcatcgaaaa gaccatctcc aaggctaagg gccagcctag ggaaccccag 1140

gtttacaccc tgcctccaag ccaagaggaa atgaccaaga accaggtgtc cctgacctgc 1200

ctggtcaagg gcttctaccc ttccgacatt gccgtggaat gggagtccaa tggccagcct 1260

gagaacaact acaagaccac acctcctgtg ctggactccg acggctcctt ctttctgtac 1320

tctcgcctga ccgtggacaa gtctagatgg caagagggca acgtgttctc ctgctctgtg 1380

ctgcacgagg ccctgcacaa tcactacacc cagaagtccc tgtctctgtc tcttggaggt 1440

ggtggcggtt ctgcccctac cagctcctct accaagaaaa cccagctcca gttggagcat 1500

ctgctgctgg acctccagat gattctgaac gggatcaaca actataagaa ccccaagctg 1560

accgccatgc tgaccgctaa gttctacatg cccaagaagg ccaccgagct gaagcacctc 1620

cagtgcctgg aaagggaact gaagcccctg gaagaggtgc tgaatctggc ccagtccaag 1680

aacttccacc tgaggccacg ggacctgatc agcaacatca acgtgatcgt gctggaactg 1740

aagggctccg agacaacctt tatgtgcgag tacgccgacg agacagccac catcgtggaa 1800

tttctgaacc ggtggatcac cttctgccag agcatcatct ccacactgac c 1851

<210> 28
<211> 592
<212> PRT
<213> Artificial Sequence

<220>
<223> fusion protein (GI102-M61)

<400> 28

```
Val Ile His Val Thr Lys Glu Val Lys Glu Val Ala Thr Leu Ser Cys
1               5                   10                  15

Gly His Asn Val Ser Val Glu Glu Leu Ala Gln Thr Arg Ile Tyr Trp
            20                  25                  30

Gln Lys Glu Lys Lys Met Val Leu Thr Met Met Ser Gly Asp Met Asn
        35                  40                  45

Ile Trp Pro Glu Tyr Lys Asn Arg Thr Ile Phe Asp Ile Thr Asn Asn
    50                  55                  60

Leu Ser Ile Val Ile Leu Ala Leu Arg Pro Ser Asp Glu Gly Thr Tyr
65                  70                  75                  80

Glu Cys Val Val Leu Lys Tyr Glu Lys Asp Ala Phe Lys Arg Glu His
                85                  90                  95

Leu Ala Glu Val Thr Leu Ser Val Lys Ala Asp Phe Pro Thr Pro Ser
            100                 105                 110
```

Ile Ser Asp Phe Glu Ile Pro Thr Ser Asn Ile Arg Arg Ile Ile Cys
115 120 125

Ser Thr Ser Gly Gly Phe Pro Glu Pro His Leu Ser Trp Leu Glu Asn
130 135 140

Gly Glu Glu Leu Asn Ala Ile Asn Thr Thr Val Ser Gln Asp Pro Glu
145 150 155 160

Thr Glu Leu Tyr Ala Val Ser Ser Lys Leu Asp Phe Asn Met Thr Thr
165 170 175

Asn His Ser Phe Met Cys Leu Ile Lys Tyr Gly His Leu Arg Val Asn
180 185 190

Gln Thr Phe Asn Trp Asn Thr Thr Lys Gln Glu His Phe Pro Asp Asn
195 200 205

Gly Ser Gly Gly Gly Gly Ser Gly Gly Gly Gly Ser Gly Gly Gly Gly
210 215 220

Ser Ala Glu Ser Lys Tyr Gly Pro Pro Cys Pro Pro Cys Pro Ala Pro
225 230 235 240

Glu Ala Ala Gly Gly Pro Ser Val Phe Leu Phe Pro Pro Lys Pro Lys
245 250 255

Asp Gln Leu Met Ile Ser Arg Thr Pro Glu Val Thr Cys Val Val Val
260 265 270

Asp Val Ser Gln Glu Asp Pro Glu Val Gln Phe Asn Trp Tyr Val Asp
275 280 285

Gly Val Glu Val His Asn Ala Lys Thr Lys Pro Arg Glu Glu Gln Phe
290 295 300

Asn Ser Thr Tyr Arg Val Val Ser Val Leu Thr Val Leu His Gln Asp
305 310 315 320

Trp Leu Asn Gly Lys Glu Tyr Lys Cys Lys Val Ser Asn Lys Gly Leu
325 330 335

Pro Ser Ser Ile Glu Lys Thr Ile Ser Lys Ala Lys Gly Gln Pro Arg
340 345 350

Glu Pro Gln Val Tyr Thr Leu Pro Pro Ser Gln Glu Glu Met Thr Lys
355 360 365

Asn Gln Val Ser Leu Thr Cys Leu Val Lys Gly Phe Tyr Pro Ser Asp
370 375 380

Ile Ala Val Glu Trp Glu Ser Asn Gly Gln Pro Glu Asn Asn Tyr Lys
385 390 395 400

Thr Thr Pro Pro Val Leu Asp Ser Asp Gly Ser Phe Phe Leu Tyr Ser
405 410 415

Arg Leu Thr Val Asp Lys Ser Arg Trp Gln Glu Gly Asn Val Phe Ser
420 425 430

Cys Ser Val Leu His Glu Ala Leu His Asn His Tyr Thr Gln Lys Ser
435 440 445

Leu Ser Leu Ser Leu Gly Gly Gly Gly Gly Ser Ala Pro Thr Ser Ser
450 455 460

Ser Thr Lys Lys Thr Gln Leu Gln Leu Glu His Leu Leu Leu Asp Leu
465 470 475 480

Gln Met Ile Leu Asn Gly Ile Asn Asn Tyr Lys Asn Pro Lys Leu Thr
485 490 495

Ala Met Leu Thr Ala Lys Phe Tyr Met Pro Lys Lys Ala Thr Glu Leu
500 505 510

Lys His Leu Gln Cys Leu Glu Arg Glu Leu Lys Pro Leu Glu Glu Val
515 520 525

Leu Asn Leu Ala Gln Ser Lys Asn Phe His Leu Arg Pro Arg Asp Leu
530 535 540

Ile Ser Asn Ile Asn Val Ile Val Leu Glu Leu Lys Gly Ser Glu Thr
545 550 555 560

Thr Phe Met Cys Glu Tyr Ala Asp Glu Thr Ala Thr Ile Val Glu Phe
565 570 575

Leu Asn Arg Trp Ile Thr Phe Cys Gln Ser Ile Ile Ser Thr Leu Thr
580 585 590

<210> 29
<211> 1857
<212> DNA
<213> Artificial Sequence

<220>
<223> nucleotiedes coding fusion protein (GI102-M72)

<400> 29
atggatgcta tgctgagagg cctgtgttgc gtgctgctgc tgtgtggcgc tgtgttcgtg 60

tctccttctc acgctgtgat ccacgtgacc aaagaagtga aagaggtcgc cacactgtcc 120

tgcggccaca acgtttcagt ggaagaactg gcccagacca ggatctactg gcagaaagaa 180

aagaaaatgg tgctgaccat gatgtccggc gacatgaaca tctggcctga gtacaagaac 240

cggaccatct tcgacatcac caacaacctg tccatcgtga ttctggccct gaggccttct 300

gatgagggca cctatgagtg cgtggtgctg aagtacgaga aggacgcctt caagcgcgag 360

cacctggctg aagtgacact gtccgtgaag gccgactttc ccacaccttc catctccgac 420

ttcgagatcc ctacctccaa catccggcgg atcatctgtt ctacctctgg cggctttcct 480

gagcctcacc tgtcttggct ggaaaacggc gaggaactga acgccatcaa caccaccgtg 540

tctcaggacc ccgaaaccga gctgtacgct gtgtcctcca agctggactt caacatgacc 600

accaaccaca gcttcatgtg cctgattaag tacggccacc tgagagtgaa ccagaccttc 660

aactggaaca ccaccaagca agagcacttc cctgacaatg gatctggcgg cggaggttct 720
ggcggaggtg gaagcggagg cggaggatct gctgagtcta agtatggccc tccttgtcct 780
ccatgtcctg ctccagaagc tgctggcgga ccctctgtgt tcctgtttcc tccaaagcct 840
aaggaccagc tcatgatctc tcggacaccc gaagtgacct gcgtggtggt ggatgtgtct 900
caagaggacc ctgaggtgca gttcaattgg tacgtggacg gcgtggaagt gcacaacgcc 960
aagaccaagc ctagagagga acagttcaac tccacctaca gagtggtgtc cgtgctgacc 1020
gtgctgcacc aggattggct gaacggcaaa gagtacaagt gcaaggtgtc caacaagggc 1080
ctgccttcca gcatcgaaaa gaccatctcc aaggctaagg gccagcctag ggaaccccag 1140
gtttacaccc tgcctccaag ccaagaggaa atgaccaaga accaggtgtc cctgacctgc 1200
ctggtcaagg gcttctaccc ttccgacatt gccgtggaat gggagtccaa tggccagcct 1260
gagaacaact acaagaccac acctcctgtg ctggactccg acggctcctt ctttctgtac 1320
tctcgcctga ccgtggacaa gtctagatgg caagagggca acgtgttctc ctgctctgtg 1380
ctgcacgagg ccctgcacaa tcactacacc cagaagtccc tgtctctgtc tcttggaggt 1440
ggtggcggtt ctgcccctac cagctcctct accaagaaaa cccagctcca gttggagcat 1500
ctgctgctgg acctccagat gattctgaac gggatcaaca actataagaa ccccaagctg 1560
accgccatgc tgaccgctaa gttctacatg cccaagaagg ccaccgagct gaagcacctc 1620
cagtgcctgg aagaagaact gaagcccctg gaagaggtgc tgaatggggc ccagtccaag 1680
aacttccacc tgaggccacg ggacctgatc agcaacatca acgtgatcgt gctggaactg 1740
aagggctccg agacaacctt tatgtgcgag tacgccgacg agacagccac catcgtggaa 1800
tttctgaacc ggtggatcac cttctgccag agcatcatct ccacactgac ctgatga 1857

<210> 30
<211> 592
<212> PRT
<213> Artificial Sequence

<220>
<223> fusion protein (GI102-M72)

<400> 30
Val Ile His Val Thr Lys Glu Val Lys Glu Val Ala Thr Leu Ser Cys
1 5 10 15

Gly His Asn Val Ser Val Glu Glu Leu Ala Gln Thr Arg Ile Tyr Trp
20 25 30

Gln Lys Glu Lys Lys Met Val Leu Thr Met Met Ser Gly Asp Met Asn
35 40 45

Ile Trp Pro Glu Tyr Lys Asn Arg Thr Ile Phe Asp Ile Thr Asn Asn
50 55 60

```
Leu Ser Ile Val Ile Leu Ala Leu Arg Pro Ser Asp Glu Gly Thr Tyr
 65                 70                  75                  80

Glu Cys Val Val Leu Lys Tyr Glu Lys Asp Ala Phe Lys Arg Glu His
                85                  90                  95

Leu Ala Glu Val Thr Leu Ser Val Lys Ala Asp Phe Pro Thr Pro Ser
            100                 105                 110

Ile Ser Asp Phe Glu Ile Pro Thr Ser Asn Ile Arg Arg Ile Ile Cys
        115                 120                 125

Ser Thr Ser Gly Gly Phe Pro Glu Pro His Leu Ser Trp Leu Glu Asn
    130                 135                 140

Gly Glu Glu Leu Asn Ala Ile Asn Thr Thr Val Ser Gln Asp Pro Glu
145                 150                 155                 160

Thr Glu Leu Tyr Ala Val Ser Ser Lys Leu Asp Phe Asn Met Thr Thr
                165                 170                 175

Asn His Ser Phe Met Cys Leu Ile Lys Tyr Gly His Leu Arg Val Asn
            180                 185                 190

Gln Thr Phe Asn Trp Asn Thr Thr Lys Gln Glu His Phe Pro Asp Asn
        195                 200                 205

Gly Ser Gly Gly Gly Gly Ser Gly Gly Gly Gly Ser Gly Gly Gly Gly
    210                 215                 220

Ser Ala Glu Ser Lys Tyr Gly Pro Pro Cys Pro Pro Cys Pro Ala Pro
225                 230                 235                 240

Glu Ala Ala Gly Gly Pro Ser Val Phe Leu Phe Pro Pro Lys Pro Lys
                245                 250                 255

Asp Gln Leu Met Ile Ser Arg Thr Pro Glu Val Thr Cys Val Val Val
            260                 265                 270

Asp Val Ser Gln Glu Asp Pro Glu Val Gln Phe Asn Trp Tyr Val Asp
        275                 280                 285

Gly Val Glu Val His Asn Ala Lys Thr Lys Pro Arg Glu Glu Gln Phe
    290                 295                 300

Asn Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Gly Leu
                325                 330                 335

Pro Ser Ser Ile Glu Lys Thr Ile Ser Lys Ala Lys Gly Gln Pro Arg
            340                 345                 350

Glu Pro Gln Val Tyr Thr Leu Pro Pro Ser Gln Glu Glu Met Thr Lys
        355                 360                 365

Asn Gln Val Ser Leu Thr Cys Leu Val Lys Gly Phe Tyr Pro Ser Asp
    370                 375                 380

Ile Ala Val Glu Trp Glu Ser Asn Gly Gln Pro Glu Asn Asn Tyr Lys
385                 390                 395                 400
```

```
Thr Thr Pro Pro Val Leu Asp Ser Asp Gly Ser Phe Phe Leu Tyr Ser
                405                 410                 415

Arg Leu Thr Val Asp Lys Ser Arg Trp Gln Glu Gly Asn Val Phe Ser
            420                 425                 430

Cys Ser Val Leu His Glu Ala Leu His Asn His Tyr Thr Gln Lys Ser
        435                 440                 445

Leu Ser Leu Ser Leu Gly Gly Gly Gly Gly Ser Ala Pro Thr Ser Ser
    450                 455                 460

Ser Thr Lys Lys Thr Gln Leu Gln Leu Glu His Leu Leu Leu Asp Leu
465                 470                 475                 480

Gln Met Ile Leu Asn Gly Ile Asn Asn Tyr Lys Asn Pro Lys Leu Thr
                485                 490                 495

Ala Met Leu Thr Ala Lys Phe Tyr Met Pro Lys Lys Ala Thr Glu Leu
            500                 505                 510

Lys His Leu Gln Cys Leu Glu Glu Glu Leu Lys Pro Leu Glu Glu Val
        515                 520                 525

Leu Asn Gly Ala Gln Ser Lys Asn Phe His Leu Arg Pro Arg Asp Leu
    530                 535                 540

Ile Ser Asn Ile Asn Val Ile Val Leu Glu Leu Lys Gly Ser Glu Thr
545                 550                 555                 560

Thr Phe Met Cys Glu Tyr Ala Asp Glu Thr Ala Thr Ile Val Glu Phe
                565                 570                 575

Leu Asn Arg Trp Ile Thr Phe Cys Gln Ser Ile Ile Ser Thr Leu Thr
            580                 585                 590
```

<210> 31
<211> 1851
<212> DNA
<213> Artificial Sequence

<220>
<223> nucleotiedes coding fusion protein (GI101w)

<400> 31

```
atggatgcta tgctgagagg cctgtgttgc gtgctgctgc tgtgtggcgc tgtgttcgtg      60

tctccttctc acgctgtgat ccacgtgacc aaagaagtga aagaggtcgc cacactgtcc     120

tgcggccaca acgtttcagt ggaagaactg gcccagacca ggatctactg gcagaaagaa     180

aagaaaatgg tgctgaccat gatgtccggc gacatgaaca tctggcctga gtacaagaac     240

cggaccatct tcgacatcac caacaacctg tccatcgtga ttctggccct gaggccttct     300

gatgagggca cctatgagtg cgtggtgctg aagtacgaga aggacgcctt caagcgcgag     360

cacctggctg aagtgacact gtccgtgaag gccgactttc ccacaccttc catctccgac     420
```

ttcgagatcc ctacctccaa catccggcgg atcatctgtt ctacctctgg cggctttcct 480

gagcctcacc tgtcttggct ggaaaacggc gaggaactga acgccatcaa caccaccgtg 540

tctcaggacc ccgaaaccga gctgtacgct gtgtcctcca agctggactt caacatgacc 600

accaaccaca gcttcatgtg cctgattaag tacggccacc tgagagtgaa ccagaccttc 660

aactggaaca ccaccaagca agagcacttc cctgacaatg gatctggcgg cggaggttct 720

ggcggaggtg gaagcggagg cggaggatct gctgagtcta agtatggccc tccttgtcct 780

ccatgtcctg ctccagaagc tgctggcgga ccctctgtgt tcctgtttcc tccaaagcct 840

aaggaccagc tcatgatctc tcggacaccc gaagtgacct gcgtggtggt ggatgtgtct 900

caagaggacc ctgaggtgca gttcaattgg tacgtggacg gcgtggaagt gcacaacgcc 960

aagaccaagc ctagagagga acagttcaac tccacctaca gagtggtgtc cgtgctgacc 1020

gtgctgcacc aggattggct gaacggcaaa gagtacaagt gcaaggtgtc caacaagggc 1080

ctgccttcca gcatcgaaaa gaccatctcc aaggctaagg gccagcctag ggaaccccag 1140

gtttacaccc tgcctccaag ccaagaggaa atgaccaaga accaggtgtc cctgacctgc 1200

ctggtcaagg gcttctaccc ttccgacatt gccgtggaat gggagtccaa tggccagcct 1260

gagaacaact acaagaccac acctcctgtg ctggactccg acggctcctt ctttctgtac 1320

tctcgcctga ccgtggacaa gtctagatgg caagagggca acgtgttctc ctgctctgtg 1380

ctgcacgagg ccctgcacaa tcactacacc cagaagtccc tgtctctgtc tcttggaggt 1440

ggtggcggtt ctgcccctac cagctcctct accaagaaaa cccagctcca gttggagcat 1500

ctgctgctgg acctccagat gattctgaac gggatcaaca actataagaa ccccaagctg 1560

acccgcatgc tgacctttaa gttctacatg cccaagaagg ccaccgagct gaagcacctc 1620

cagtgcctgg aagaagaact gaagcccctg gaagaggtgc tgaatctggc ccagtccaag 1680

aacttccacc tgaggccacg ggacctgatc agcaacatca acgtgatcgt gctggaactg 1740

aagggctccg agacaacctt tatgtgcgag tacgccgacg agacagccac catcgtggaa 1800

tttctgaacc ggtggatcac cttctgccag agcatcatct ccacactgac c 1851

<210> 32
<211> 592
<212> PRT
<213> Artificial Sequence

<220>
<223> fusion protein (GI101w)

<400> 32

Val Ile His Val Thr Lys Glu Val Lys Glu Val Ala Thr Leu Ser Cys
1 5 10 15

Gly His Asn Val Ser Val Glu Glu Leu Ala Gln Thr Arg Ile Tyr Trp

```
            20                  25                  30

Gln Lys Glu Lys Lys Met Val Leu Thr Met Met Ser Gly Asp Met Asn
        35                  40                  45

Ile Trp Pro Glu Tyr Lys Asn Arg Thr Ile Phe Asp Ile Thr Asn Asn
    50                  55                  60

Leu Ser Ile Val Ile Leu Ala Leu Arg Pro Ser Asp Glu Gly Thr Tyr
65                  70                  75                  80

Glu Cys Val Val Leu Lys Tyr Glu Lys Asp Ala Phe Lys Arg Glu His
                85                  90                  95

Leu Ala Glu Val Thr Leu Ser Val Lys Ala Asp Phe Pro Thr Pro Ser
            100                 105                 110

Ile Ser Asp Phe Glu Ile Pro Thr Ser Asn Ile Arg Arg Ile Ile Cys
        115                 120                 125

Ser Thr Ser Gly Gly Phe Pro Glu Pro His Leu Ser Trp Leu Glu Asn
    130                 135                 140

Gly Glu Glu Leu Asn Ala Ile Asn Thr Thr Val Ser Gln Asp Pro Glu
145                 150                 155                 160

Thr Glu Leu Tyr Ala Val Ser Ser Lys Leu Asp Phe Asn Met Thr Thr
                165                 170                 175

Asn His Ser Phe Met Cys Leu Ile Lys Tyr Gly His Leu Arg Val Asn
            180                 185                 190

Gln Thr Phe Asn Trp Asn Thr Thr Lys Gln Glu His Phe Pro Asp Asn
        195                 200                 205

Gly Ser Gly Gly Gly Gly Ser Gly Gly Gly Gly Ser Gly Gly Gly Gly
    210                 215                 220

Ser Ala Glu Ser Lys Tyr Gly Pro Pro Cys Pro Pro Cys Pro Ala Pro
225                 230                 235                 240

Glu Ala Ala Gly Gly Pro Ser Val Phe Leu Phe Pro Pro Lys Pro Lys
                245                 250                 255

Asp Gln Leu Met Ile Ser Arg Thr Pro Glu Val Thr Cys Val Val Val
            260                 265                 270

Asp Val Ser Gln Glu Asp Pro Glu Val Gln Phe Asn Trp Tyr Val Asp
        275                 280                 285

Gly Val Glu Val His Asn Ala Lys Thr Lys Pro Arg Glu Glu Gln Phe
    290                 295                 300

Asn Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Gly Leu
                325                 330                 335

Pro Ser Ser Ile Glu Lys Thr Ile Ser Lys Ala Lys Gly Gln Pro Arg
            340                 345                 350

Glu Pro Gln Val Tyr Thr Leu Pro Pro Ser Gln Glu Glu Met Thr Lys
```

355 360 365

Asn Gln Val Ser Leu Thr Cys Leu Val Lys Gly Phe Tyr Pro Ser Asp
370 375 380

Ile Ala Val Glu Trp Glu Ser Asn Gly Gln Pro Glu Asn Asn Tyr Lys
385 390 395 400

Thr Thr Pro Pro Val Leu Asp Ser Asp Gly Ser Phe Phe Leu Tyr Ser
405 410 415

Arg Leu Thr Val Asp Lys Ser Arg Trp Gln Glu Gly Asn Val Phe Ser
420 425 430

Cys Ser Val Leu His Glu Ala Leu His Asn His Tyr Thr Gln Lys Ser
435 440 445

Leu Ser Leu Ser Leu Gly Gly Gly Gly Gly Ser Ala Pro Thr Ser Ser
450 455 460

Ser Thr Lys Lys Thr Gln Leu Gln Leu Glu His Leu Leu Leu Asp Leu
465 470 475 480

Gln Met Ile Leu Asn Gly Ile Asn Asn Tyr Lys Asn Pro Lys Leu Thr
485 490 495

Arg Met Leu Thr Phe Lys Phe Tyr Met Pro Lys Lys Ala Thr Glu Leu
500 505 510

Lys His Leu Gln Cys Leu Glu Glu Glu Leu Lys Pro Leu Glu Glu Val
515 520 525

Leu Asn Leu Ala Gln Ser Lys Asn Phe His Leu Arg Pro Arg Asp Leu
530 535 540

Ile Ser Asn Ile Asn Val Ile Val Leu Glu Leu Lys Gly Ser Glu Thr
545 550 555 560

Thr Phe Met Cys Glu Tyr Ala Asp Glu Thr Ala Thr Ile Val Glu Phe
565 570 575

Leu Asn Arg Trp Ile Thr Phe Cys Gln Ser Ile Ile Ser Thr Leu Thr
580 585 590

<210> 33
<211> 1848
<212> DNA
<213> Artificial Sequence

<220>
<223> nucleotiedes coding fusion protein (mGI102-M61)

<400> 33
atggatgcta tgctgagagg cctgtgttgc gtgctgctgc tgtgtggcgc tgtgttcgtg 60

tctccttctc acgctgtgga cgagcagctc tccaagtccg tgaaggataa ggtcctgctg 120

ccttgccggt acaactctcc tcacgaggac gagtctgagg accggatcta ctggcagaaa 180

```
cacgacaagg tggtgctgtc cgtgatcgcc ggaaagctga aagtgtggcc tgagtacaag        240
aacaggaccc tgtacgacaa caccacctac agcctgatca tcctgggcct cgtgctgagc        300
gatagaggca cctattcttg cgtggtgcag aagaaagagc ggggcaccta cgaagtgaag        360
cacctggctc tggtcaagct gtccatcaag gccgacttca gcacccctaa catcaccgag        420
tctggcaacc cttccgccga caccaagaga atcacctgtt tcgcctctgg cggcttccct        480
aagcctcggt tctcttggct ggaaaacggc agagagctgc ccggcatcaa taccaccatt        540
tctcaggacc cagagtccga gctgtacacc atctccagcc agctcgactt taacaccacc        600
agaaaccaca ccatcaagtg cctgattaag tacggcgacg cccacgtgtc cgaggacttt        660
acttgggaga aacctcctga ggaccctcct gactctggat ctggcggcgg aggttctggc        720
ggaggtggaa gcggaggcgg aggatctgct gagtctaagt atggccctcc ttgtcctcca        780
tgtcctgctc cagaagctgc tggcggaccc tctgtgttcc tgtttcctcc aaagcctaag        840
gaccagctca tgatctctcg gacccctgaa gtgacctgcg tggtggtgga tgtgtctcaa        900
gaggaccctg aggtgcagtt caattggtac gtggacggcg tggaagtgca caacgccaag        960
accaagccta gagaggaaca gttcaactcc acctatagag tggtgtccgt gctgaccgtg       1020
ctgcaccagg attggctgaa cggcaaagag tacaagtgca aggtgtccaa caagggcctg       1080
ccttccagca tcgaaaagac catcagcaag gctaagggcc agcctaggga accccaggtt       1140
tacaccctgc ctccaagcca agaggaaatg accaagaacc aggtgtccct gacctgcctg       1200
gtcaagggct tctacccttc cgacattgcc gtggaatggg agtccaatgg ccagcctgag       1260
aacaactaca agaccacacc tcctgtgctg gactccgacg gctccttctt tctgtactct       1320
cgcctgaccg tggacaagtc taggtggcaa gagggcaacg tgttctcctg ctctgtgctg       1380
cacgaggctc tgcacaacca ctacacccag aagtccctgt ctctgtctct tggaggtggt       1440
ggcggttctg cccctacctc cagctctacc aagaaaaccc agctccagtt ggagcatctg       1500
ctgctggacc tccagatgat cctgaatggc atcaacaatt acaagaaccc caagctgacc       1560
gccatgctga ccgctaagtt ctacatgccc aagaaggcca ccgagctgaa gcacttgcag       1620
tgcctggaaa gggaactgaa gcccctggaa gaagtgctga atctggccca gtccaagaac       1680
ttccacctga ggcctaggga cctgatctcc aacatcaacg tgatcgtgct ggaactgaaa       1740
ggctccgaga caaccttcat gtgcgagtac gccgacgaga cagccaccat cgtggaattt       1800
ctgaaccggt ggatcacctt ctgccagagc atcatctcca cactgacc                    1848
```

<210> 34
<211> 616
<212> PRT
<213> Artificial Sequence

<220>

<223> fusion protein (mGI102-M61)

<400> 34

```
Met Asp Ala Met Leu Arg Gly Leu Cys Cys Val Leu Leu Leu Cys Gly
1               5                   10                  15

Ala Val Phe Val Ser Pro Ser His Ala Val Asp Glu Gln Leu Ser Lys
            20                  25                  30

Ser Val Lys Asp Lys Val Leu Leu Pro Cys Arg Tyr Asn Ser Pro His
        35                  40                  45

Glu Asp Glu Ser Glu Asp Arg Ile Tyr Trp Gln Lys His Asp Lys Val
    50                  55                  60

Val Leu Ser Val Ile Ala Gly Lys Leu Lys Val Trp Pro Glu Tyr Lys
65                  70                  75                  80

Asn Arg Thr Leu Tyr Asp Asn Thr Thr Tyr Ser Leu Ile Ile Leu Gly
                85                  90                  95

Leu Val Leu Ser Asp Arg Gly Thr Tyr Ser Cys Val Val Gln Lys Lys
            100                 105                 110

Glu Arg Gly Thr Tyr Glu Val Lys His Leu Ala Leu Val Lys Leu Ser
        115                 120                 125

Ile Lys Ala Asp Phe Ser Thr Pro Asn Ile Thr Glu Ser Gly Asn Pro
    130                 135                 140

Ser Ala Asp Thr Lys Arg Ile Thr Cys Phe Ala Ser Gly Gly Phe Pro
145                 150                 155                 160

Lys Pro Arg Phe Ser Trp Leu Glu Asn Gly Arg Glu Leu Pro Gly Ile
                165                 170                 175

Asn Thr Thr Ile Ser Gln Asp Pro Glu Ser Glu Leu Tyr Thr Ile Ser
            180                 185                 190

Ser Gln Leu Asp Phe Asn Thr Thr Arg Asn His Thr Ile Lys Cys Leu
        195                 200                 205

Ile Lys Tyr Gly Asp Ala His Val Ser Glu Asp Phe Thr Trp Glu Lys
    210                 215                 220

Pro Pro Glu Asp Pro Pro Asp Ser Gly Ser Gly Gly Gly Gly Ser Gly
225                 230                 235                 240

Gly Gly Gly Ser Gly Gly Gly Gly Ser Ala Glu Ser Lys Tyr Gly Pro
                245                 250                 255

Pro Cys Pro Pro Cys Pro Ala Pro Glu Ala Ala Gly Gly Pro Ser Val
            260                 265                 270

Phe Leu Phe Pro Pro Lys Pro Lys Asp Gln Leu Met Ile Ser Arg Thr
        275                 280                 285

Pro Glu Val Thr Cys Val Val Val Asp Val Ser Gln Glu Asp Pro Glu
    290                 295                 300

Val Gln Phe Asn Trp Tyr Val Asp Gly Val Glu Val His Asn Ala Lys
305                 310                 315                 320
```

```
Thr Lys Pro Arg Glu Glu Gln Phe Asn Ser Thr Tyr Arg Val Val Ser
                325                 330                 335

Val Leu Thr Val Leu His Gln Asp Trp Leu Asn Gly Lys Glu Tyr Lys
            340                 345                 350

Cys Lys Val Ser Asn Lys Gly Leu Pro Ser Ser Ile Glu Lys Thr Ile
        355                 360                 365

Ser Lys Ala Lys Gly Gln Pro Arg Glu Pro Gln Val Tyr Thr Leu Pro
    370                 375                 380

Pro Ser Gln Glu Glu Met Thr Lys Asn Gln Val Ser Leu Thr Cys Leu
385                 390                 395                 400

Val Lys Gly Phe Tyr Pro Ser Asp Ile Ala Val Glu Trp Glu Ser Asn
                405                 410                 415

Gly Gln Pro Glu Asn Asn Tyr Lys Thr Thr Pro Pro Val Leu Asp Ser
            420                 425                 430

Asp Gly Ser Phe Phe Leu Tyr Ser Arg Leu Thr Val Asp Lys Ser Arg
        435                 440                 445

Trp Gln Glu Gly Asn Val Phe Ser Cys Ser Val Leu His Glu Ala Leu
    450                 455                 460

His Asn His Tyr Thr Gln Lys Ser Leu Ser Leu Ser Leu Gly Gly Gly
465                 470                 475                 480

Gly Gly Ser Ala Pro Thr Ser Ser Ser Thr Lys Lys Thr Gln Leu Gln
                485                 490                 495

Leu Glu His Leu Leu Leu Asp Leu Gln Met Ile Leu Asn Gly Ile Asn
            500                 505                 510

Asn Tyr Lys Asn Pro Lys Leu Thr Ala Met Leu Thr Ala Lys Phe Tyr
        515                 520                 525

Met Pro Lys Lys Ala Thr Glu Leu Lys His Leu Gln Cys Leu Glu Arg
    530                 535                 540

Glu Leu Lys Pro Leu Glu Glu Val Leu Asn Leu Ala Gln Ser Lys Asn
545                 550                 555                 560

Phe His Leu Arg Pro Arg Asp Leu Ile Ser Asn Ile Asn Val Ile Val
                565                 570                 575

Leu Glu Leu Lys Gly Ser Glu Thr Thr Phe Met Cys Glu Tyr Ala Asp
            580                 585                 590

Glu Thr Ala Thr Ile Val Glu Phe Leu Asn Arg Trp Ile Thr Phe Cys
        595                 600                 605

Gln Ser Ile Ile Ser Thr Leu Thr
    610                 615


<210>   35
<211>   153
<212>   PRT
<213>   Artificial Sequence
```

<220>
<223> wild type hIL-2

<400> 35
Met Tyr Arg Met Gln Leu Leu Ser Cys Ile Ala Leu Ser Leu Ala Leu
1 5 10 15

Val Thr Asn Ser Ala Pro Thr Ser Ser Ser Thr Lys Lys Thr Gln Leu
20 25 30

Gln Leu Glu His Leu Leu Leu Asp Leu Gln Met Ile Leu Asn Gly Ile
35 40 45

Asn Asn Tyr Lys Asn Pro Lys Leu Thr Arg Met Leu Thr Phe Lys Phe
50 55 60

Tyr Met Pro Lys Lys Ala Thr Glu Leu Lys His Leu Gln Cys Leu Glu
65 70 75 80

Glu Glu Leu Lys Pro Leu Glu Glu Val Leu Asn Leu Ala Gln Ser Lys
85 90 95

Asn Phe His Leu Arg Pro Arg Asp Leu Ile Ser Asn Ile Asn Val Ile
100 105 110

Val Leu Glu Leu Lys Gly Ser Glu Thr Thr Phe Met Cys Glu Tyr Ala
115 120 125

Asp Glu Thr Ala Thr Ile Val Glu Phe Leu Asn Arg Trp Ile Thr Phe
130 135 140

Cys Gln Ser Ile Ile Ser Thr Leu Thr
145 150

<210> 36
<211> 158
<212> PRT
<213> Artificial Sequence

<220>
<223> IL-2 with signal sequence

<400> 36
Met Asp Ala Met Leu Arg Gly Leu Cys Cys Val Leu Leu Leu Cys Gly
1 5 10 15

Ala Val Phe Val Ser Pro Ser His Ala Ala Pro Thr Ser Ser Ser Thr
20 25 30

Lys Lys Thr Gln Leu Gln Leu Glu His Leu Leu Leu Asp Leu Gln Met
35 40 45

Ile Leu Asn Gly Ile Asn Asn Tyr Lys Asn Pro Lys Leu Thr Arg Met
50 55 60

Leu Thr Phe Lys Phe Tyr Met Pro Lys Lys Ala Thr Glu Leu Lys His
65 70 75 80

Leu Gln Cys Leu Glu Glu Glu Leu Lys Pro Leu Glu Glu Val Leu Asn

85 90 95

Leu Ala Gln Ser Lys Asn Phe His Leu Arg Pro Arg Asp Leu Ile Ser
100 105 110

Asn Ile Asn Val Ile Val Leu Glu Leu Lys Gly Ser Glu Thr Thr Phe
115 120 125

Met Cys Glu Tyr Ala Asp Glu Thr Ala Thr Ile Val Glu Phe Leu Asn
130 135 140

Arg Trp Ile Thr Phe Cys Gln Ser Ile Ile Ser Thr Leu Thr
145 150 155

<210> 37
<211> 474
<212> DNA
<213> Artificial Sequence

<220>
<223> nucleotide sequence coding IL-2 with signal sequence

<400> 37
atggatgcta tgctgagagg cctgtgttgc gtgctgctgc tgtgtggcgc tgtgttcgtg 60

tctccttctc acgctgcccc taccagctcc tctaccaaga aaacccagct ccagttggag 120

catctgctgc tggacctcca gatgattctg aacgggatca acaactataa gaaccccaag 180

ctgacccgca tgctgacctt taagttctac atgcccaaga aggccaccga gctgaagcac 240

ctccagtgcc tggaagaaga actgaagccc ctggaagagg tgctgaatct ggcccagtcc 300

aagaacttcc acctgaggcc acgggacctg atcagcaaca tcaacgtgat cgtgctggaa 360

ctgaagggct ccgagacaac ctttatgtgc gagtacgccg acgagacagc caccatcgtg 420

gaatttctga accggtggat caccttctgc cagagcatca tctccacact gacc 474

<210> 38
<211> 13
<212> PRT
<213> Artificial Sequence

<220>
<223> hinge

<400> 38
Ala Glu Ser Lys Tyr Gly Pro Pro Cys Pro Pro Cys Pro
1 5 10

<210> 39
<211> 1461
<212> DNA
<213> Artificial Sequence

<220>
<223> nucleotides coding CD80-Fc protein

<400> 39
ggatccgcca ccatggatgc tatgctgaga ggcctgtgtt gcgtgctgct gctgtgtggc 60

gctgtgttcg tgtctccttc tcacgctgtg atccacgtga ccaaagaagt gaaagaggtc 120

gccacactgt cctgcggcca caacgtttca gtggaagaac tggcccagac caggatctac 180

tggcagaaag aaaagaaaat ggtgctgacc atgatgtccg gcgacatgaa catctggcct 240

gagtacaaga accggaccat cttcgacatc accaacaacc tgtccatcgt gattctggcc 300

ctgaggcctt ctgatgaggg cacctatgag tgcgtggtgc tgaagtacga gaaggacgcc 360

ttcaagcgcg agcacctggc tgaagtgaca ctgtccgtga aggccgactt tcccacacct 420

tccatctccg acttcgagat ccctacctcc aacatccggc ggatcatctg ttctacctct 480

ggcggctttc ctgagcctca cctgtcttgg ctggaaaacg gcgaggaact gaacgccatc 540

aacaccaccg tgtctcagga ccccgaaacc gagctgtacg ctgtgtcctc caagctggac 600

ttcaacatga ccaccaacca cagcttcatg tgcctgatta agtacggcca cctgagagtg 660

aaccagacct tcaactggaa caccaccaag caagagcact tccctgacaa tggatctggc 720

ggcggaggtt ctggcggagg tggaagcgga ggcggaggat ctgctgagtc taagtatggc 780

cctccttgtc ctccatgtcc tgctccagaa gctgctggcg gaccctctgt gttcctgttt 840

cctccaaagc ctaaggacca gctcatgatc tctcggacac ccgaagtgac ctgcgtggtg 900

gtggatgtgt ctcaagagga ccctgaggtg cagttcaatt ggtacgtgga cggcgtggaa 960

gtgcacaacg ccaagaccaa gcctagagag gaacagttca actccaccta cagagtggtg 1020

tccgtgctga ccgtgctgca ccaggattgg ctgaacggca aagagtacaa gtgcaaggtg 1080

tccaacaagg gcctgccttc cagcatcgaa aagaccatct ccaaggctaa gggccagcct 1140

agggaacccc aggtttacac cctgcctcca agccaagagg aaatgaccaa gaaccaggtg 1200

tccctgacct gcctggtcaa gggcttctac ccttccgaca ttgccgtgga atgggagtcc 1260

aatggccagc ctgagaacaa ctacaagacc acacctcctg tgctggactc cgacggctcc 1320

ttctttctgt actctcgcct gaccgtggac aagtctaggt ggcaagaggg caacgtgttc 1380

tcctgctctg tgctgcacga ggccctgcac aatcactaca cccagaagtc cctgtctctg 1440

tccctgggct gatgactcga g 1461

<210> 40
<211> 479
<212> PRT
<213> Artificial Sequence

<220>
<223> CD80-Fc protein

<400> 40

```
Met Asp Ala Met Leu Arg Gly Leu Cys Cys Val Leu Leu Leu Cys Gly
1               5                   10                  15

Ala Val Phe Val Ser Pro Ser His Ala Val Ile His Val Thr Lys Glu
            20                  25                  30

Val Lys Glu Val Ala Thr Leu Ser Cys Gly His Asn Val Ser Val Glu
        35                  40                  45

Glu Leu Ala Gln Thr Arg Ile Tyr Trp Gln Lys Glu Lys Lys Met Val
    50                  55                  60

Leu Thr Met Met Ser Gly Asp Met Asn Ile Trp Pro Glu Tyr Lys Asn
65                  70                  75                  80

Arg Thr Ile Phe Asp Ile Thr Asn Asn Leu Ser Ile Val Ile Leu Ala
                85                  90                  95

Leu Arg Pro Ser Asp Glu Gly Thr Tyr Glu Cys Val Val Leu Lys Tyr
            100                 105                 110

Glu Lys Asp Ala Phe Lys Arg Glu His Leu Ala Glu Val Thr Leu Ser
        115                 120                 125

Val Lys Ala Asp Phe Pro Thr Pro Ser Ile Ser Asp Phe Glu Ile Pro
    130                 135                 140

Thr Ser Asn Ile Arg Arg Ile Ile Cys Ser Thr Ser Gly Gly Phe Pro
145                 150                 155                 160

Glu Pro His Leu Ser Trp Leu Glu Asn Gly Glu Glu Leu Asn Ala Ile
                165                 170                 175

Asn Thr Thr Val Ser Gln Asp Pro Glu Thr Glu Leu Tyr Ala Val Ser
            180                 185                 190

Ser Lys Leu Asp Phe Asn Met Thr Thr Asn His Ser Phe Met Cys Leu
        195                 200                 205

Ile Lys Tyr Gly His Leu Arg Val Asn Gln Thr Phe Asn Trp Asn Thr
    210                 215                 220

Thr Lys Gln Glu His Phe Pro Asp Asn Gly Ser Gly Gly Gly Gly Ser
225                 230                 235                 240

Gly Gly Gly Gly Ser Gly Gly Gly Gly Ser Ala Glu Ser Lys Tyr Gly
                245                 250                 255

Pro Pro Cys Pro Pro Cys Pro Ala Pro Glu Ala Ala Gly Gly Pro Ser
            260                 265                 270

Val Phe Leu Phe Pro Pro Lys Pro Lys Asp Gln Leu Met Ile Ser Arg
        275                 280                 285

Thr Pro Glu Val Thr Cys Val Val Val Asp Val Ser Gln Glu Asp Pro
    290                 295                 300

Glu Val Gln Phe Asn Trp Tyr Val Asp Gly Val Glu Val His Asn Ala
305                 310                 315                 320

Lys Thr Lys Pro Arg Glu Glu Gln Phe Asn Ser Thr Tyr Arg Val Val
                325                 330                 335
```

Ser Val Leu Thr Val Leu His Gln Asp Trp Leu Asn Gly Lys Glu Tyr
340 345 350

Lys Cys Lys Val Ser Asn Lys Gly Leu Pro Ser Ser Ile Glu Lys Thr
355 360 365

Ile Ser Lys Ala Lys Gly Gln Pro Arg Glu Pro Gln Val Tyr Thr Leu
370 375 380

Pro Pro Ser Gln Glu Glu Met Thr Lys Asn Gln Val Ser Leu Thr Cys
385 390 395 400

Leu Val Lys Gly Phe Tyr Pro Ser Asp Ile Ala Val Glu Trp Glu Ser
405 410 415

Asn Gly Gln Pro Glu Asn Asn Tyr Lys Thr Thr Pro Pro Val Leu Asp
420 425 430

Ser Asp Gly Ser Phe Phe Leu Tyr Ser Arg Leu Thr Val Asp Lys Ser
435 440 445

Arg Trp Gln Glu Gly Asn Val Phe Ser Cys Ser Val Leu His Glu Ala
450 455 460

Leu His Asn His Tyr Thr Gln Lys Ser Leu Ser Leu Ser Leu Gly
465 470 475

<210> 41
<211> 1851
<212> DNA
<213> Artificial Sequence

<220>
<223> nucleotiedes coding fusion protein (hCD80-Fc-IL2wt)

<400> 41
atggatgcta tgctgagagg cctgtgttgc gtgctgctgc tgtgtggcgc tgtgttcgtg 60

tctccttctc acgctgtgat ccacgtgacc aaagaagtga aagaggtcgc cacactgtcc 120

tgcggccaca acgtttcagt ggaagaactg gcccagacca ggatctactg gcagaaagaa 180

aagaaaatgg tgctgaccat gatgtccggc gacatgaaca tctggcctga gtacaagaac 240

cggaccatct tcgacatcac caacaacctg tccatcgtga ttctggccct gaggccttct 300

gatgagggca cctatgagtg cgtggtgctg aagtacgaga aggacgcctt caagcgcgag 360

cacctggctg aagtgacact gtccgtgaag gccgactttc ccacaccttc catctccgac 420

ttcgagatcc ctacctccaa catccggcgg atcatctgtt ctacctctgg cggctttcct 480

gagcctcacc tgtcttggct ggaaaacggc gaggaactga acgccatcaa caccaccgtg 540

tctcaggacc ccgaaaccga gctgtacgct gtgtcctcca agctggactt caacatgacc 600

accaaccaca gcttcatgtg cctgattaag tacggccacc tgagagtgaa ccagaccttc 660

aactggaaca ccaccaagca agagcacttc cctgacaatg gatctggcgg cggaggttct 720

ggcggaggtg gaagcggagg cggaggatct gctgagtcta agtatggccc tccttgtcct 780

ccatgtcctg ctccagaagc tgctggcgga ccctctgtgt tcctgtttcc tccaaagcct 840

aaggaccagc tcatgatctc tcggacaccc gaagtgacct gcgtggtggt ggatgtgtct 900

caagaggacc ctgaggtgca gttcaattgg tacgtggacg gcgtggaagt gcacaacgcc 960

aagaccaagc ctagagagga acagttcaac tccacctaca gagtggtgtc cgtgctgacc 1020

gtgctgcacc aggattggct gaacggcaaa gagtacaagt gcaaggtgtc caacaagggc 1080

ctgccttcca gcatcgaaaa gaccatctcc aaggctaagg gccagcctag ggaaccccag 1140

gtttacaccc tgcctccaag ccaagaggaa atgaccaaga accaggtgtc cctgacctgc 1200

ctggtcaagg gcttctaccc ttccgacatt gccgtggaat gggagtccaa tggccagcct 1260

gagaacaact acaagaccac acctcctgtg ctggactccg acggctcctt ctttctgtac 1320

tctcgcctga ccgtggacaa gtctagatgg caagagggca acgtgttctc ctgctctgtg 1380

ctgcacgagg ccctgcacaa tcactacacc cagaagtccc tgtctctgtc tcttggaggt 1440

ggtggcggtt ctgcccctac cagctcctct accaagaaaa cccagctcca gttggagcat 1500

ctgctgctgg acctccagat gattctgaac gggatcaaca actataagaa ccccaagctg 1560

acccgcatgc tgacctttaa gttctacatg cccaagaagg ccaccgagct gaagcacctc 1620

cagtgcctgg aagaagaact gaagcccctg gaagaggtgc tgaatctggc ccagtccaag 1680

aacttccacc tgaggccacg ggacctgatc agcaacatca acgtgatcgt gctggaactg 1740

aagggctccg agacaacctt tatgtgcgag tacgccgacg agacagccac catcgtggaa 1800

tttctgaacc ggtggatcac cttctgccag agcatcatct ccacactgac c 1851

<210> 42
<211> 367
<212> PRT
<213> Artificial Sequence

<220>
<223> Fc-IL2wt

<400> 42

Ala Glu Ser Lys Tyr Gly Pro Pro Cys Pro Pro Cys Pro Ala Pro Glu
1 5 10 15

Ala Ala Gly Gly Pro Ser Val Phe Leu Phe Pro Pro Lys Pro Lys Asp
20 25 30

Gln Leu Met Ile Ser Arg Thr Pro Glu Val Thr Cys Val Val Val Asp
35 40 45

Val Ser Gln Glu Asp Pro Glu Val Gln Phe Asn Trp Tyr Val Asp Gly
50 55 60

Val Glu Val His Asn Ala Lys Thr Lys Pro Arg Glu Glu Gln Phe Asn
65 70 75 80

```
Ser Thr Tyr Arg Val Val Ser Val Leu Thr Val Leu His Gln Asp Trp
                85                  90                  95

Leu Asn Gly Lys Glu Tyr Lys Cys Lys Val Ser Asn Lys Gly Leu Pro
            100                 105                 110

Ser Ser Ile Glu Lys Thr Ile Ser Lys Ala Lys Gly Gln Pro Arg Glu
        115                 120                 125

Pro Gln Val Tyr Thr Leu Pro Pro Ser Gln Glu Glu Met Thr Lys Asn
    130                 135                 140

Gln Val Ser Leu Thr Cys Leu Val Lys Gly Phe Tyr Pro Ser Asp Ile
145                 150                 155                 160

Ala Val Glu Trp Glu Ser Asn Gly Gln Pro Glu Asn Asn Tyr Lys Thr
                165                 170                 175

Thr Pro Pro Val Leu Asp Ser Asp Gly Ser Phe Phe Leu Tyr Ser Arg
            180                 185                 190

Leu Thr Val Asp Lys Ser Arg Trp Gln Glu Gly Asn Val Phe Ser Cys
        195                 200                 205

Ser Val Leu His Glu Ala Leu His Asn His Tyr Thr Gln Lys Ser Leu
    210                 215                 220

Ser Leu Ser Leu Gly Gly Gly Gly Gly Ser Ala Pro Thr Ser Ser Ser
225                 230                 235                 240

Thr Lys Lys Thr Gln Leu Gln Leu Glu His Leu Leu Leu Asp Leu Gln
                245                 250                 255

Met Ile Leu Asn Gly Ile Asn Asn Tyr Lys Asn Pro Lys Leu Thr Arg
            260                 265                 270

Met Leu Thr Phe Lys Phe Tyr Met Pro Lys Lys Ala Thr Glu Leu Lys
        275                 280                 285

His Leu Gln Cys Leu Glu Glu Glu Leu Lys Pro Leu Glu Glu Val Leu
    290                 295                 300

Asn Leu Ala Gln Ser Lys Asn Phe His Leu Arg Pro Arg Asp Leu Ile
305                 310                 315                 320

Ser Asn Ile Asn Val Ile Val Leu Glu Leu Lys Gly Ser Glu Thr Thr
                325                 330                 335

Phe Met Cys Glu Tyr Ala Asp Glu Thr Ala Thr Ile Val Glu Phe Leu
            340                 345                 350

Asn Arg Trp Ile Thr Phe Cys Gln Ser Ile Ile Ser Thr Leu Thr
        355                 360                 365
```

```
<210>   43
<211>   1176
<212>   DNA
<213>   Artificial Sequence
```

<220>
<223> Fc-IL2wt

<400> 43
atggatgcta tgctgagagg cctgtgttgc gtgctgctgc tgtgtggcgc tgtgttcgtg 60

tctccttctc acgctgctga gtctaagtat ggccctcctt gtcctccatg tcctgctcca 120

gaagctgctg gcggaccctc tgtgttcctg tttcctccaa agcctaagga ccagctcatg 180

atctctcgga cacccgaagt gacctgcgtg gtggtggatg tgtctcaaga ggaccctgag 240

gtgcagttca attggtacgt ggacggcgtg gaagtgcaca acgccaagac caagcctaga 300

gaggaacagt tcaactccac ctacagagtg gtgtccgtgc tgaccgtgct gcaccaggat 360

tggctgaacg gcaaagagta caagtgcaag gtgtccaaca agggcctgcc ttccagcatc 420

gaaaagacca tctccaaggc taagggccag cctagggaac cccaggttta caccctgcct 480

ccaagccaag aggaaatgac caagaaccag gtgtccctga cctgcctggt caagggcttc 540

tacccttccg acattgccgt ggaatgggag tccaatggcc agcctgagaa caactacaag 600

accacacctc ctgtgctgga ctccgacggc tccttctttc tgtactctcg cctgaccgtg 660

gacaagtcta gatggcaaga gggcaacgtg ttctcctgct ctgtgctgca cgaggccctg 720

cacaatcact acacccagaa gtccctgtct ctgtctcttg gaggtggtgg cggttctgcc 780

cctaccagct cctctaccaa gaaaacccag ctccagttgg agcatctgct gctggacctc 840

cagatgattc tgaacgggat caacaactat aagaacccca agctgacccg catgctgacc 900

tttaagttct acatgcccaa gaaggccacc gagctgaagc acctccagtg cctggaagaa 960

gaactgaagc ccctggaaga ggtgctgaat ctggcccagt ccaagaactt ccacctgagg 1020

ccacgggacc tgatcagcaa catcaacgtg atcgtgctgg aactgaaggg ctccgagaca 1080

acctttatgt gcgagtacgc cgacgagaca gccaccatcg tggaatttct gaaccggtgg 1140

atcaccttct gccagagcat catctccaca ctgacc 1176

<210> 44
<211> 392
<212> PRT
<213> Artificial Sequence

<220>
<223> Fc-IL2v2

<400> 44
Met Asp Ala Met Leu Arg Gly Leu Cys Cys Val Leu Leu Leu Cys Gly
1 5 10 15

Ala Val Phe Val Ser Pro Ser His Ala Ala Glu Ser Lys Tyr Gly Pro
20 25 30

Pro Cys Pro Pro Cys Pro Ala Pro Glu Ala Ala Gly Gly Pro Ser Val

35 40 45

Phe Leu Phe Pro Pro Lys Pro Lys Asp Gln Leu Met Ile Ser Arg Thr
50 55 60

Pro Glu Val Thr Cys Val Val Val Asp Val Ser Gln Glu Asp Pro Glu
65 70 75 80

Val Gln Phe Asn Trp Tyr Val Asp Gly Val Glu Val His Asn Ala Lys
85 90 95

Thr Lys Pro Arg Glu Glu Gln Phe Asn Ser Thr Tyr Arg Val Val Ser
100 105 110

Val Leu Thr Val Leu His Gln Asp Trp Leu Asn Gly Lys Glu Tyr Lys
115 120 125

Cys Lys Val Ser Asn Lys Gly Leu Pro Ser Ser Ile Glu Lys Thr Ile
130 135 140

Ser Lys Ala Lys Gly Gln Pro Arg Glu Pro Gln Val Tyr Thr Leu Pro
145 150 155 160

Pro Ser Gln Glu Glu Met Thr Lys Asn Gln Val Ser Leu Thr Cys Leu
165 170 175

Val Lys Gly Phe Tyr Pro Ser Asp Ile Ala Val Glu Trp Glu Ser Asn
180 185 190

Gly Gln Pro Glu Asn Asn Tyr Lys Thr Thr Pro Pro Val Leu Asp Ser
195 200 205

Asp Gly Ser Phe Phe Leu Tyr Ser Arg Leu Thr Val Asp Lys Ser Arg
210 215 220

Trp Gln Glu Gly Asn Val Phe Ser Cys Ser Val Leu His Glu Ala Leu
225 230 235 240

His Asn His Tyr Thr Gln Lys Ser Leu Ser Leu Ser Leu Gly Gly Gly
245 250 255

Gly Gly Ser Ala Pro Thr Ser Ser Ser Thr Lys Lys Thr Gln Leu Gln
260 265 270

Leu Glu His Leu Leu Leu Asp Leu Gln Met Ile Leu Asn Gly Ile Asn
275 280 285

Asn Tyr Lys Asn Pro Lys Leu Thr Ala Met Leu Thr Ala Lys Phe Tyr
290 295 300

Met Pro Lys Lys Ala Thr Glu Leu Lys His Leu Gln Cys Leu Glu Glu
305 310 315 320

Glu Leu Lys Pro Leu Glu Glu Val Leu Asn Leu Ala Gln Ser Lys Asn
325 330 335

Phe His Leu Arg Pro Arg Asp Leu Ile Ser Asn Ile Asn Val Ile Val
340 345 350

Leu Glu Leu Lys Gly Ser Glu Thr Thr Phe Met Cys Glu Tyr Ala Asp
355 360 365

Glu Thr Ala Thr Ile Val Glu Phe Leu Asn Arg Trp Ile Thr Phe Cys

370 375 380

Gln Ser Ile Ile Ser Thr Leu Thr
385 390

<210> 45
<211> 1200
<212> DNA
<213> Artificial Sequence

<220>
<223> nucleotides coding Fc-IL2v2

<400> 45
ggatccgcca ccatggatgc tatgctgaga ggcctgtgtt gcgtgctgct gctgtgtggc 60

gctgtgttcg tgtctccatc tcacgccgct gagtctaagt acggccctcc ttgtcctcca 120

tgtcctgctc cagaagctgc tggcggaccc tctgtgttcc tgtttcctcc aaagcctaag 180

gaccagctca tgatctctcg gacccctgaa gtgacctgcg tggtggtgga tgtgtctcaa 240

gaggaccctg aggtgcagtt caattggtac gtggacggcg tggaagtgca caacgccaag 300

accaagccta gagaggaaca gttcaactcc acctacagag tggtgtccgt gctgaccgtg 360

ctgcaccagg attggctgaa cggcaaagag tacaagtgca aggtgtccaa caagggcctg 420

ccttccagca tcgaaaagac catctccaag gctaagggcc agcctaggga accccaggtt 480

tacaccctgc ctccaagcca agaggaaatg accaagaacc aggtgtccct gacctgcctg 540

gtcaagggct tctacccttc cgacattgcc gtggaatggg agtccaatgg ccagcctgag 600

aacaactaca agaccacacc tcctgtgctg gactccgacg gctccttctt tctgtactct 660

cgcctgaccg tggacaagtc taggtggcaa gagggcaacg tgttctcctg ctctgtgctg 720

cacgaggccc tgcacaatca ctacacccag aagtccctgt ctctgtctct tggcggaggc 780

ggaggatctg ctcctacctc cagctccacc aagaaaaccc agctccagtt ggagcatctg 840

ctgctggacc tccagatgat cctgaatggc atcaacaatt acaagaaccc caagctgacc 900

gccatgctga ccgctaagtt ctacatgccc aagaaggcca ccgagctgaa gcacctccag 960

tgcctggaag aggaactgaa gcccctggaa gaagtgctga atctggccca gtccaagaac 1020

ttccacctga ggcctaggga cctgatctcc aacatcaacg tgatcgtgct ggaactgaaa 1080

ggctccgaga caaccttcat gtgcgagtac gccgacgaga cagccaccat cgtggaattt 1140

ctgaaccggt ggatcacctt ctgccagtcc atcatctcca cactgacctg atgactcgag 1200

1200

<210> 46
<211> 592
<212> PRT
<213> Artificial Sequence

<220>
<223> CD80-Fc-IL2wt

<400> 46

```
Val Ile His Val Thr Lys Glu Val Lys Glu Val Ala Thr Leu Ser Cys
1               5                   10                  15

Gly His Asn Val Ser Val Glu Glu Leu Ala Gln Thr Arg Ile Tyr Trp
            20                  25                  30

Gln Lys Glu Lys Lys Met Val Leu Thr Met Met Ser Gly Asp Met Asn
        35                  40                  45

Ile Trp Pro Glu Tyr Lys Asn Arg Thr Ile Phe Asp Ile Thr Asn Asn
    50                  55                  60

Leu Ser Ile Val Ile Leu Ala Leu Arg Pro Ser Asp Glu Gly Thr Tyr
65                  70                  75                  80

Glu Cys Val Val Leu Lys Tyr Glu Lys Asp Ala Phe Lys Arg Glu His
                85                  90                  95

Leu Ala Glu Val Thr Leu Ser Val Lys Ala Asp Phe Pro Thr Pro Ser
            100                 105                 110

Ile Ser Asp Phe Glu Ile Pro Thr Ser Asn Ile Arg Arg Ile Ile Cys
        115                 120                 125

Ser Thr Ser Gly Gly Phe Pro Glu Pro His Leu Ser Trp Leu Glu Asn
    130                 135                 140

Gly Glu Glu Leu Asn Ala Ile Asn Thr Thr Val Ser Gln Asp Pro Glu
145                 150                 155                 160

Thr Glu Leu Tyr Ala Val Ser Ser Lys Leu Asp Phe Asn Met Thr Thr
                165                 170                 175

Asn His Ser Phe Met Cys Leu Ile Lys Tyr Gly His Leu Arg Val Asn
            180                 185                 190

Gln Thr Phe Asn Trp Asn Thr Thr Lys Gln Glu His Phe Pro Asp Asn
        195                 200                 205

Gly Ser Gly Gly Gly Gly Ser Gly Gly Gly Gly Ser Gly Gly Gly Gly
    210                 215                 220

Ser Ala Glu Ser Lys Tyr Gly Pro Pro Cys Pro Pro Cys Pro Ala Pro
225                 230                 235                 240

Glu Ala Ala Gly Gly Pro Ser Val Phe Leu Phe Pro Pro Lys Pro Lys
                245                 250                 255

Asp Gln Leu Met Ile Ser Arg Thr Pro Glu Val Thr Cys Val Val Val
            260                 265                 270

Asp Val Ser Gln Glu Asp Pro Glu Val Gln Phe Asn Trp Tyr Val Asp
        275                 280                 285

Gly Val Glu Val His Asn Ala Lys Thr Lys Pro Arg Glu Glu Gln Phe
    290                 295                 300
```

Asn Ser Thr Tyr Arg Val Val Ser Val Leu Thr Val Leu His Gln Asp
305 310 315 320

Trp Leu Asn Gly Lys Glu Tyr Lys Cys Lys Val Ser Asn Lys Gly Leu
325 330 335

Pro Ser Ser Ile Glu Lys Thr Ile Ser Lys Ala Lys Gly Gln Pro Arg
340 345 350

Glu Pro Gln Val Tyr Thr Leu Pro Pro Ser Gln Glu Glu Met Thr Lys
355 360 365

Asn Gln Val Ser Leu Thr Cys Leu Val Lys Gly Phe Tyr Pro Ser Asp
370 375 380

Ile Ala Val Glu Trp Glu Ser Asn Gly Gln Pro Glu Asn Asn Tyr Lys
385 390 395 400

Thr Thr Pro Pro Val Leu Asp Ser Asp Gly Ser Phe Phe Leu Tyr Ser
405 410 415

Arg Leu Thr Val Asp Lys Ser Arg Trp Gln Glu Gly Asn Val Phe Ser
420 425 430

Cys Ser Val Leu His Glu Ala Leu His Asn His Tyr Thr Gln Lys Ser
435 440 445

Leu Ser Leu Ser Leu Gly Gly Gly Gly Gly Ser Ala Pro Thr Ser Ser
450 455 460

Ser Thr Lys Lys Thr Gln Leu Gln Leu Glu His Leu Leu Leu Asp Leu
465 470 475 480

Gln Met Ile Leu Asn Gly Ile Asn Asn Tyr Lys Asn Pro Lys Leu Thr
485 490 495

Arg Met Leu Thr Phe Lys Phe Tyr Met Pro Lys Lys Ala Thr Glu Leu
500 505 510

Lys His Leu Gln Cys Leu Glu Glu Glu Leu Lys Pro Leu Glu Glu Val
515 520 525

Leu Asn Leu Ala Gln Ser Lys Asn Phe His Leu Arg Pro Arg Asp Leu
530 535 540

Ile Ser Asn Ile Asn Val Ile Val Leu Glu Leu Lys Gly Ser Glu Thr
545 550 555 560

Thr Phe Met Cys Glu Tyr Ala Asp Glu Thr Ala Thr Ile Val Glu Phe
565 570 575

Leu Asn Arg Trp Ile Thr Phe Cys Gln Ser Ile Ile Ser Thr Leu Thr
580 585 590

<210> 47
<211> 591
<212> PRT
<213> Artificial Sequence

<220>

<223> mGI-101

<400> 47

```
Val Asp Glu Gln Leu Ser Lys Ser Val Lys Asp Lys Val Leu Leu Pro
1               5                   10                  15

Cys Arg Tyr Asn Ser Pro His Glu Asp Glu Ser Glu Asp Arg Ile Tyr
            20                  25                  30

Trp Gln Lys His Asp Lys Val Val Leu Ser Val Ile Ala Gly Lys Leu
        35                  40                  45

Lys Val Trp Pro Glu Tyr Lys Asn Arg Thr Leu Tyr Asp Asn Thr Thr
    50                  55                  60

Tyr Ser Leu Ile Ile Leu Gly Leu Val Leu Ser Asp Arg Gly Thr Tyr
65                  70                  75                  80

Ser Cys Val Val Gln Lys Lys Glu Arg Gly Thr Tyr Glu Val Lys His
                85                  90                  95

Leu Ala Leu Val Lys Leu Ser Ile Lys Ala Asp Phe Ser Thr Pro Asn
            100                 105                 110

Ile Thr Glu Ser Gly Asn Pro Ser Ala Asp Thr Lys Arg Ile Thr Cys
        115                 120                 125

Phe Ala Ser Gly Gly Phe Pro Lys Pro Arg Phe Ser Trp Leu Glu Asn
    130                 135                 140

Gly Arg Glu Leu Pro Gly Ile Asn Thr Thr Ile Ser Gln Asp Pro Glu
145                 150                 155                 160

Ser Glu Leu Tyr Thr Ile Ser Ser Gln Leu Asp Phe Asn Thr Thr Arg
                165                 170                 175

Asn His Thr Ile Lys Cys Leu Ile Lys Tyr Gly Asp Ala His Val Ser
            180                 185                 190

Glu Asp Phe Thr Trp Glu Lys Pro Pro Glu Asp Pro Pro Asp Ser Gly
        195                 200                 205

Ser Gly Gly Gly Gly Ser Gly Gly Gly Gly Ser Gly Gly Gly Gly Ser
    210                 215                 220

Ala Glu Ser Lys Tyr Gly Pro Pro Cys Pro Pro Cys Pro Ala Pro Glu
225                 230                 235                 240

Ala Ala Gly Gly Pro Ser Val Phe Leu Phe Pro Pro Lys Pro Lys Asp
                245                 250                 255

Gln Leu Met Ile Ser Arg Thr Pro Glu Val Thr Cys Val Val Val Asp
            260                 265                 270

Val Ser Gln Glu Asp Pro Glu Val Gln Phe Asn Trp Tyr Val Asp Gly
        275                 280                 285

Val Glu Val His Asn Ala Lys Thr Lys Pro Arg Glu Glu Gln Phe Asn
    290                 295                 300

Ser Thr Tyr Arg Val Val Ser Val Leu Thr Val Leu His Gln Asp Trp
305                 310                 315                 320
```

```
Leu Asn Gly Lys Glu Tyr Lys Cys Lys Val Ser Asn Lys Gly Leu Pro
                325                 330                 335

Ser Ser Ile Glu Lys Thr Ile Ser Lys Ala Lys Gly Gln Pro Arg Glu
            340                 345                 350

Pro Gln Val Tyr Thr Leu Pro Pro Ser Gln Glu Glu Met Thr Lys Asn
        355                 360                 365

Gln Val Ser Leu Thr Cys Leu Val Lys Gly Phe Tyr Pro Ser Asp Ile
    370                 375                 380

Ala Val Glu Trp Glu Ser Asn Gly Gln Pro Glu Asn Asn Tyr Lys Thr
385                 390                 395                 400

Thr Pro Pro Val Leu Asp Ser Asp Gly Ser Phe Phe Leu Tyr Ser Arg
                405                 410                 415

Leu Thr Val Asp Lys Ser Arg Trp Gln Glu Gly Asn Val Phe Ser Cys
            420                 425                 430

Ser Val Leu His Glu Ala Leu His Asn His Tyr Thr Gln Lys Ser Leu
        435                 440                 445

Ser Leu Ser Leu Gly Gly Gly Gly Gly Ser Ala Pro Thr Ser Ser Ser
    450                 455                 460

Thr Lys Lys Thr Gln Leu Gln Leu Glu His Leu Leu Leu Asp Leu Gln
465                 470                 475                 480

Met Ile Leu Asn Gly Ile Asn Asn Tyr Lys Asn Pro Lys Leu Thr Ala
                485                 490                 495

Met Leu Thr Ala Lys Phe Tyr Met Pro Lys Lys Ala Thr Glu Leu Lys
            500                 505                 510

His Leu Gln Cys Leu Glu Glu Glu Leu Lys Pro Leu Glu Glu Val Leu
        515                 520                 525

Asn Leu Ala Gln Ser Lys Asn Phe His Leu Arg Pro Arg Asp Leu Ile
    530                 535                 540

Ser Asn Ile Asn Val Ile Val Leu Glu Leu Lys Gly Ser Glu Thr Thr
545                 550                 555                 560

Phe Met Cys Glu Tyr Ala Asp Glu Thr Ala Thr Ile Val Glu Phe Leu
                565                 570                 575

Asn Arg Trp Ile Thr Phe Cys Gln Ser Ile Ile Ser Thr Leu Thr
            580                 585                 590
```

## Description

### Technical Field

The present invention relates to a pharmaceutical composition for treating cancer including, as active ingredients, a fusion protein comprising a CD80 protein and an IL-2 variant, and a NK cell.

### Background Art

IL-2, also called as T-cell growth factor (TCGF), is a globular glycoprotein that plays a central role in production, survival, and homeostasis of lymphocyte. IL-2 protein has a size of 15.5 kDa to 16 kDa and consists of 133 amino acids. IL-2 mediates various immune actions by binding to the IL-2 receptor which has three distinct subunits. In addition, IL-2 is synthesized mainly by activated T cells, in particular by CD4+ helper T cells. IL-2 stimulates proliferation and differentiation of T cells, and induces production of cytotoxic T lymphocytes (CTLs) and differentiation of peripheral blood lymphocytes into cytotoxic cells and lymphokine-activated killer cells (LAK cells).

Meanwhile, CD80, also known as B7-1, is a member of the B7 family of membrane-bound proteins that are involved in immune regulation by binding to its ligand by way of delivering costimulatory responses and coinhibitory responses. CD80 is a transmembrane protein expressed on the surface of T cells, B cells, dendritic cells, and monocytes. CD80 is known to bind CD28, CTLA4 (CD152), and PD-L1. CD80, CD86, CTLA4, and CD28 are involved in a costimulatory-coinhibitory system. For example, they regulate activity of T cells and are involved in proliferation, differentiation, and survival thereof.

In addition, natural killer cells (hereinafter, NK cells) are known to exhibit anticancer activity by removing cancer cells (Loris Zamai et.al., J. Immunol., 178:4011-4016, 2007). The activity of NK cells is regulated by a balance of various activating and inhibitory receptor signaling. It is known that the anticancer activity of NK cells is also achieved by discriminating cancer cells through various immune receptors present on the surface. Due to major histocompatibility complex (MHC) class I present in normal cells, the normal cells are recognized by the inhibitory receptors of NK cells and not attacked thereby, but cancer cells or some infected cells are eliminated by NK cells due to reduced MHC Class I or ligands for activating receptors of NK cells. NK cells can eliminate cancer stem cells in addition to cancer cells, and thus are in the spotlight as a source for therapeutics that can not only inhibit the development, proliferation, and metastasis of cancer, but also reduce recurrence of cancer after complete recovery.

### Detailed Description of the Invention

### Technical Problem

Accordingly, as a result of studying to develop a safe and effective IL-2, the present inventors found out that co-administration of a novel fusion protein dimer comprising an IL-2 protein and a CD80 protein in one molecule in combination with natural killer cells exhibits an excellent anticancer effect, and have completed the present invention.

### Solution to Problem

To achieve the above purpose, in accordance with one aspect of the present invention, there is provided an anticancer agent including, as active ingredients, a fusion protein dimer comprising an IL-2 protein and a CD80 protein, and a natural killer cell.

### EFFECTS OF THE INVENTION

It was confirmed that a fusion protein dimer comprising an IL-2 protein and a CD80 protein may not only activate immune cells, but also exhibit synergistic effects when administered in combination with natural killer cells. Therefore, such combination therapy can be usefully applied to the treatment of cancer.

### Brief Description of Drawings

FIG. 1 is a schematic diagram of an embodiment of a fusion protein dimer used in the present invention;
FIG. 2 shows an image of SDS-PAGE confirming the obtained fusion protein dimer (GI-101);
FIG. 3 shows size exclusion chromatography (SEC) analysis of the obtained fusion protein dimer (GI-101);

FIG. 4 shows an image of SDS-PAGE confirming the obtained Fc-IL2v2 fusion protein;

FIG. 5 shows size exclusion chromatography (SEC) analysis of the obtained Fc-IL2v2 fusion protein;

FIG. 6 shows an image of SDS-PAGE confirming the obtained hCD80-Fc fusion protein;

FIG. 7 shows size exclusion chromatography (SEC) analysis of the obtained hCD80-Fc fusion protein;

FIG. 8 shows the results of the cancer cell viability according to treatment with GI-101 or CD80-Fc+Fc-IL2v2 when culturing a K562 cell line alone without natural killer cells (E/T ratio = 0/1). In this case, E indicates an NK cell as an effector cell, and T indicates a K562 cancer cell line as a target cell.

FIG. 9 shows the results of the cancer cell viability according to treatment with GI-101 or CD80-Fc+Fc-IL2v2 when culturing an MDA-MB-231 cell line alone without natural killer cells (E/T ratio = 0/1). In this case, E indicates an NK cell as an effector cell, and T indicates an MDA-MB-231 cancer cell line as a target cell.

FIG. 10 shows the results of the cancer cell viability according to treatment with GI-101 or CD80-Fc+Fc-IL2v2 when culturing an HCT-116 cell line alone without natural killer cells (E/T ratio = 0/1). In this case, E indicates an NK cell as an effector cell, and T indicates an HCT-116 cancer cell line as a target cell.

FIG. 11 shows the results of the cancer cell viability according to treatment with GI-101 or CD80-Fc+Fc-IL2v2 when culturing a A549 cell line alone without natural killer cells (E/T ratio = 0/1). In this case, E indicates an NK cell as an effector cell, and T indicates a A549 cancer cell line as a target cell.

FIG. 12 shows the results of the cancer cell viability according to treatment with a combination material GI-101 or CD80-Fc+Fc-IL2v2 when co-culturing a K562 cell line (target cell) with natural killer cells (effector cell) in E/T ratio = 1/3.

Here, Y-axis=0 indicates that the range of the viability signal (red signal) of cancer cells at the time of initial seeding is set to as 0. Y-axis>0 is the case where the range of viability signal of cancer cells found after the time of seeding increases, indicating that cell proliferation rate is faster than the cell death rate (cell proliferation rate>cell death rate). That is, it can be seen as increase in the number of cancer cells. However, it is thought that the lower the value of increase amount, the more inhibited the proliferation of cancer cells. Meanwhile, if the area of the viable cancer cells measured is smaller than that of the viable cancer cells determined at the time of initial seeding, it is expressed as a negative value, which indicates that the cell proliferation rate is slower than the cell death rate (cell proliferation rate<cell death rate). That is, decrease in the area compared to that measured at the time of initial seeding is expressed as a negative value. Therefore, it is suggested that a larger negative value may be related to not only inhibition of proliferation of cancer cells but also death of cancer cells thereof.

FIG. 13 shows the results of the cancer cell viability according to treatment with a combination material GI-101 or CD80-Fc+Fc-IL2v2 when co-culturing a K562 cell line with natural killer cells in E/T ratio = 1/1.

FIG. 14 shows the results of the cancer cell viability according to treatment with a combination material GI-101 or CD80-Fc+Fc-IL2v2 when co-culturing a K562 cell line with natural killer cells in E/T ratio = 3/1.

FIG. 15 shows the results of the cancer cell viability according to treatment with a combination material GI-101 or CD80-Fc+Fc-IL2v2 when co-culturing a K562 cell line with natural killer cells in E/T ratio = 10/1.

FIG. 16 shows the results of the cancer cell viability according to treatment with a combination material GI-101 or CD80-Fc+Fc-IL2v2 when co-culturing a MDA-MB231 cell line (target cell) with natural killer cells (effector cell) in E/T ratio = 1/3.

FIG. 17 shows the results of the cancer cell viability according to treatment with a combination material GI-101 or CD80-Fc+Fc-IL2v2 when co-culturing a MDA-MB231 cell line with natural killer cells in E/T ratio = 1/1.

FIG. 18 shows the results of the cancer cell viability according to treatment with a combination material GI-101 or CD80-Fc+Fc-IL2v2 when co-culturing a MDA-MB231 cell line with natural killer cells in E/T ratio = 3/1.

FIG. 19 shows the results of the cancer cell viability according to treatment with a combination material GI-101 or CD80-Fc+Fc-IL2v2 when co-culturing a MDA-MB231 cell line with natural killer cells in E/T ratio = 10/1.

FIG. 20 shows the results of the cancer cell viability according to treatment with a combination material GI-101 or CD80-Fc+Fc-IL2v2 when co-culturing a HCT-116 cell line (target cell) with natural killer cells (effector cell) in E/T ratio = 1/3.

FIG. 21 shows the results of the cancer cell viability according to treatment with a combination material GI-101 or CD80-Fc+Fc-IL2v2 when co-culturing a HCT-116 cell line with natural killer cells in E/T ratio = 1/1.

FIG. 22 shows the results of the cancer cell viability according to treatment with a combination material GI-101 or CD80-Fc+Fc-IL2v2 when co-culturing a HCT-116 cell line with natural killer cells in E/T ratio = 3/1.

FIG. 23 shows the results of the cancer cell viability according to treatment with a combination material GI-101 or CD80-Fc+Fc-IL2v2 when co-culturing a HCT-116 cell line with natural killer cells in E/T ratio = 10/1.

FIG. 24 shows the results of the cancer cell viability according to treatment with a combination material GI-101 or CD80-Fc+Fc-IL2v2 when co-culturing a A549 cell line (target cell) with natural killer cells (effector cell) in E/T ratio = 1/3.

FIG. 25 shows the results of the cancer cell viability according to treatment with a combination material GI-101 or CD80-Fc+Fc-IL2v2 when co-culturing a A549 cell line with natural killer cells in E/T ratio = 1/1.

FIG. 26 shows the results of the cancer cell viability according to treatment with a combination material GI-101 or CD80-Fc+Fc-IL2v2 when co-culturing a A549 cell line with natural killer cells in E/T ratio = 3/1.
FIG. 27 shows the results of the cancer cell viability according to treatment with a combination material GI-101 or CD80-Fc+Fc-IL2v2 when co-culturing a A549 cell line with natural killer cells in E/T ratio = 10/1.
FIG. 28 is a schematic diagram showing an administration schedule of mGI-101 and/or NK cells combination therapies to a CT26 transplanted carcinoma mouse model.
FIG. 29 shows the results of the tumor growth inhibitory effect according to co-administration of natural killer cells and mGI-101 to a CT26 transplanted carcinoma mouse model.
FIG. 30 shows the percentage of the tumor growth inhibition according to co-administration of natural killer cells and mGI-101 to a CT26 transplanted carcinoma mouse model.
FIG. 31 shows the tumor growth measurements of individual laboratory animals in each treatment group (vehicle, NK cell, mGI-101, NK cell + mGI-101) in a CT26 transplanted carcinoma mouse model.
FIG. 32 shows the tumor growth measurements of individual laboratory animals in the vehicle group in a CT26 transplanted carcinoma mouse model.
FIG. 33 shows the tumor growth measurements of individual laboratory animals in the NK cell treatment group in a CT26 transplanted carcinoma mouse model.
FIG. 34 shows the tumor growth measurements of individual laboratory animals in the mGI-101 treatment group in a CT26 transplanted carcinoma mouse model.
FIG. 35 shows the tumor growth measurements of individual laboratory animals in the co-administration group of natural killer cells and mGI-101 in a CT26 transplanted carcinoma mouse model.

## Best Mode for Carrying Out the Invention

An aspect of the present invention provides a pharmaceutical composition including, as active ingredients, a fusion protein comprising a CD80 protein and an IL-2 protein, and NK cells.

Another aspect of the present invention provides a pharmaceutical composition for treating cancer including, as active ingredients, a fusion protein comprising a CD80 protein and an IL-2 protein, and NK cells.

## Natural Killer Cell

As used herein, the term "NK cell" refers to a natural killer cell (hereinafter NK cell), and is one of innate immune cells which directly interacts with various macrophages and T cells or generates cytokines to regulate immune responses, and thereby playing an important role in autoimmune diseases. In the present invention, NK cells may be isolated from the spleen or bone marrow, but are not limited thereto. Specifically, the NK cells may be obtained from autologous or heterologous cells.

In addition, the NK cells may be derived from mammals or humans. Preferably, it may be obtained from an individual who intends to receive NK cell treatment. In this case, the NK cells may be directly isolated from blood of an individual and used, or immature NK cells or stem cells obtained from the individual may be differentiated and used.

In addition, the natural killer cells may be obtained by the following steps including: i) isolating cells that do not express CD3 from peripheral blood mononuclear cells (PBMC); ii) isolating cells that express CD56 from cells that do not express CD3 isolated in the above step; and iii) culturing an isolated cell in the presence of a fusion protein dimer comprising IL-2 or a variant thereof and CD80 or a fragment thereof.

Further, the natural killer cells may be obtained by the following steps including: i) isolating cells that do not express CD3 from PBMCs; and ii) culturing the isolated cells in the presence of a fusion protein dimer comprising IL-2 or a variant thereof and CD80 or a fragment thereof.

Furthermore, the natural killer cells may be obtained by the following steps including: i) isolating cells that do express CD56 from PBMCs; and ii) culturing the isolated cells in the presence of a fusion protein dimer comprising IL-2 or a variant thereof and CD80 or a fragment thereof.

## A fusion protein dimer comprising an IL-2 protein and a CD80 protein

As used herein, the term "IL-2" or "interleukin-2", unless otherwise stated, refers to any wild-type IL-2 obtained from any vertebrate source, including mammals, for example, primates (such as humans) and rodents (such as mice and rats). IL-2 may be obtained from animal cells, and also includes one obtained from recombinant cells capable of producing IL-2. In addition, IL-2 may be wild-type IL-2 or a variant thereof.

In the present specification, IL-2 or a variant thereof may be collectively expressed by the term "IL-2 protein" or "IL-2 polypeptide." IL-2, an IL-2 protein, an IL-2 polypeptide, and an IL-2 variant specifically bind to, for example, an IL-2 receptor. This specific binding may be identified by methods known to those skilled in the art.

An embodiment of IL-2 may have the amino acid sequence of SEQ ID NO: 35 or SEQ ID NO: 36. Here, IL-2 may also be in a mature form. Specifically, the mature IL-2 may not comprise a signal sequence, and may have the amino acid sequence of SEQ ID NO: 10. Here, IL-2 may be used under a concept encompassing a fragment of wild-type IL-2 in which a portion of N-terminus or C-terminus of the wild-type IL-2 is truncated.

In addition, the fragment of IL-2 may be in a form in which 1, 2, 3, 4, 5, 6, 7, 8, 9, 10, 11, 12, 13, 14, 15, 16, 17, 18, 19, 20, 21, 22, 23, 24, or 25 continuous amino acids are truncated from N-terminus of a protein having the amino acid sequence of SEQ ID NO: 35 or SEQ ID NO: 36. In addition, the fragment of IL-2 may be in a form in which 1, 2, 3, 4, 5, 6, 7, 8, 9, 10, 11, 12, 13, 14, 15, 16, 17, 18, 19, 20, 21, 22, 23, 24, or 25 continuous amino acids are truncated from C-terminus of a protein having the amino acid sequence of SEQ ID NO: 35 or SEQ ID NO: 36.

As used herein, the term "IL-2 variant" refers to a form in which a portion of amino acids in the full-length IL-2 or the above-described fragment of IL-2 is substituted. That is, an IL-2 variant may have an amino acid sequence different from wild-type IL-2 or a fragment thereof. However, an IL-2 variant may have activity equivalent or similar to the wild-type IL-2. Here, "IL-2 activity" may, for example, refer to specific binding to an IL-2 receptor, which specific binding can be measured by methods known to those skilled in the art.

Specifically, an IL-2 variant may be obtained by substitution of a portion of amino acids in the wild-type IL-2. An embodiment of the IL-2 variant obtained by amino acid substitution may be obtained by substitution of at least one of the $38^{th}$, $42^{nd}$, $45^{th}$, $61^{st}$, and $72^{nd}$ amino acids in the amino acid sequence of SEQ ID NO: 10.

Specifically, the IL-2 variant may be obtained by substitution of at least one of the $38^{th}$, $42^{nd}$, $45^{th}$, $61^{st}$, or $72^{nd}$ amino acid in the amino acid sequence of SEQ ID NO: 10 with another amino acid. In addition, when IL-2 is in a form in which a portion of N-terminus in the amino acid sequence of SEQ ID NO: 35 is truncated, the amino acid at a position complementarily corresponding to that in the amino acid sequence of SEQ ID NO: 10 may be substituted with another amino acid. For example, when IL-2 has the amino acid sequence of SEQ ID NO: 35, its IL-2 variant may be obtained by substitution of at least one of $58^{th}$, $62^{nd}$, $65^{th}$, $81^{st}$, or $92^{nd}$ amino acid in the amino acid sequence of SEQ ID NO: 35 with another amino acid. These amino acid residues correspond to the $38^{th}$, $42^{nd}$, $45^{th}$, $61^{st}$, and $72^{nd}$ amino acid residues in the amino acid sequence of SEQ ID NO: 10, respectively. According to an embodiment, one, two, three, four, five, six, seven, eight, nine, or ten amino acids may be substituted as long as such IL-2 variant maintains IL-2 activity. According to another embodiment, one to five amino acids may be substituted.

In an embodiment, an IL-2 variant may be in a form in which two amino acids are substituted. Specifically, the IL-2 variant may be obtained by substitution of the $38^{th}$ and $42^{nd}$ amino acids in the amino acid sequence of SEQ ID NO: 10. In addition, in an embodiment, the IL-2 variant may be obtained by substitution of the $38^{th}$ and $45^{th}$ amino acids in the amino acid sequence of SEQ ID NO: 10. In addition, in an embodiment, the IL-2 variant may be obtained by substitution of the $38^{th}$ and $61^{st}$ amino acids in the amino acid sequence of SEQ ID NO: 10. In addition, in an embodiment, the IL-2 variant may be obtained by substitution of the $38^{th}$ and $72^{nd}$ amino acids in the amino acid sequence of SEQ ID NO: 10. In addition, in an embodiment, the IL-2 variant may be obtained by substitution of the $42^{nd}$ and $45^{th}$ amino acids in the amino acid sequence of SEQ ID NO: 10. In addition, in an embodiment, the IL-2 variant may be obtained by substitution of the $42^{nd}$ and $61^{st}$ amino acids in the amino acid sequence of SEQ ID NO: 10. In addition, in an embodiment, the IL-2 variant may be obtained by substitution of the $42^{nd}$ and $72^{nd}$ amino acids in the amino acid sequence of SEQ ID NO: 10. In addition, in an embodiment, the IL-2 variant may be obtained by substitution of the $45^{th}$ and $61^{st}$ amino acids in the amino acid sequence of SEQ ID NO: 10. In addition, in an embodiment, the IL-2 variant may be obtained by substitution of the $45^{th}$ and $72^{nd}$ amino acids in the amino acid sequence of SEQ ID NO: 10. In addition, in an embodiment, the IL-2 variant may be obtained by substitution of the $61^{st}$ and $72^{nd}$ amino acids in the amino acid sequence of SEQ ID NO: 10.

Furthermore, an IL-2 variant may be in a form in which three amino acids are substituted. Specifically, the IL-2 variant may be obtained by substitution of the $38^{th}$, $42^{nd}$, and $45^{th}$ amino acids in the amino acid sequence of SEQ ID NO: 10. In addition, in an embodiment, the IL-2 variant may be obtained by substitution of the $38^{th}$, $42^{nd}$, and $61^{st}$ amino acids in the amino acid sequence of SEQ ID NO: 10. In addition, in an embodiment, the IL-2 variant may be obtained by substitution of the $38^{th}$, $42^{nd}$, and $72^{nd}$ amino acids in the amino acid sequence of SEQ ID NO: 10. In addition, in an embodiment, the IL-2 variant may be obtained by substitution of the $38^{th}$, $45^{th}$, and $61^{st}$ amino acids in the amino acid sequence of SEQ ID NO: 10. In addition, in an embodiment, the IL-2 variant may be obtained by substitution of the $38^{th}$, $45^{th}$, and $72^{nd}$ amino acids in the amino acid sequence of SEQ ID NO: 10. In addition, in an embodiment, the IL-2 variant may be obtained by substitution of the $38^{th}$, $61^{st}$, and $72^{nd}$ amino acids in the amino acid sequence of SEQ ID NO: 10. In addition, in an embodiment, the IL-2 variant may be obtained by substitution of the $42^{nd}$, $45^{th}$, and $61^{st}$ amino acids in the amino acid sequence of SEQ ID NO: 10. In addition, in an embodiment, the IL-2 variant may be obtained by substitution of the $42^{nd}$, $45^{th}$, and $72^{nd}$ amino acids in the amino acid sequence of SEQ ID NO: 10. In addition, in an embodiment, the IL-2 variant may be obtained by substitution of the $45^{th}$, $61^{st}$, and $72^{nd}$ amino acids in the amino acid sequence of SEQ ID NO: 10.

In addition, an IL-2 variant may be in a form in which four amino acids are substituted. Specifically, the IL-2 variant may be obtained by substitution of the $38^{th}$, $42^{nd}$, $45^{th}$, and $61^{st}$ amino acids in the amino acid sequence of SEQ ID NO: 10. In addition, in an embodiment, the IL-2 variant may be obtained by substitution of the 38th, 42nd, 45th, and 72nd amino acids in the amino acid sequence of SEQ ID NO: 10. In addition, in an embodiment, the IL-2 variant may be obtained by substitution of the 38th, 45th, 61st, and 72nd amino acids in the amino acid sequence of SEQ ID NO: 10. In addition, in an embodiment, the IL-2 variant may be obtained by substitution of the 38th, 42nd, 61st, and 72nd amino acids in the amino acid sequence of SEQ ID NO: 10. In addition, in an embodiment, the IL-2 variant may be obtained by substitution of 42nd, 45th, 61st, and 72nd amino acids in the amino acid sequence of SEQ ID NO: 10.

Furthermore, an IL-2 variant may be in a form in which five amino acids are substituted. Specifically, the IL-2 variant may be obtained by substitution of each of the 38th, 42nd, 45th, 61st, and 72nd amino acids in the amino acid sequence of SEQ ID NO: 10 with another amino acid.

Here, the "another amino acid" introduced by the substitution may be any one selected from the group consisting of alanine, arginine, asparagine, aspartic acid, cysteine, glutamic acid, glutamine, histidine, isoleucine, leucine, lysine, methionine, phenylalanine, proline, serine, threonine, tryptophan, tyrosine, and valine. However, regarding amino acid substitution for the IL-2 variant, in the amino acid sequence of SEQ ID NO: 10, the 38th amino acid cannot be substituted with arginine, the 42nd amino acid cannot be substituted with phenylalanine, the 45th amino acid cannot be substituted with tyrosine, the 61st amino acid cannot be substituted with glutamic acid, and the 72nd amino acid cannot be substituted with leucine.

Regarding amino acid substitution for an IL-2 variant, in the amino acid sequence of SEQ ID NO: 10, the 38th amino acid, arginine, may be substituted with an amino acid other than arginine. Preferably, regarding amino acid substitution for an IL-2 variant, in the amino acid sequence of SEQ ID NO: 10, the 38th amino acid, arginine, may be substituted with alanine (R38A).

Regarding amino acid substitution for an IL-2 variant, in the amino acid sequence of SEQ ID NO: 10, the 42nd amino acid, phenylalanine, may be substituted with an amino acid other than phenylalanine. Preferably, regarding amino acid substitution for an IL-2 variant, in the amino acid sequence of SEQ ID NO: 10, the 42nd amino acid, phenylalanine, may be substituted with alanine (F42A).

Regarding amino acid substitution for an IL-2 variant, in the amino acid sequence of SEQ ID NO: 10, the 45th amino acid, tyrosine, may be substituted with an amino acid other than tyrosine. Preferably, regarding amino acid substitution for an IL-2 variant, in the amino acid sequence of SEQ ID NO: 10, the 45th amino acid, tyrosine, may be substituted with alanine (Y45A).

Regarding amino acid substitution for an IL-2 variant, in the amino acid sequence of SEQ ID NO: 10, the 61st amino acid, glutamic acid, may be substituted with an amino acid other than glutamic acid. Preferably, regarding amino acid substitution for an IL-2 variant, in the amino acid sequence of SEQ ID NO: 10, the 61st amino acid, glutamic acid, may be substituted with arginine (E61R).

Regarding amino acid substitution for an IL-2 variant, in the amino acid sequence of SEQ ID NO: 10, the 72nd amino acid, leucine, may be substituted with an amino acid other than leucine. Preferably, regarding amino acid substitution for an IL-2 variant, in the amino acid sequence of SEQ ID NO: 10, the 72nd amino acid, leucine, may be substituted with glycine (L72G).

Specifically, an IL-2 variant may be obtained by at least one substitution selected from the group consisting of R38A, F42A, Y45A, E61R, and L72G, in the amino acid sequence of SEQ ID NO: 10.

Specifically, an IL-2 variant may be obtained by amino acid substitutions at two, three, four, or five positions among the positions selected from the group consisting of R38A, F42A, Y45A, E61R, and L72G.

In addition, an IL-2 variant may be in a form in which two amino acids are substituted. Specifically, an IL-2 variant may be obtained by the substitutions, R38A and F42A. In addition, in an embodiment, an IL-2 variant may be obtained by the substitutions, R38A and Y45A. In addition, in an embodiment, an IL-2 variant may be obtained by the substitutions, R38A and E61R. In addition, in an embodiment, an IL-2 variant may be obtained by the substitutions, R38A and L72G. In addition, in an embodiment, an IL-2 variant may be obtained by the substitutions, F42A and Y45A. In addition, in an embodiment, an IL-2 variant may be obtained by the substitutions, F42A and E61R. In addition, in an embodiment, an IL-2 variant may be obtained by the substitutions, F42A and L72G. In addition, in an embodiment, an IL-2 variant may be obtained by the substitutions, E61R and L72G.

Furthermore, an IL-2 variant may be in a form in which three amino acids are substituted. Specifically, an IL-2 variant may be obtained by the substitutions, R38A, F42A, and Y45A. In addition, in an embodiment, an IL-2 variant may be obtained by the substitutions, R38A, F42A, and E61R. In addition, in an embodiment, an IL-2 variant may be obtained by the substitutions, R38A, F42A, and L72G. In addition, in an embodiment, an IL-2 variant may be obtained by the substitutions, R38A, Y45A, and E61R. In addition, in an embodiment, an IL-2 variant may be obtained by the substitutions, R38A, Y45A, and L72G. In addition, in an embodiment, an IL-2 variant may be obtained by the substitutions, F42A, Y45A, and E61R. In addition, in an embodiment, an IL-2 variant may be obtained by the substitutions, F42A, Y45A, and L72G. In addition, in an embodiment, an IL-2 variant may be obtained by the substitutions, F42A, E61R, and L72G. In addition, in an embodiment, an IL-2 variant may be obtained by the substitutions, Y45A, E61R, and L72G.

In addition, an IL-2 variant may be in a form in which four amino acids are substituted. Specifically, an IL-2 variant may be obtained by the substitutions, R38A, F42A, Y45A, and E61R. In addition, in an embodiment, an IL-2 variant may be obtained by the substitutions, R38A, F42A, Y45A, and L72G. In addition, in an embodiment, an IL-2 variant may be obtained by the substitutions, R38A, F42A, E61R, and L72G. In addition, in an embodiment, an IL-2 variant may be obtained by the substitutions, R38A, Y45A, E61R, and L72G. In addition, in an embodiment, an IL-2 variant may be obtained by the substitutions, F42A, Y45A, E61R, and L72G.

Furthermore, an IL-2 variant may be obtained by the substitutions, R38A, F42A, Y45A, E61R, and L72G.

Preferably, an embodiment of the IL-2 variant may comprise which are any one selected from the following substitution combinations (a) to (d) in the amino acid sequence of SEQ ID NO: 10:

(a) R38A/F42A;
(b) R38A/F42A/Y45A;
(c) R38A/F42A/E61R; or
(d) R38A/F42A/L72G.

Here, when IL-2 has the amino acid sequence of SEQ ID NO: 35, an amino acid substitution may be present at a position complementarily corresponding to that in the amino acid sequence of SEQ ID NO: 10. In addition, even when IL-2 is a fragment of the amino acid sequence of SEQ ID NO: 35, an amino acid substitution may be present at a position complementarily corresponding to that in the amino acid sequence of SEQ ID NO: 10.

Specifically, the IL-2 variant may have the amino acid sequence of SEQ ID NO: 6, 22, 23, or 24.

In addition, the IL-2 variant may be characterized by having low *in vivo* toxicity. Here, the low *in vivo* toxicity may be a side effect caused by binding of IL-2 to the IL-2 receptor alpha chain (IL-2Rα). Various IL-2 variants have been developed to ameliorate the side effect caused by binding of IL-2 to IL-2Rα, and such IL-2 variants may be those disclosed in US Patent No. 5,229,109 and Korean Patent No. 1667096. In particular, IL-2 variants described in the present application have low binding ability for the IL-2 receptor alpha chain (IL-2Rα) and thus have lower *in vivo* toxicity than the wild-type IL-2.

As used herein, the term "CD80", also called "B7-1", is a membrane protein present in dendritic cells, activated B cells, and monocytes. CD80 provides costimulatory signals essential for activation and survival of T cells. CD80 is known as a ligand for the two different proteins, CD28 and CTLA-4, present on the surface of T cells. CD80 consists of 288 amino acids, and may specifically have the amino acid sequence of SEQ ID NO: 11. In addition, as used herein, the term "CD80 protein" refers to the full-length CD80 or a CD80 fragment.

As used herein, the term "CD80 fragment" refers to a truncated form of CD80. In addition, the CD80 fragment may be an extracellular domain of CD80. An embodiment of the CD80 fragment may be obtained by deletion of the $1^{st}$ to $34^{th}$ amino acids from N-terminus which are a signal sequence of CD80. Specifically, an embodiment of the CD80 fragment may be a protein consisting of the $35^{th}$ to $288^{th}$ amino acids in SEQ ID NO: 11. In addition, an embodiment of the CD80 fragment may be a protein consisting of the $35^{th}$ to $242^{nd}$ amino acids in SEQ ID NO: 11. In addition, an embodiment of the CD80 fragment may be a protein consisting of the $35^{th}$ to $232^{nd}$ amino acids in SEQ ID NO: 11. In addition, an embodiment of the CD80 fragment may be a protein consisting of the $35^{th}$ to $139^{th}$ amino acids in SEQ ID NO: 11. In addition, an embodiment of the CD80 fragment may be a protein consisting of the $142^{nd}$ to $242^{nd}$ amino acids in SEQ ID NO: 11. In an embodiment, a CD80 fragment may have the amino acid sequence of SEQ ID NO: 2.

In addition, the IL-2 protein and the CD80 protein may be linked to each other via a linker or a carrier. Specifically, the IL-2 or a variant thereof and the CD80 (B7-1) or a fragment thereof may be linked to each other via a linker or a carrier. In the present description, the linker and the carrier may be used interchangeably.

The linker links two proteins. An embodiment of the linker may include 1 to 50 amino acids, albumin or a fragment thereof, an Fc domain of an immunoglobulin, or the like. Here, the Fc domain of immunoglobulin refers to a protein that comprises heavy chain constant region 2 (CH2) and heavy chain constant region 3 (CH3) of an immunoglobulin, and does not comprise heavy and light chain variable regions and light chain constant region 1 (CH1) of an immunoglobulin. The immunoglobulin may be IgG, IgA, IgE, IgD, o45r IgM, and may preferably be IgG4. Here, Fc domain of wild-type immunoglobulin G4 may have the amino acid sequence of SEQ ID NO: 4.

In addition, the Fc domain of an immunoglobulin may be an Fc domain variant as well as wild-type Fc domain. In addition, as used herein, the term "Fc domain variant" may refer to a form which is different from the wild-type Fc domain in terms of glycosylation pattern, has a high glycosylation as compared with the wild-type Fc domain, or has a low glycosylation as compared with the wild-type Fc domain, or a deglycosylated form. In addition, an aglycosylated Fc domain is included therein. The Fc domain or a variant thereof may be adapted to have an adjusted number of sialic acids, fucosylations, or glycosylations, through culture conditions or genetic engineering of a host.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc regions of immunoglobulins, IgG, IgA, IgE, IgD, and IgM. In addition, the Fc domain variant may be in a form in which some amino acids of the Fc domain are substituted with other amino acids. An embodiment of the Fc domain variant may have the amino acid sequence of SEQ ID NO: 12.

The fusion protein may have a structure in which, using an Fc domain as a linker (or carrier), a CD80 protein and an IL-2 protein, or an IL-2 protein and a CD80 protein are linked to N-terminus and C-terminus of the fusion protein (FIG. 1). Linkage between N-terminus or C-terminus of the Fc domain and CD-80 or IL-2 may optionally be achieved by a linker peptide.

Specifically, a fusion protein may consist of the following structural formula (I) or (II):

N'-X-[linker (1)]n-Fc domain-[linker $(2)]_m$-Y-C' (I)

N'-Y-[linker (1)]n-Fc domain-[linker $(2)]_m$-X-C' (II)

Here, in the structural formulas (I) and (II),

N' is the N-terminus of the fusion protein,
C' is the C-terminus of the fusion protein,
X is a CD80 protein,
Y is an IL-2 protein,
the linkers (1) and (2) are peptide linkers, and
n and m are each independently 0 or 1.

Preferably, the fusion protein may consist of the structural formula (I). The IL-2 protein is as described above. In addition, the CD80 protein is as described above. According to an embodiment, the IL-2 protein may be an IL-2 variant with one to five amino acid substitutions as compared with the wild-type IL-2. The CD80 protein may be a fragment obtained by truncation of up to about 34 continuous amino acid residues from the N-terminus or C-terminus of the wild-type CD80. Alternatively, the CD80 protein may be an extracellular immunoglobulin-like domain having the activity of binding to the T cell surface receptors CTLA-4 and CD28.

Specifically, the fusion protein may have the amino acid sequence of SEQ ID NO: 9, 26, 28, or 30. According to another embodiment, the fusion protein includes a polypeptide having a sequence identity of 85%, 86%, 87%, 88%, 89%, 90%, 91%, 92%, 93%, 94%, 95%, 96%, 97%, 98%, 99%, or 100% to the amino acid sequence of SEQ ID NO: 9, 26, 28, or 30. Here, the identity is, for example, percent homology, and may be determined through homology comparison software such as BlastN software of the National Center of Biotechnology Information (NCBI).

The peptide linker (1) may be included between the CD80 protein and the Fc domain. The peptide linker (1) may consist of 5 to 80 continuous amino acids, 20 to 60 continuous amino acids, 25 to 50 continuous amino acids, or 30 to 40 continuous amino acids. In an embodiment, the peptide linker (1) may consist of 30 amino acids. In addition, the peptide linker (1) may comprise at least one cysteine. Specifically, the peptide linker (1) may comprise one, two, or three cysteines. In addition, the peptide linker (1) may be derived from the hinge of an immunoglobulin. In an embodiment, the peptide linker (1) may be a peptide linker consisting of the amino acid sequence of SEQ ID NO: 3.

The peptide linker (2) may consist of 1 to 50 continuous amino acids, 3 to 30 continuous amino acids, or 5 to 15 continuous amino acids. In an embodiment, the peptide linker (2) may be $(G4S)_n$ (where n is an integer of 1 to 10). Here, in $(G4S)_n$, n may be 1, 2, 3, 4, 5, 6, 7, 8, 9, or 10. In an embodiment, the peptide linker (2) may be a peptide linker consisting of the amino acid sequence of SEQ ID NO: 5.

In another aspect of the present invention, there is provided a pharmaceutical composition including, as an active ingredient, a dimer obtained by binding of two fusion proteins, each of which comprises an IL-2 protein and a CD80 protein, and a NK cell. The fusion protein comprising IL-2 or a variant thereof and CD80 or a fragment thereof is as described above.

Here, the binding between the fusion proteins constituting the dimer may be achieved by, but is not limited to, a disulfide bond formed by cysteines present in the linker. The fusion proteins constituting the dimer may be the same or different fusion proteins from each other. Preferably, the dimer may be a homodimer. An embodiment of the fusion protein constituting the dimer may be a protein having the amino acid sequence of SEQ ID NO: 9.

In the present invention, the cancer may be any one selected from the group consisting of gastric cancer, liver cancer, lung cancer, colorectal cancer, breast cancer, prostate cancer, ovarian cancer, pancreatic cancer, cervical cancer, thyroid cancer, larynx cancer, acute lymphoblastic leukemia, brain tumor, neuroblastoma, retinoblastoma, head and neck cancer, salivary cancer, and lymphoma.

A preferred dose of the pharmaceutical composition varies depending on the patient's condition and body weight, severity of disease, form of drug, route and duration of administration and may be appropriately selected by those skilled in the art. In the pharmaceutical composition for treating or preventing cancer of the present invention, the active ingredient may be comprised in any amount (effective amount) depending on application, use, dosage form, blending purpose, and the like, as long as the active ingredient can exhibit an anticancer activity. A conventional effective amount thereof will be determined within a range of 0.001% to 20.0% by weight, based on the total weight of the composition. Here, the term "effective amount" refers to an amount of an active ingredient capable of inducing an anticancer effect. Such an effective amount can be experimentally determined within the scope of common knowledge of those skilled in the art.

As used herein, the term "treatment" may be used to mean both therapeutic and prophylactic treatment. Here, prophylaxis may be used to mean that a pathological condition or disease of an individual is alleviated or relieved. In an embodiment, the term "treatment" includes both application or any form of administration for treating a disease in a mammal, including a human. In addition, the term includes inhibiting or slowing down a disease or disease progression; and includes meanings of restoring or repairing impaired or lost function so that a disease is partially or completely alleviated; stimulating inefficient processes; or alleviating a serious disease.

As used herein, the term "efficacy" refers to capability that can be determined by one or parameters, for example, survival or disease-free survival over a certain period of time such as one year, five years, or ten years. In addition, the parameter may include decrease of size of at least one tumor in an individual.

Pharmacokinetic parameters such as bioavailability and underlying parameters such as clearance rate may also affect efficacy. Thus, "enhanced efficacy" (for example, improvement in efficacy) may be due to enhanced pharmacokinetic parameters and improved efficacy, which may be measured by comparing clearance rate and tumor growth in laboratory animals or human subjects, or by comparing parameters such as survival, recurrence, or disease-free survival.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause adverse effects. A level of the effective amount may be determined depending on factors including the patient's health condition, kinds and severity of disease, activity of drug, the patient's sensitivity to drug, mode of administration, time of administration, route of administration and excretion rate, duration of treatment, formulation or simultaneously used drugs, and other factors well known in the medical field. In an embodiment, the therapeutically effective amount means an amount of drug effective to treat cancer.

Here, the pharmaceutical composition may further include a pharmaceutically acceptable carrier. The pharmaceutically acceptable carrier may be any carrier as long as the carrier is a non-toxic substance suitable for delivery to a patient. Distilled water, alcohol, fat, wax, and inert solid may be contained as the carrier. A pharmaceutically acceptable adjuvant (buffer, dispersant) may also be contained in the pharmaceutical composition.

Specifically, by including a pharmaceutically acceptable carrier in addition to the active ingredient, the pharmaceutical composition may be prepared into a parenteral formulation depending on its route of administration using conventional methods known in the art. Here, the term "pharmaceutically acceptable" means that the carrier does not have more toxicity than the subject to be applied (prescribed) can adapt while not inhibiting activity of the active ingredient.

When the pharmaceutical composition is prepared into a parenteral formulation, it may be made into preparations in the form of injections, transdermal patches, nasal inhalants, or suppositories with suitable carriers according to methods known in the art. In a case of being made into injections, sterile water, ethanol, polyol such as glycerol or propylene glycol, or a mixture thereof may be used as a suitable carrier; and an isotonic solution, such as Ringer's solution, phosphate buffered saline (PBS) containing triethanol amine or sterile water for injection, and 5% dextrose, or the like may preferably be used. Formulation of pharmaceutical compositions is known in the art, and reference may specifically be made to Remington's Pharmaceutical Sciences (19th ed., 1995) and the like. This document is considered part of the present description.

A preferred dose of a dimer in the pharmaceutical composition may range from 0.01 μg/kg to 10 g/kg, or 0.01 mg/kg to 1 g/kg, per day, depending on the patient's condition, body weight, sex, age, severity of the patient, and route of administration. The dose may be administered once a day or may be divided into several times a day. Such a dose should not be construed as limiting the scope of the present invention in any aspect. In addition, NK cells in the pharmaceutical composition may be administered at an amount of $1\times10^2$ to $1\times10^{13}$ cells, $1\times10^7$ to $1.5\times10^{11}$ cells, with being adjusted appropriately in the range showing a pharmacological effect.

Subjects to which the pharmaceutical composition can be applied (prescribed) are mammals and humans, with humans being particularly preferred. In addition to the active ingredient, the pharmaceutical composition of the present application may further include any compound or natural extract, which has already been validated for safety and is known to have anticancer activity so as to boost or reinforce anticancer activity.

In still yet another aspect of the present invention, there is provided a use of a fusion protein dimer comprising an IL-2 protein and a CD80 protein, and a NK cell for treating cancer.

In still yet another aspect of the present invention, there is provided a use of a fusion protein dimer comprising an IL-2 protein and a CD80 protein, and NK cells for enhancing a therapeutic effect on cancer.

In still yet another aspect of the present invention, there is provided a use of a fusion protein dimer comprising an IL-2 protein and a CD80 protein, and NK cells for manufacture of a medicament for treating cancer.

In still yet another aspect of the present invention, there is provided a method for treating cancer and/or a method for enhancing a therapeutic effect on cancer, including administering, to a subject, a fusion protein comprising an IL-2 protein and a CD80 protein or a fusion protein dimer where the two fusion proteins are linked, and a NK cell.

Here, the fusion protein dimer and the NK cells may be administered simultaneously or sequentially. In this case, the order of administration may be determined such that the administration of the fusion protein dimer may be followed by the administration of NK cells, or the administration of the NK cells may be followed by the fusion protein dimer.

The subject may be an individual suffering from cancer or an infectious disease. In addition, the subject may be a mammal, preferably a human. The fusion protein comprising an IL-2 protein and a CD80 protein, or the fusion protein dimer where the two fusion proteins are linked is as described above.

Route of administration, dose, and frequency of administration of the fusion protein or fusion protein dimer and NK cells may vary depending on the patient's condition and the presence or absence of side effects, and thus the fusion protein or fusion protein dimer may be administered to a subject in various ways and amounts. The optimal administration method, dose, and frequency of administration can be selected in an appropriate range by those skilled in the art. In addition, the fusion protein or fusion protein dimer may be administered in combination with other drugs or physiologically active substances whose therapeutic effect is known with respect to a disease to be treated, or may be formulated in the form of combination preparations with other drugs.

Due to IL-2 activity, the fusion protein in an embodiment of the present invention can activate immune cells such as natural killer cells. Thus, the fusion protein can be effectively used for cancer and infectious diseases. In particular, it was identified that as compared with the wild type, an IL-2 variant with two to five amino acid substitutions, in particular, an IL-2 variant that comprises amino acid substitutions at two, three, four, or five positions among the positions selected from the group consisting of R38A, F42A, Y45A, E61R, and L72G, has low binding ability for the IL-2 receptor alpha chain and thus exhibits improved characteristics with respect to pharmacological side effects of conventional IL-2. Thus, such an IL-2 variant, when used alone or in the form of a fusion protein, can decrease incidence of vascular (or capillary) leakage syndrome (VLS), a problem with IL-2 conventionally known.

## Mode for the Invention

Hereinafter, the present invention will be described in more detail by way of the following examples. However, the following examples are only for illustrating the present invention, and the scope of the present invention is not limited thereto.

### I. Preparation of fusion protein dimer comprising IL-2 and CD80 Preparatory Example 1. Preparation of a hCD80-Fc-IL-2 variant (2M): GI-101

In order to produce a fusion protein comprising a human CD80 fragment, a Fc domain, and an IL-2 variant, a polynucleotide including a nucleotide sequence (SEQ ID NO: 8) encoding a fusion protein comprising a signal peptide (SEQ ID NO: 1), a CD80 fragment (SEQ ID NO: 2), a linker-bound Ig hinge (SEQ ID NO: 3), a Fc domain (SEQ ID NO: 4), a linker (SEQ ID NO: 5), and an IL-2 variant (2M) in which two amino acids are substituted (R38A, F42A) (SEQ ID NO: 6) in this order from N-terminus was synthesized through Invitrogen GeneArt Gene Synthesis service of ThermoFisher Scientific Inc., and cloned into a pcDNA3_4 vector. In addition, the vector was introduced into CHO cells (EXPI-CHO™) to express a fusion protein of SEQ ID NO: 9. After introducing the vector, the CHO cells were cultured in an environment of 37°C, 125 RPM, and 8% $CO_2$ for 7 days, and then collected to purify a fusion protein. The purified fusion protein dimer was named as "GI-101."

Purification was performed using chromatography including MabSelect SuRe protein A resin. The fusion protein was bound under the condition of 25 mM Tris, 25 mM NaCl, and pH 7.4. Then, it was eluted with 100 mM NaCl and 100 mM acetic acid at pH 3. After putting 20% of 1M Tris-HCl at pH 9 into a collection tube, the fusion protein was collected. The collected fusion protein was dialyzed into PBS buffer for 16 hours to change.

Then, absorbance at a wavelength of 280 nm over time was measured by using size exclusion chromatography with TSKgel G3000SWXL column (TOSOH Bioscience) to obtain a high concentration of fusion protein. At this time, the isolated and purified fusion protein was subjected to SDS-PAGE under the reducing (R) or non-reducing (NR) conditions, and stained with Coomassie blue to confirm its purity (FIG. 2). It was confirmed that the fusion protein was included at a concentration of 2.78 mg/mL as detected using NanoDrop. Also, the result analyzed using size exclusion chromatography is as shown in FIG. 3.

### Preparatory Example 2. Preparation of a Fc-IL-2 variant (2M) dimer: Fc-IL-2v2

In order to produce a fusion protein comprising a Fc domain and an IL-2 variant, a polynucleotide including a nucleotide sequence (SEQ ID NO: 45) encoding a fusion protein comprising a signal peptide (SEQ ID NO: 1), Ig hinge (SEQ ID NO: 38), a Fc domain (SEQ ID NO: 4), a linker (SEQ ID NO: 5), and an IL-2 variant (2M) in which two amino acids are substituted (R38A, F42A) (SEQ ID NO: 6) in this order from N-terminus was synthesized through Invitrogen GeneArt Gene Synthesis service of ThermoFisher Scientific Inc., and cloned into a pcDNA3_4 vector. In addition, the vector was introduced into CHO cells (EXPI-CHO™) to express a fusion protein of SEQ ID NO: 44. After introducing the vector, the CHO cells were cultured in an environment of 37°C, 125 RPM, and 8% $CO_2$ for 7 days, and then collected to purify a fusion protein dimer. The purified fusion protein dimer was named as "Fc-IL2v2."

The purification and collection of the fusion protein were performed in the same manner as in the Preparatory Example 1. The isolated and purified fusion protein was subjected to SDS-PAGE under the reducing (R) or non-reducing (NR) conditions, and stained with Coomassie blue to confirm its purity (FIG. 4). As a result, it was confirmed that the fusion protein forms a dimer. Also, the result analyzed using size exclusion chromatography is as shown in FIG. 5.

**Preparatory Example 3. Preparation of a hCD80-Fc dimer: hCD80-Fc**

In order to produce a fusion protein comprising a human CD80 fragment and a Fc domain, a polynucleotide (SEQ ID NO: 39) including a nucleotide sequence encoding a fusion protein comprising a signal peptide (SEQ ID NO: 1), a CD80 fragment (SEQ ID NO: 2), a linker-bound Ig hinge (SEQ ID NO: 3), and a Fc domain (SEQ ID NO: 4) in this order from N-terminus was synthesized through Invitrogen GeneArt Gene Synthesis service of ThermoFisher Scientific Inc., and cloned into a pcDNA3_4 vector. In addition, the vector was introduced into CHO cells (EXPI-CHO™) to express a fusion protein of SEQ ID NO: 40. After introducing the vector, the CHO cells were cultured in an environment of 37°C, 125 RPM, and 8% $CO_2$ for 7 days, and then collected to purify a fusion protein dimer. The purified fusion protein dimer was named "hCD80-Fc."

Purification was performed using chromatography including MabSelect SuRe protein A resin. The fusion protein was bound under the condition of 25 mM Tris, 25 mM NaCl, and pH 7.4. Then, it was eluted with 100 mM NaCl and 100 mM acetic acid at pH 3. After putting 20% of 1 M Tris-HCl at pH 9 into a collection tube, the fusion protein was collected. The collected fusion protein was dialyzed into PBS buffer for 16 hours to change.

Then, absorbance at a wavelength of 280 nm over time was measured by using size exclusion chromatography with TSKgel G3000SWXL column (TOSOH Bioscience) to obtain a high concentration of fusion protein. At this time, the isolated and purified fusion protein was subjected to SDS-PAGE under the reducing (R) or non-reducing (NR) conditions, and stained with Coomassie blue to confirm its purity (FIG. 6). As a result, it was confirmed that the fusion protein forms a dimer. Also, the result analyzed using size exclusion chromatography is as shown in FIG. 7.

**Preparatory Example 4. Preparation of mCD80-Fc-IL-2 variant (2M): mGI101**

In accordance with the same method as in Preparatory Example 1, a mouse type GI-101 comprising a mouse-derived CD80 and IL-2 was manufactured. Specifically, in order to produce a fusion protein comprising a mouse CD80, an Fc domain, and an IL-2 variant, a polynucleotide was synthesized through the Invitrogen GeneArt Gene Synthesis service of ThermoFisher Scientific. The polynucleotide compriss a nucleotide sequence (SEQ ID NO: 14) which encodes a fusion protein that compriss a signal peptide (SEQ ID NO: 1), a mCD80 (SEQ ID NO: 13), a linker-bound Ig hinge (SEQ ID NO: 3), an Fc domain (SEQ ID NO: 4), a linker (SEQ ID NO: 5), and an IL-2 variant (2M) (R38A, F42A) (SEQ ID NO: 6) with two amino acid substitutions, in this order, from the N-terminus. The polynucleotide was inserted into pcDNA3_4 vector. In addition, the vector was introduced into CHO cells (EXPI-CHO™) to express the fusion protein of SEQ ID NO: 47. After the vector was introduced, culture was performed for 7 days in an environment of 37°C, 125 RPM, and 8% $CO_2$. Then, the culture was harvested and the fusion protein was purified therefrom. The purified fusion protein was designated "mGI101".

**II. Preparation and Culture of NK cells**

**Preparation Example 1. Isolation of peripheral blood mononuclear cells (PBMC) derived CD3(-)CD56(+) natural killer cells**

In order to obtain CD3(-) cells, the number of PBMCs (peripheral blood mononuclear cells, Zen-Bio. Inc, NC 27709, USA, Cat#: SER-PBMC-200-F) was measured using an ADAM-MC2 automated cell counter (NanoEnTek, purchased from Cosmo Genetech Co., Ltd.). The PBMCs were transferred to a new tube, and then centrifuged at 300×g for 5 minutes at a temperature of 4°C. 0.5% (v/v) of bovine serum albumin (BSA) and 2 mM of EDTA were included in PBS to prepare MACS buffer (pH 7.2). After centrifugation was completed, a cell pellet was treated with 80 μl of MACs buffer and 20 μl of CD3 magnetic beads (Miltenyi biotech, 130-050-101) per $1 \times 10^7$ cells to suspend, and then reacted at a temperature of 4°C for 15 minutes. 10 mL of MACs buffer was added for washing and centrifuged at 300×g for 10 minutes at a temperature of 4°C, and then the cell pellet was resuspended in 0.5 mL of MACs buffer.

2 mL of MACs buffer was first flowed into the LD column (Miltenyi Biotec, Bergisch Gladbach, Germany, Cat#: 130-042-901), and then the cell suspension was flowed. Then, CD3(-) cells passing through the LD column were obtained. At this time, CD3(-) cells were obtained by flowing 2 mL of MACs buffer three times so that the cells remaining in the LD column could be sufficiently separated. The obtained CD3(-) cells were counted using a cell counter, and then placed in a new tube and centrifuged at 300×g for 5 minutes at a temperature of 4°C. Then, the supernatant was removed, and then 80 μl of MACs buffer and 20 μl of CD56 magnetic beads (Miltenyi biotech, Cat#: 130-050-401) were added per $1\times10^7$ cells, followed by reaction at a temperature of 4°C for 15 minutes. 10 mL of MACs buffer was added for washing and centrifuged at 300×g for 10 minutes at a temperature of 4°C, and then the cell pellet was resuspended in 0.5 mL of MACs buffer.

3 mL of MACs buffer was first flowed into the LS column (Miltenyi Biotec, Bergisch Gladbach, Germany, Cat#: 130-042-901), and then the cell suspension was flowed. At this time, 2 mL of MACs buffer was flowed three times so that the cells remaining in the LS column could be sufficiently separated. Then, after the LS column was separated from a magnet stand, 5 mL of MACs buffer was added, and pressure was applied with a piston to obtain CD3(-)CD56(+) natural killer cells. The obtained CD3(-)CD56(+) natural killer cells was placed in a new tube and centrifuged at 300×g for 5 minutes at a temperature of 4°C. After removing the supernatant, the cells were suspended in the culture media, and the number of suspended cells was measured using a cell counter.

**Preparation Example 2. Culture and obtainment of CD3-CD56+ natural killer cells**

100 μl of CD335 (NKp46)-biotin and 100 μl of CD2-biotin included in a NK Cell Activation/Expansion Kit (Cat#: 130-112-968)(Miltenyi Biotec, Bergisch Gladbach, Germany) were placed in a 1.5 mL microtube and mixed. 500 μl of Anti-Biotin MACSiBead Particles was added and mixed. Then, 300 μl of MACs buffer was added, and mixed at 2°C to 8°C for 2 hours using a microtube rotator.

Then, 5 μl of NK activation beads per $1x10^6$ cells was transferred to a new tube. 1 mL of PBS was added and centrifuged at 300×g for 5 minutes. After removing the supernatant, an RPMI1640 medium added with 5% human AB serum (Cat#:H4522)(Sigma, St. Louis, Missouri, US) to be used was added on the basis of 5 μl per $10^6$ NK cells, and suspended beads, followed by inoculating into the CD3-CD56+ natural killer cells isolated in Preparation Example 1.

Next, the CD3-CD56+ natural killer cells were seeded in a 24-well plate, and an RPMI1640 medium added with 5% human AB serum (Cat#:H4522)(Sigma, St. Louis, Missouri, US) and rhIL-2(500 IU/mL) was added thereto, followed by culturing under the condition of 37°C and 5% $CO_2$. Then, the number of cells was determined every 2 days to subculture in the order of a 12-well plate, a 6-well plate, and a 25T flask when the cells occupied 80% or more of culture vessel (confluency), and finally all cells were harvested on day 21.

**Preparation example 3. Culture and collection of mouse-derived natural killer cell**

**Preparation example 3.1. Preparation of mouse spleen cell and bone marrow**

To obtain mouse-derived natural killer cells, first mouse spleen cells and bone marrow were prepared. Specifically, the spleen and femur were extracted from a 6-week-old female Balb/c (ORIENT BIO Inc.), and the fat and muscle were removed as much as possible while taking care of the femur not to break. The extracted femur was placed in 70% ethanol, followed by in a 50 mL tube containing 5 mL of PBS, and the spleen were immediately transferred to a 50 mL tube containing 5 mL of PBS, and then stored on ice. A 70 μm strainer was overlapped a 50 mL tube containing 5 mL of FACS buffer and prepared for the spleen and bone marrow, respectively. The composition of FACS buffer A is as described in table 1 below.

[Table 1]

| Composition | | | Volume | Final concentration |
|---|---|---|---|---|
| Product | | Manufacturer | | |
| FACS buffer A | FBS | Hyclone | 15 mL | 3% |
| | EDTA | Welgene | 10 mL | 10 mM |
| | HEPES | Welgene | 10 mL | 20 mM |
| | PolymyxinB | Merk | 300 mL | 10 g/mL |
| | Penicillin/ Streptomycin | Biolegend | 5 mL | 10,000 U/mL penicillin / 10,000 g/mL Streptomycin |
| | 100 mM sodium pyruvate | Gibco | 5 mL | 1 mM |
| | PBS | Sigma | to 500 mL | |

After the tissue was mashed with a syringe stick, 5 mL of FACS buffer A was added to collect the cells. Then, centrifugation was performed at 1,300 rpm for 5 minutes at 4°C, and the supernatant was removed. The spleen was dissolved with 3 mL of ACK lysis buffer to remove red blood cells, and then incubated on ice for 3 minutes. After 3 minutes, FACS buffer A was added to make the total volume of 20 mL. The resultant was vortexed, and then centrifuged at 1,300 rpm for 5 minutes at 4°C and the supernatant was removed. Red blood cells were removed by repeating the above process until the color of a pellet turned pink, and dissolved with 10 mL of FACS buffer A to count the cells.

Both sides of the femur bone on the 70 μm strainer for bone marrow was cut, and FACS buffer A was flowed into holes of the bone using a 1 ml needle of a syringe filled with 10 mL of FACS buffer A while moving back and forth, and then the cells were collected while flowing FACS buffer A to the cut tissue as well. The cells were collected, and then centrifuged at 1,300 rpm for 5 minutes at 4°C. Then, the supernatant was removed and dissolved with 10 mL of FACS buffer A, followed by counting the cell.

**Preparation example 3.2. Isolation and culture of mouse NK cell**

Using an NK cell isolation Kit (#130-115-818), NK cells were isolated from the spleen and bone marrow which were isolated from the 6-week-old female Balb/c (ORIENT BIO Inc.) mouse in Preparation Example 3.1 above. After isolation, centrifugation was performed at 1,300×g at 4°C, and the supernatant was removed.

40 μl of MACS buffer per $10^7$ cells (600 μl for spleen, 200 μl for bone marrow) was added to resuspend the cell pellet, and 40 μl of NK cell cocktail per $10^7$ cells (150 μl for spleen, 50 μl for bone marrow) was added. However, in this case, more than $5\times10^7$ cells showed aggregation phenomenon, so they were divided and mixed. Then, centrifugation was performed at 300×g at 4°C, and the supernatant was removed.

2 mL of washing buffer per $10^7$ cells was added (30 mL for spleen, 10 mL for bone marrow), washed, centrifuged at 4°C at 300×g, and the supernatant was removed. Then, 80 μl of MACS buffer per $10^7$ cells was added (1.2 mL for spleen, 400 μl for bone marrow), and then 20 μl of anti-biotin microbeads per $10^7$ cells was added (300 μl for spleen, 100 μl for bone marrow), mixed, and cultured in a refrigerator for 10 minutes.

500 μl of cells mixed with anti-biotin microbeads was flowed into an MS column to obtain a supernatant that passed through the column. The same process was repeated to obtain the supernatant that passed through the column, and centrifuged at 4°C at 300×g. Then, the supernatant was removed. The cells were counted by resuspending in a GC-RPMI medium (1×) (spleen: $6.95\times10^5$/mL viability 59%, bone marrow: $1.58\times10^6$/mL viability 64%). The composition of a GC-RPMI medium is as described in Table 2 below.

[Table 2]

| Composition | | | | Final concentration |
|---|---|---|---|---|
| Product | | Manufacturer | Cat.# | |
| GC-RPMI | RPMI 1640 | Welgene | LM 011-01 | |
| | Pen-Strep | Welgene | LS 202-02 | 1× |
| | Gentamicin | Gibco | 15750-060 | 50 μg/mL |
| | Sodium pyruvate | Welgene | LS 013-01 | 1 mM |
| | 2-Mercaptoethanol | Gibco | 21985-023 | 55 μM |
| | NEAA | Gibco | 11140050 | 2 mM |
| | L-Glutamine | Gibco | 25030149 | 2 mM |
| | FBS | Hyclone | SH30084.03 | 10% |

After seeding $3.5\times10^5$ NK cells isolated from the spleen and bone marrow in a 60 mm culture vessel, 50 ng/mL of rmIL-2 was treated and cultured for 14 days.

**III. Preparation of cancer cells and construction of mouse model**

**Preparation example 4. Preparation of human-derived carcinoma cell line and culture medium thereof**

A culture solution suitable for each cancer cell line was used with reference to Table 3 below.

[Table 3]

| Cancer cell line | Organism of origin | Disease | Manufact urer | Components of culture medium |
|---|---|---|---|---|
| **K562** | Homo sapiens, Human | chronic myeloid leukemia | ATCC | RPMI1640 + 10% FBS |
| **MDA-MB-231** | Homo sapiens, Human | breast cancer | Korean Cell Line Bank | High glucose RPMI1640 + 10% FBS |
| **HCT-116** | Homo sapiens, Human | colon cancer | ATCC | McCoy's 5A + 10% FBS |
| **A549** | Homo sapiens, Human | lung cancer | ATCC | RPMI1640 + 10% FBS |

Specifically, $2\times10^6$ cells of cancer cell lines were resuspended in 8 mL of each culture solution and cultured in a 25T flask. When recover the cells, 1 mL of trypsin-EDTA (0.25%) was treated and then reacted in 5% $CO_2$ for 2 minutes for adherent type cells. Then, 5 mL of culture solution was added to recover the cells that had detached from the flask, and centrifuged at 300×g for 5 minutes.

Table 4 below shows specific culture solution compositions for each cancer cell lines.

[Table 4]

| Composition | | | | | Volume | Final conc. |
|---|---|---|---|---|---|---|
| Product | | | Manufacturer | Cat.# | | |
| RPMI164 0 (10% FBS) | Basic components | RPMI 1640 Medium(1 ×), liquid | Welgene | LM011-01 | 500 mL | |
| | | FBS | HYCLONE™ | SV30207. 02 | 50 mL | 10% |
| | | Penicillin-Streptomycin Solutions(× 100) | Welgene | LS 202-02 | 0.5 mL | 1× |

(continued)

| Composition | | | | | Volume | Final conc. |
|---|---|---|---|---|---|---|
| Product | | | Manufacturer | Cat.# | | |
| High glucose RPMI164 0 (10% FBS) | Basic components | High-glucose RPMI 1640 Medium | ATCC | 30-2001 | 500 mL | |
| | | FBS | HYCLONE™ | SV30207. 02 | 50 mL | 10% |
| | | Penicillin-Streptomycin Solutions(× 100) | Welgene | LS 202-02 | 0.5 mL | 1× |
| McCoy's (10% FBS) | Basic components | McCoy's 5A(ATCC) | ATCC | 30-2007 | 500 mL | |
| | | FBS | HYCLONE™ | SV30207. 02 | 50 mL | 10% |
| | | Penicillin-Streptomycin Solutions(× 100) | Welgene | LS 202-02 | 0.5 mL | 1× |

**Preparation example 5. Preparation of mouse-derived carcinoma cell line and culture medium thereof**

CT26.WT, a Mus musculus colon carcinoma cell, was purchased from ATCC (American Type Culture Collection, USA) (Table 5). Carcinoma cells to be used in the experiment were thawed, placed in a flask for cell culture, and cultured at 37°C, in a 5% $CO_2$ incubator (MCO-170M, Panasonic, Japan). The cells cultured on the day of cell line transplantation were placed in a centrifuge tube and collected, and then centrifuged at 125×g for 5 minutes to remove the supernatant. Then, PBS was added to prepare cell suspension ($5 \times 10^7$ cells/mL), dispensed in aliquot for 9 mice, and stored on ice until administration.

[Table 5]

| | Organism of origin | Disease | Manufacturer | Final concentration |
|---|---|---|---|---|
| **CT26** | Mus musculus, mouse | colon carcinoma | ATCC | $5\times10^5$ |

Fetal bovine serum (FBS; 16000-044, Thermofisher scientific, USA), penicillin-streptomycin (10,000 units/mL of penicillin and 10,000 $\mu$g/mL of streptomycin; 15140122, Thermofisher scientific, USA) and RPMI1640(A1049101, Thermofisher scientific, USA) were mixed to have the composition described in Table 6 below per 100 mL and used as a culture medium for carcinoma cells.

[Table 6]

| Composition | | | | Volume | Final concentration |
|---|---|---|---|---|---|
| Product | | Manufacturer | Cat.# | | |
| **Culture medium for cancer cells** | FBS | Thermofisher scientific | 16000-044 | 10 mL | 10% |
| | Penicillin & Streptomycin | Thermofisher scientific | 15140122 | 1 mL | 10,000 U/mL penicillin & 10,000 $\mu$g/mL Streptomycin |
| | RPMI 1640 | Thermofisher scientific | A1049101 | To 100 mL | |

**Preparation example 6. Preparation of carcinoma mouse model**

**Preparation example 6.1. Quarantine and acclimation processes for experimental mouse**

36 female 12-week-old BALB/c mice were purchased from ORIENT BIO Inc. Mice were brought into the animal lab and acclimated for 5 days prior to being used in the experiment. When received a mouse, they were evaluated for appearance and weighted to measure body weight. General symptoms were observed once a day during the acclimatization period for 5 days, and the body weight was measured at the end of the acclimation period, and then general symptoms and body weight changes were checked to evaluate the health condition of the mice. Mice with abnormality were euthanized under $CO_2$ gas anesthesia. Information about laboratory mice is summarized and shown in Table 7 below.

[Table 7]

| | Strain of origin | Purchased from | Age | Gender |
|---|---|---|---|---|
| **Mouse** | Balb/c | OrientBio | 12 weeks old | female |

**Preparation example 6.2. Transplantation of carcinoma cell line**

For a tumor growth inhibition model, the body weight was measured the next day after the end of the quarantine and acclimatization period, and then the CT26 cell suspension ($5\times10^5$ cells/0.1 mL) prepared for healthy animals was dispensed, filled in a disposable syringe, and administered subcutaneously (0.1 mL/head) to the right back of the mouse to transplant. General symptoms were observed once a day during the engraftment and growth period following cell line transplantation.

**Preparation example 6.3. Grouping of tumor growth inhibition mouse models**

After a certain period of CT26 cell transplantation, the tumor volume and body weight were measured for mice without abnormal health status, and were divided into 4 groups (9 mice per group) so that the average of each group reached 50 $mm^3$.

IV. **Determination of anticancer activity of NK cell and fusion protein dimer: *In vitro***

**Example 1. Determination of cancer cell growth inhibitory effect of NK cell and/or fusion protein dimer against various carcinoma**

Referring to a plate design to be used for a 96-well plate, 50 μl of 0.01% poly-L-ornithine solution (Cat#. P4957)(Sigma Aldrich, US) was respectively dispensed into well and coated. Then, it was left at room temperature for 1 hour. After 1 hour, the dispensed 0.01% poly-L-ornithine solution was removed and completely dried at room temperature for 1 hour. 2 μl of CELLTRACKER™ Deep Red Dye (Cat# C34565)(Thermo Scientific, Waltham, MA, USA) was added to the cancer cells (target cell) prepared at $4\times10^5$ cells/mL and allowed to react for 60 minutes at 37°C and 5% $CO_2$ condition.

After the reaction, the resultant was centrifuged at 300×g for 5 minutes. The supernatant was removed, and then dissolved in RPMI1640 + 5% hABS culture solution to $4\times10^5$ cells/mL. 50 μl of the prepared cancer cells was dispensed into a well in a coated 96-well plate, respectively. Then, the prepared plate was placed in an INCUCYTE® Live-Cell Analysis system (Satorius, Germany) instrument, and then allowed to stabilize for 10 minutes. A culture solution containing a test material was prepared in the RPMI1640 + 5% hABS referring to Table 8 below.

[Table 8]

| Test substance | control | GI-101 | CD80-Fc+Fc-IL2v2 |
|---|---|---|---|
| **Concentration** | 0 nM | 100 nM | CD80-Fc: 100 nM, Fc-IL2v2: 100 nM |

Natural killer cells (effector cells) were prepared by suspending in RPMI1640 + 5% hABS culture solution to $4\times10^5$ cells/mL. Referring to table 9 below, the natural killer cells prepared in Preparation examples 1 and 2 were added to each well in the plate having dispensed cancer cells, and then 100 μl of culture solution treated with INCUCYTE® CytoTox (250 nM) was added.

[Table 9]

| E/T ratio | 0/1 | 1/3 | 1/1 | 3/1 | 10/1 |
|---|---|---|---|---|---|
| **Number of NK cells** | - | $6.7\times10^3$ | $2\times10^4$ | $6\times10^4$ | $2\times10^5$ |

(continued)

| E/T ratio | 0/1 | 1/3 | 1/1 | 3/1 | 10/1 |
|---|---|---|---|---|---|
| Number of cancer cells | $2\times10^4$ | $2\times10^4$ | $2\times10^4$ | $2\times10^4$ | $2\times10^4$ |

Then, it was placed into the INCUCYTE® Live-Cell Analysis system and analyzed for 3 days with time interval of 30 minutes.

**Example 1.1. Determination of efficacy of fusion protein dimer alone in the presence of target cancer cell without NK cell**

As described in Example 1 above, it was observed that respective test material GI-101 and CD80-Fc+Fc-IL2v2 did not significantly affect the viability of cancer cells when culturing cell lines for various cancer types (K562, MDA-MB-231, HCT-116, and A549 cell lines) in the presence of target cancer cells alone without NK cells (see Table 9, E/T ratio=0/1)(FIGS. 8 to 11).

**Example 1.2. Cancer cell proliferative inhibition effect of NK cell and fusion protein dimer against K562 cell (lymphoblast)**

The cancer cell killing capability of natural killer cells in a K562 cell line, a Leukemia cancer cell line, was confirmed. Specifically, the cancer cell viability according to treatment with a test material GI-101 or CD80-Fc+Fc-IL2v2 as a combination material when co-culturing K562 cell lines as target cells (T) and natural killer cells as effector cells (E) at E/T ratio = 1/3, 1/1, 3/1 and 10/1, respectively was confirmed.

The result showed that when killing K562 cancer cells, treatment of GI-101 as a combination material of natural killer cell, which is a form of IL2-Fc-CD80 fusion protein, inhibited viability of cancer cells more than treatment of CD80-Fc+Fc-IL2v2 (FIGS. 16 to 19).

**Example 1.3. Cancer cell proliferative inhibition effect of NK cell and fusion protein dimer against MDA-MB231 cell**

The cancer cell killing capability of natural killer cells in an MDA-MB231 cell line, a breast cancer cell line, was confirmed. Specifically, the cancer cell viability according to treatment with a test material GI-101 or CD80-Fc+Fc-IL2v2 as a combination material when co-culturing MDA-MB231 cell lines as target cells (T) and natural killer cells as effector cells (E) at E/T ratio = 1/3, 1/1, 3/1 and 10/1, respectively was confirmed.

The result showed that when killing MDA-MB231 cancer cells, treatment of GI-101 as a combination material of natural killer cell, which is a form of IL2-Fc-CD80 fusion protein, inhibited viability of cancer cells more than treatment of CD80-Fc+Fc-IL2v2 (FIGS. 16 to 19).

**Example 1.4. Cancer cell proliferative inhibition effect of NK cell and fusion protein dimer against HCT-116 cell**

The cancer cell killing capability of natural killer cells in an HCT-116 cell line, a colon cancer cell line, was confirmed. Specifically, the cancer cell viability according to treatment with a test material GI-101 or CD80-Fc+Fc-IL2v2 as a combination material when co-culturing HCT-116 cell lines as target cells (T) and natural killer cells as effector cells (E) at E/T ratio = 1/3, 1/1, 3/1 and 10/1, respectively was confirmed.

The result showed that when killing HCT-116 cancer cells, treatment of GI-101 as a combination material of natural killer cell, which is a form of IL2-Fc-CD80 fusion protein, inhibited viability of cancer cells more than treatment of CD80-Fc+Fc-IL2v2 (FIGS. 20 to 23).

**Example 1.5. Cancer cell proliferative inhibition effect of NK cell and fusion protein dimer against A549 cell**

The cancer cell killing capability of natural killer cells in a A549 cell line, a colon cancer cell line, was confirmed. Specifically, the cancer cell viability according to treatment with a test material GI-101 or CD80-Fc+Fc-IL2v2 as a combination material when co-culturing A549 cell lines as target cells (T) and natural killer cells as effector cells (E) at E/T ratio = 1/3, 1/1, 3/1 and 10/1, respectively was confirmed.

The result showed that when killing A549 cancer cells, treatment of GI-101 as a combination material of natural killer cell, which is a form of IL2-Fc-CD80 fusion protein, inhibited viability of cancer cells more than treatment of CD80-Fc+Fc-IL2v2 (FIGS. 24 to 27).

**V. Determination of cancer cell killing capability of NK cell and fusion protein dimer in a carcinoma mouse model: *In vivo***

**Example 2. Administration of mGI-101 and NK cell**

To a carcinoma mouse model, the mGI-101 obtained in Preparation example 4 was administered by the intraperitoneal route and the mouse-derived NK cells prepared in Preparation example 3 were administered by the intravenous route. The administration was performed a total of three times once on the day of administration (day 6, day 10, and day 13 after tumor transplantation) using a disposable syringe (31G, 1 mL). For a tumor growth inhibition model, as shown in Table 10 below, cells to be administered and administration doses of the four groups were different (FIG. 28).

[Table 10]

| Group | Cells administered | Dosage of hIgG4 or mGI-101 (mg/kg) | Number of NK cell |
|---|---|---|---|
| G1 (control) | hIgG4 | 4 | 0 |
| G2 | hIgG4 + NK cell | 4 | $1\times10^6$ |
| G3 | mGI101 alone | 0.6 | 0 |
| G4 | mGI101 + NK cell | 0.6 | $1\times10^6$ |

**Example 3. Measurement of tumor volume of carcinoma mouse model**

Maximum length (L) and perpendicular width (W) of the tumor were measured twice a week using a Digital caliper (mitutoyo, Japan) and applied to Equation 1 below to calculate the tumor volume (TV).

[Equation 1]

$$TV\ (mm^3) = W \times W \times L \times 0.5$$

The percentage of tumor growth inhibition was calculated by using the following Equation 2:

**[Equation 2]**

$$\text{Tumor growth inhibition (\%)} = (1-(Ti-T0)/(Vi-V0))\times100$$

Ti = tumor volume before administration in the test group
T0 = tumor volume after administration in the test group
Vi = tumor volume before administration in the control group
V0 = tumor volume after administration in the control group

The tumor volume of each individual before administration was set as the value measured at the time of grouping.

**Example 4. Determination of tumor growth inhibitory effect in the CT26 transplanted carcinoma mouse model**

**Example 4.1. Measurement of tumor volume**

The CT26 colorectal cancer cell suspension ($5\times10^5$ cells/0.1 mL) prepared for healthy Balb/c mice was dispensed, and the prepared solution was filled in a disposable syringe, and administered subcutaneously (0.1 mL/head) to the right back of the animal to transplant. After tumor transplantation, the drugs shown in Table 10 were administered, respectively. Then, the size of the tumor was measured on day 10, day 13, and day 17. Tumor growth was inhibited in the groups treated with natural killer cell (NK cell) or mGI-101 alone compared to the control group (vehicle). Tumor growth was inhibited in the group treated with natural killer cell (NK cell) in combination with mGI-101 compared to the control group (vehicle). Tumor growth of the group treated with natural killer cells in combination with mGI-101 was inhibited compared to the groups treated with natural killer cells or mGI-101 alone (FIG. 29).

**Example 4.2. Tumor growth inhibition assay**

Tumor growth inhibition rate was calculated at the end of the experiment (after tumor transplantation, day 17) compared to the drug treatment day 1 (after tumor transplantation, day 10). The control group (vehicle) had 3 mice having tumor growth inhibition rate of 30% or more, 3 mice having tumor growth inhibition rate of 50% or more, and 2 mice having tumor growth inhibition rate of 80%. The natural killer cell treatment group had 6 mice having tumor growth inhibition rate of 30% or more, 5 mice having tumor growth inhibition rate of 50% or more, and 2 mice having tumor growth inhibition rate of 80%. The mGI-101 treatment group had 5 mice having tumor growth inhibition rate of 30% or more, 5 mice having tumor growth inhibition rate of 50% or more, and 1 mouse having tumor growth inhibition rate of 80%. The natural killer cell and mGI-101 combination treatment group had 7 mice having tumor growth inhibition rate of 30% or more, 6 mice having tumor growth inhibition rate of 50% or more, and 3 mice having tumor growth inhibition rate of 80% (FIG. 30). In FIG. 30, a black bar indicates the tumor growth inhibition rate of 30% or more, a light gray bar indicates the tumor growth inhibition rate of 50% or more, and a dark gray bar indicates the tumor growth inhibition rate of 80% or more.

**Example 4.3. Measurement of tumor volume for individual laboratory animals**

Tumor growth of individual laboratory animals in each treatment group was determined. Specifically, tumor growth of individual laboratory animals in the control group (vehicle), natural killer cells (NK cells), mGI-101, and natural killer cells + mGI-101 treatment groups were determined and shown in FIG. 31. In FIG. 31, a dotted line indicates a tumor size of 500 $mm^3$ and a solid line indicates a tumor size of 250 $mm^3$.

More specifically, the degree of tumor growth of individual laboratory animals of the control group was determined and shown in FIG. 32, and the degree of tumor growth of individual laboratory animals of the natural killer cell treatment group was determined and shown in FIG. 33. In addition, the degree of tumor growth of individual laboratory animals of the mGI-101 treatment group was determined and shown in FIG. 34, and the degree of tumor growth of individual laboratory animals of the natural killer cells and mGI-101 combination treatment group was determined and shown in FIG 35.

## Claims

1. A pharmaceutical composition for treating cancer comprising, as an active ingredient, a fusion protein comprising an IL-2 protein and a CD80 protein, and a natural killer (NK) cell.

2. The composition for treating cancer according to claim 1, wherein the IL-2 protein and the CD80 protein are linked via a linker.

3. The composition for treating cancer according to claim 1, wherein the IL-2 protein has the amino acid sequence of SEQ ID NO: 10.

4. The composition for treating cancer according to claim 1, wherein the IL-2 protein is an IL-2 variant.

5. The composition for treating cancer according to claim 4, wherein the IL-2 variant is obtained by substitution of at least one of the $38^{th}$, $42^{nd}$, $45^{th}$, $61^{st}$, and $72^{nd}$ amino acid in the amino acid sequence of SEQ ID NO: 10.

6. The composition for treating cancer according to claim 4, wherein the IL-2 variant is obtained by at least one substitution selected from the group consisting of R38A, F42A, Y45A, E61R, and L72G, in the amino acid sequence of SEQ ID NO: 10.

7. The composition for treating cancer according to claim 4, wherein the IL-2 variant comprises any one selected from the following substitution combinations (a) to (d) in the amino acid sequence of SEQ ID NO: 10:

(a) R38A/F42A
(b) R38A/F42A/Y45A
(c) R38A/F42A/E61R
(d) R38A/F42A/L72G.

8. The composition for treating cancer according to claim 4, wherein the IL-2 variant has the amino acid sequence of SEQ ID NO: 6, 22, 23, or 24.

9. The composition for treating cancer according to claim 1, wherein the CD80 protein has the amino acid sequence of SEQ ID NO: 11.

10. The composition for treating cancer according to claim 1, wherein the CD80 protein is a CD80 fragment.

11. The composition for treating cancer according to claim 10, wherein the CD80 fragment consists of the $35^{th}$ to $242^{nd}$ amino acids in SEQ ID NO: 11.

12. The composition for treating cancer according to claim 2, wherein the linker is an albumin or an Fc domain of an immunoglobulin.

13. The composition for treating cancer according to claim 12, wherein the Fc domain is a wild-type Fc domain or an Fc domain variant.

14. The composition for treating cancer according to claim 12, wherein the Fc domain has the amino acid sequence of SEQ ID NO: 4.

15. The composition for treating cancer according to claim 13, wherein the Fc domain variant has the amino acid sequence of SEQ ID NO: 12.

16. The composition for treating cancer according to claim 1, wherein the fusion protein consists of the following structural formula (I) or (II):

N'-X-[linker (1)]n-Fc domain-[linker $(2)]_m$-Y-C'    (I)

N'-Y-[linker (1)]n-Fc domain-[linker $(2)]_m$-X-C'    (II)

wherein, in the structural formulas (I) and (II),

N' is the N-terminus of the fusion protein,
C' is the C-terminus of the fusion protein,
X is a CD80 protein,
Y is an IL-2 protein,
the linkers (1) and (2) are peptide linkers, and
n and m are each independently 0 or 1.

**17.** The composition for treating cancer according to claim 16, wherein the linker (1) is a peptide linker consisting of the amino acid sequence of SEQ ID NO: 3.

**18.** The composition for treating cancer according to claim 16, wherein the linker (2) is a peptide linker consisting of the amino acid sequence of SEQ ID NO: 5.

**19.** The composition for treating cancer according to claim 16, wherein the fusion protein consists of the structural formula (I).

**20.** The composition for treating cancer according to claim 1, wherein the fusion protein has a sequence identity of 85% or more to the amino acid sequence of SEQ ID NO: 9, 26, 28, or 30.

**21.** The composition for treating cancer according to claim 1, wherein the fusion protein is a dimer.

**22.** The composition for treating cancer according to claim 1, wherein the cancer is any one selected from the group consisting of gastric cancer, liver cancer, lung cancer, colorectal cancer, breast cancer, prostate cancer, ovarian cancer, pancreatic cancer, cervical cancer, thyroid cancer, larynx cancer, acute lymphoblastic leukemia, brain tumor, neuroblastoma, retinoblastoma, head and neck cancer, salivary cancer, and lymphoma.

FIG.1

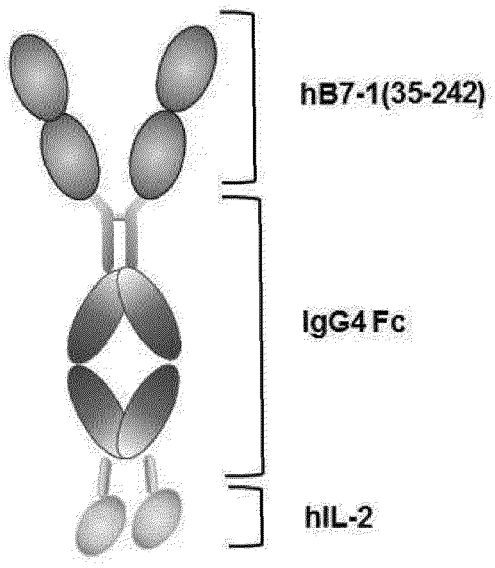
hB7-1(35-242)
IgG4 Fc
hIL-2

FIG.2

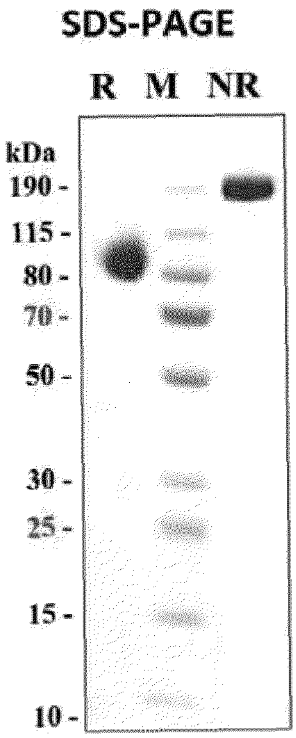
SDS-PAGE
R M NR
kDa
190 -
115 -
80 -
70 -
50 -
30 -
25 -
15 -
10 -

FIG.3

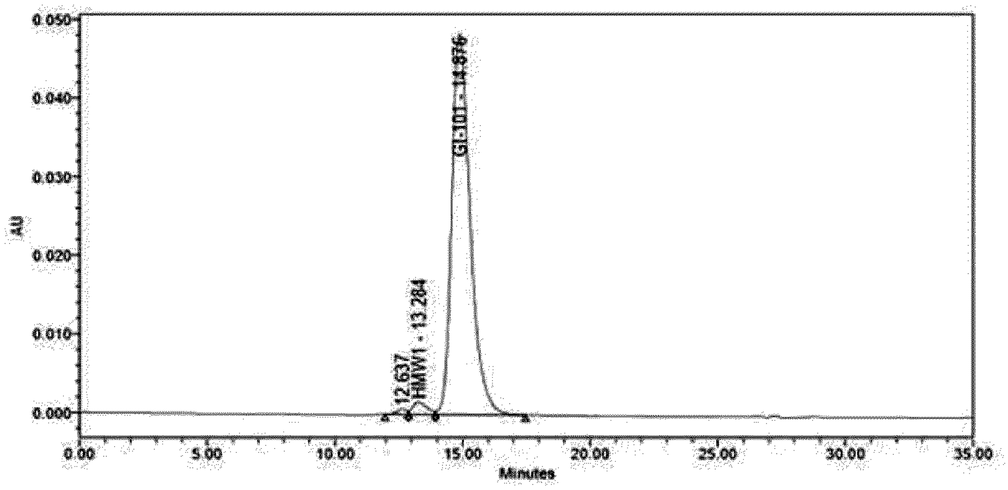


| | Peak Name | RT | Area | % Area | Height |
|---|---|---|---|---|---|
| 1 | | 12.637 | 16974 | 0.68 | 646 |
| 2 | HMW1 | 13.284 | 60001 | 2.39 | 1575 |
| 3 | HMW2 | 13.710 | | | |
| 4 | GI-101 | 14.876 | 2434297 | 96.93 | 48462 |

FIG.4

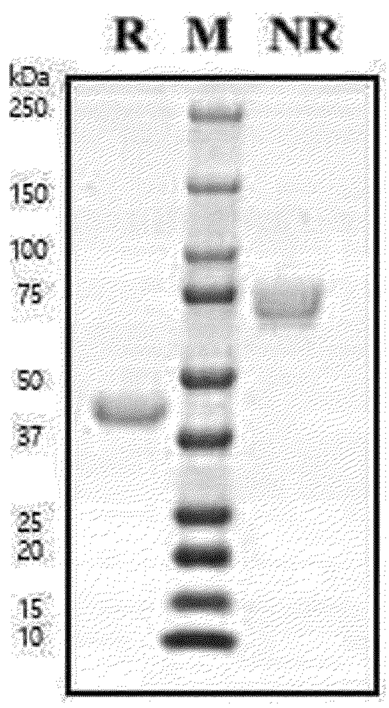

FIG.5

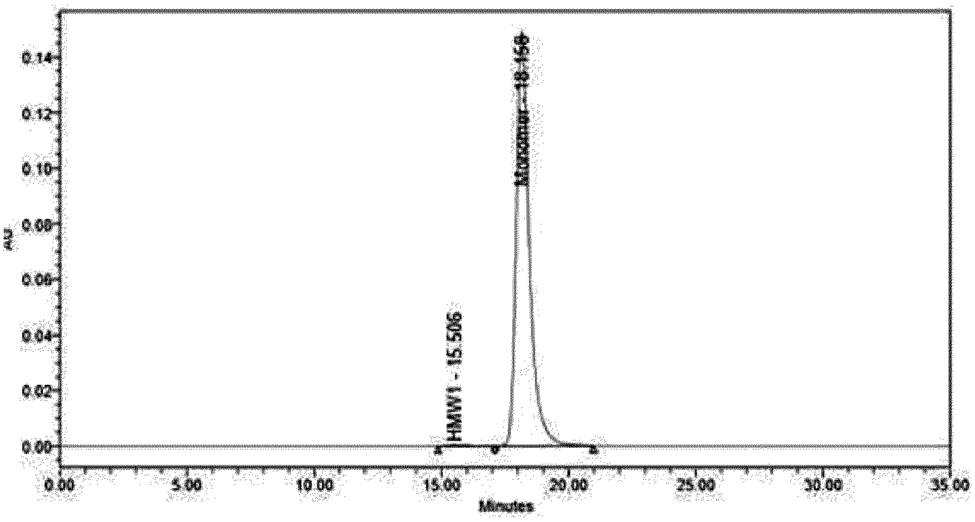


| | Peak Name | RT | Area | % Area | Height |
|---|---|---|---|---|---|
| 1 | HMW1 | 15.506 | 28137 | 0.52 | 426 |
| 2 | Monomer | 18.158 | 5363386 | 99.48 | 148945 |

FIG.6

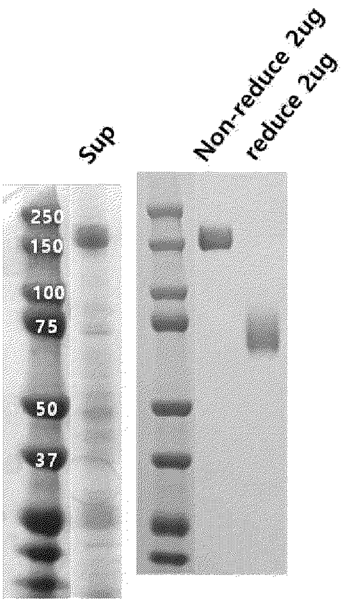


Expected size
(kDa)
monomer: 50.5
Dimer : 101

FIG.7

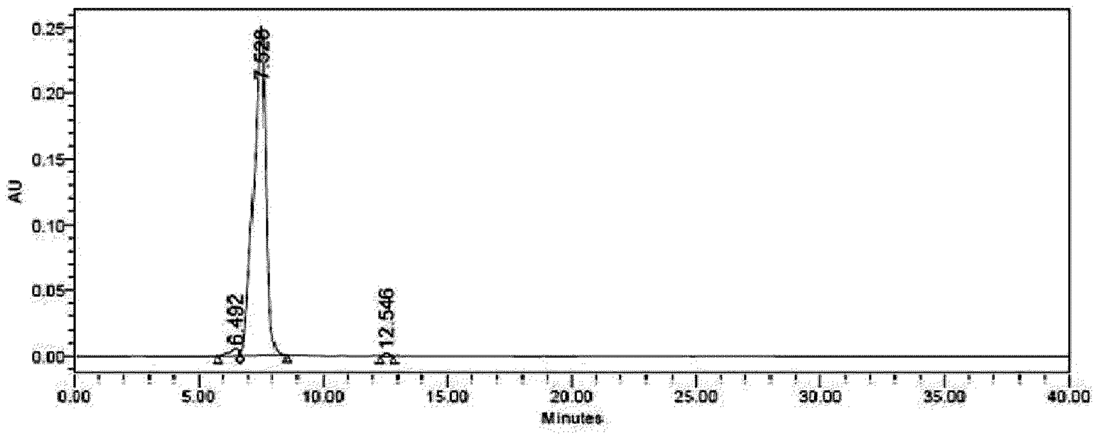


| | RT | Area | % Area | Height |
|---|---|---|---|---|
| 1 | 6.492 | 143663 | 1.66 | 5185 |
| 2 | 7.528 | 8497629 | 98.06 | 250509 |
| 3 | 12.546 | 24077 | 0.28 | 2005 |

FIG.8

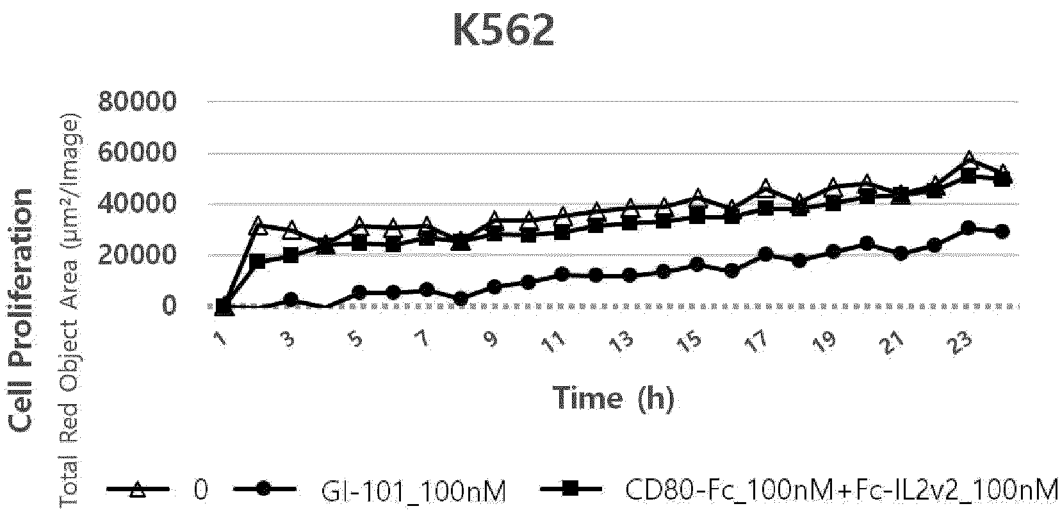

FIG.9

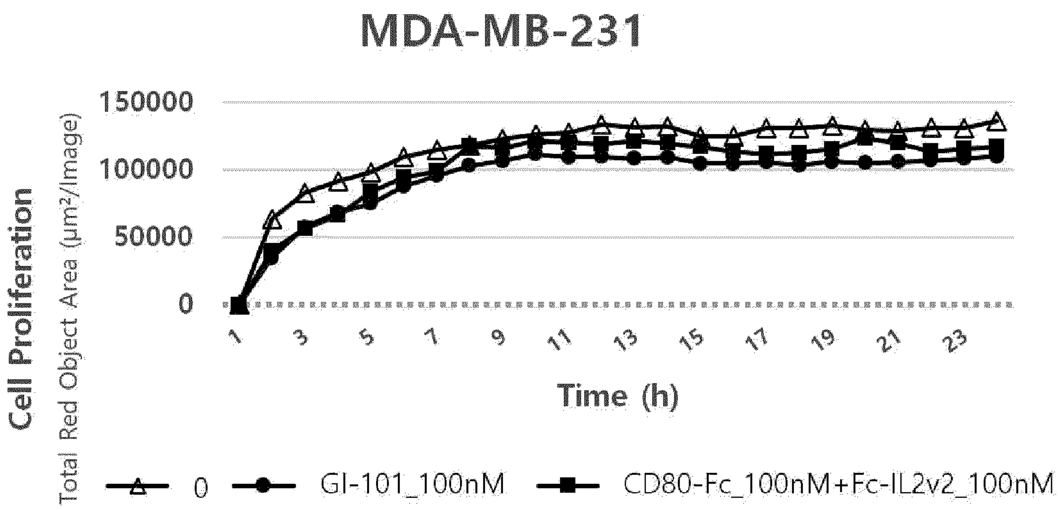
MDA-MB-231
150000
100000
50000
0
Cell Proliferation
Total Red Object Area (µm²/Image)
1
3
5
7
9
11
13
15
17
19
21
23
Time (h)
0
GI-101_100nM
CD80-Fc_100nM+Fc-IL2v2_100nM

FIG.10

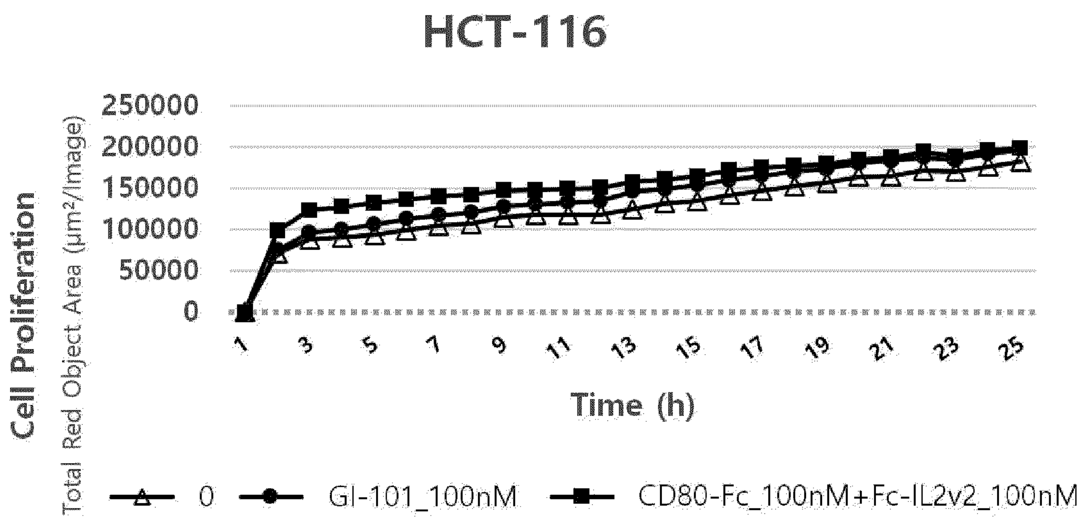
HCT-116
250000
200000
150000
100000
50000
0
Cell Proliferation
Total Red Object Area (µm²/Image)
1
3
5
7
9
11
13
15
17
19
21
23
25
Time (h)
0
GI-101_100nM
CD80-Fc_100nM+Fc-IL2v2_100nM

FIG.11

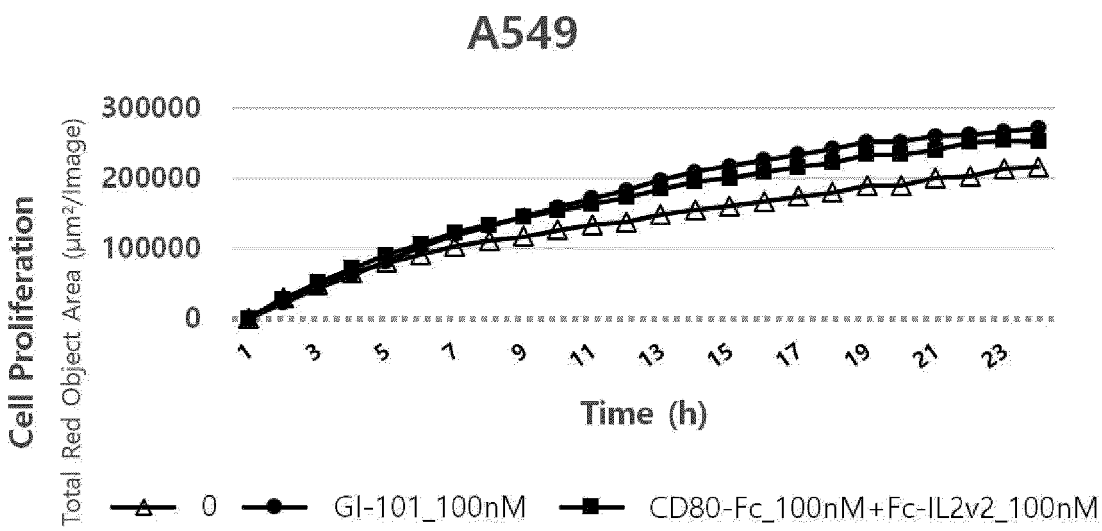
A549
Cell Proliferation
Total Red Object Area (μm²/Image)
300000
200000
100000
0
1
3
5
7
9
11
13
15
17
19
21
23
Time (h)
0
GI-101_100nM
CD80-Fc_100nM+Fc-IL2v2_100nM

FIG.12

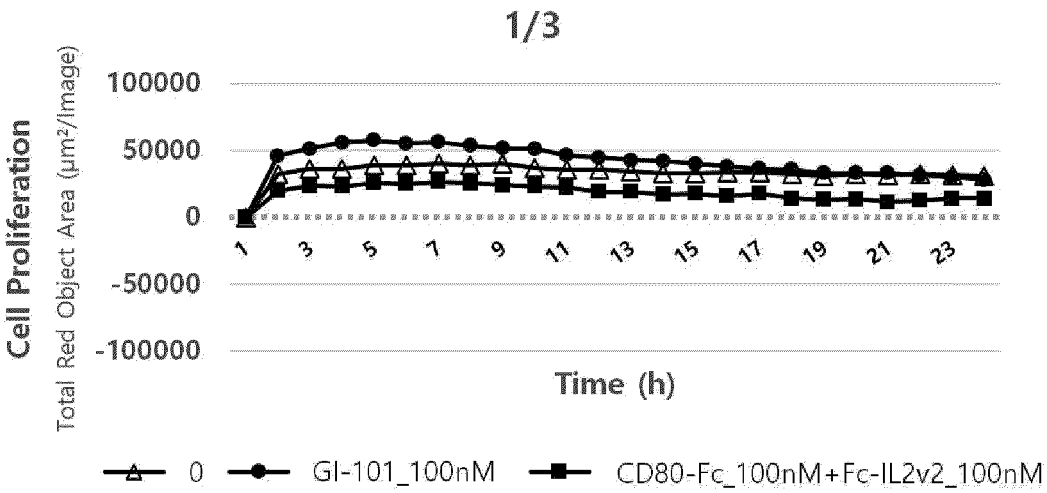
1/3
Cell Proliferation
Total Red Object Area (μm²/Image)
100000
50000
0
-50000
-100000
1
3
5
7
9
11
13
15
17
19
21
23
Time (h)
0
GI-101_100nM
CD80-Fc_100nM+Fc-IL2v2_100nM

FIG.13

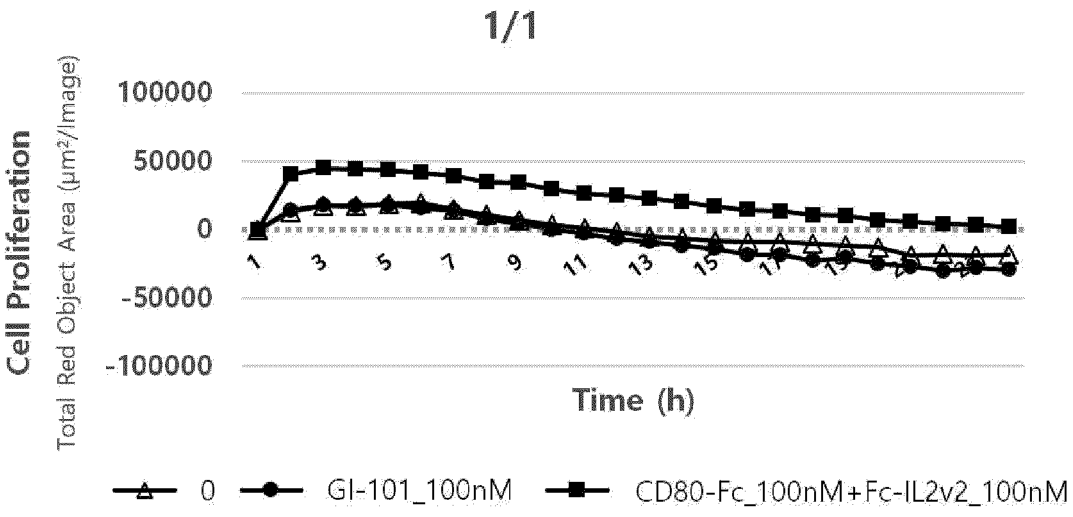
1/1
Cell Proliferation
Total Red Object Area (μm²/Image)
100000
50000
0
-50000
-100000
1
3
5
7
9
11
13
15
17
Time (h)
0
GI-101_100nM
CD80-Fc_100nM+Fc-IL2v2_100nM

FIG.14

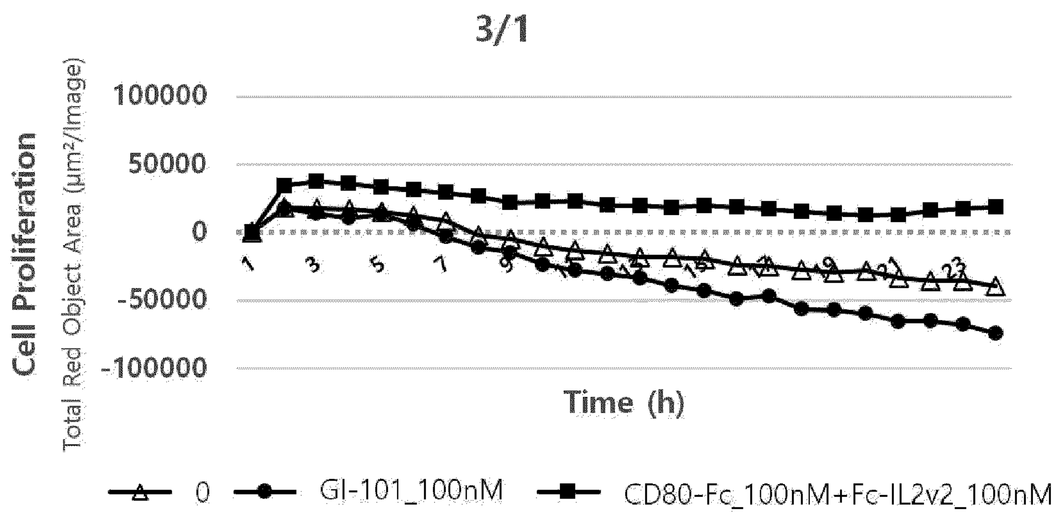
3/1
Cell Proliferation
Total Red Object Area (μm²/Image)
100000
50000
0
-50000
-100000
1
3
5
7
9
23
Time (h)
0
GI-101_100nM
CD80-Fc_100nM+Fc-IL2v2_100nM

FIG.15

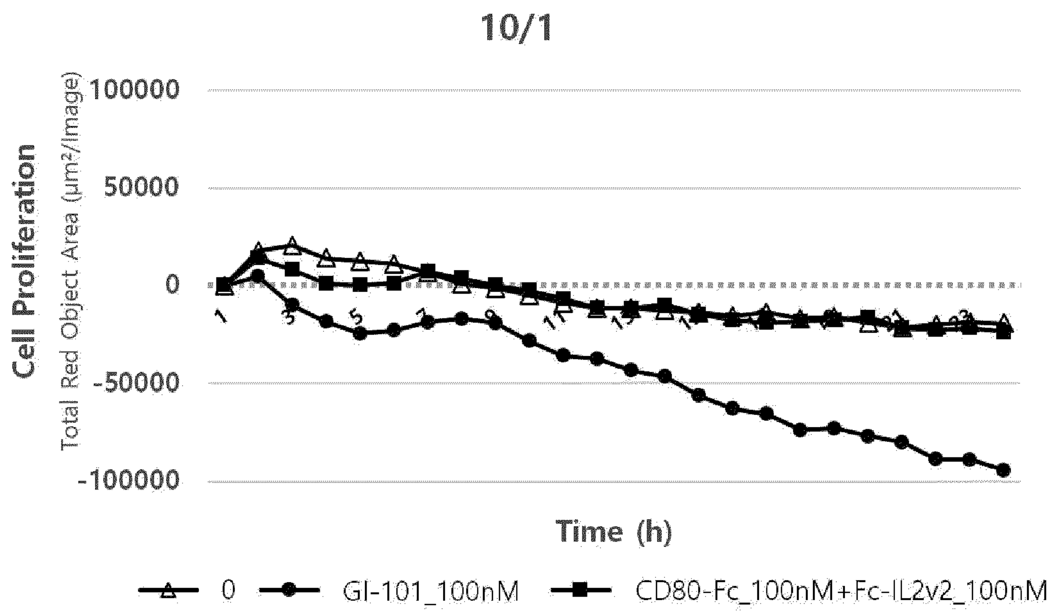
10/1
Cell Proliferation
Total Red Object Area (μm²/Image)
100000
50000
0
-50000
-100000
1
3
5
7
9
11
13
Time (h)
0
GI-101_100nM
CD80-Fc_100nM+Fc-IL2v2_100nM

FIG.16

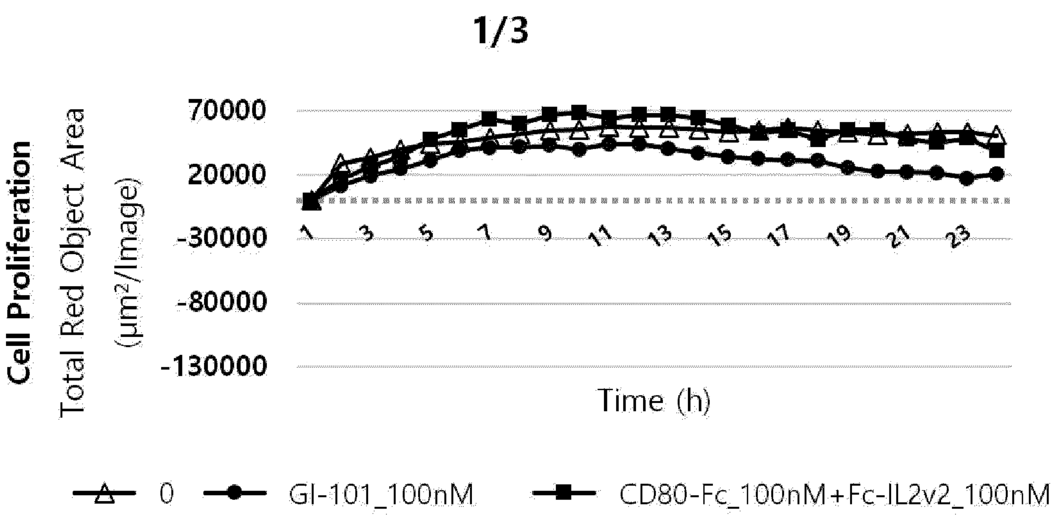
1/3
Cell Proliferation
Total Red Object Area
(μm²/Image)
70000
20000
-30000
-80000
-130000
1
3
5
7
9
11
13
15
17
19
21
23
Time (h)
0
GI-101_100nM
CD80-Fc_100nM+Fc-IL2v2_100nM

FIG.17

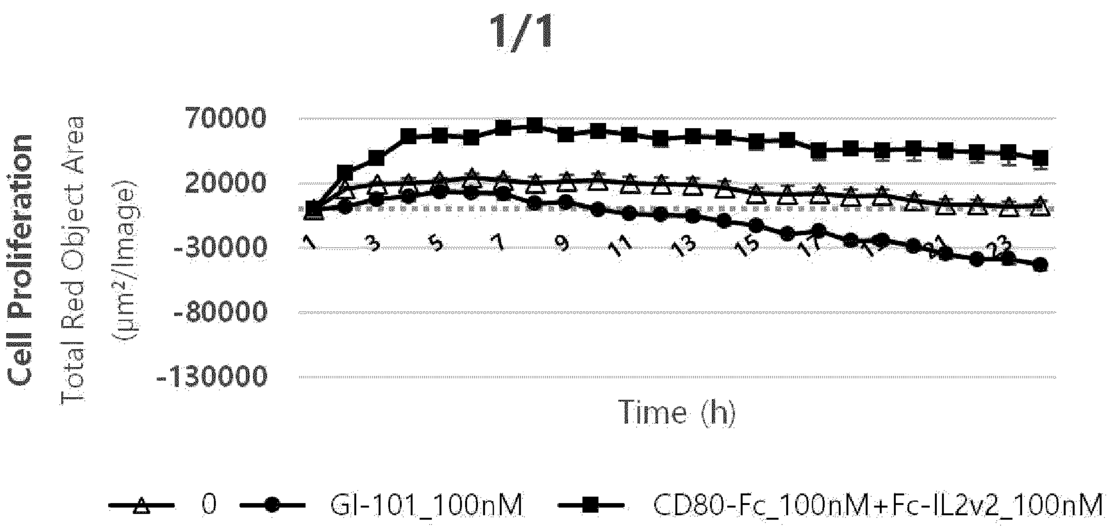
1/1
Cell Proliferation
Total Red Object Area (µm²/Image)
70000
20000
-30000
-80000
-130000
1
3
5
7
9
11
13
15
17
19
21
23
Time (h)
0
GI-101_100nM
CD80-Fc_100nM+Fc-IL2v2_100nM

FIG.18

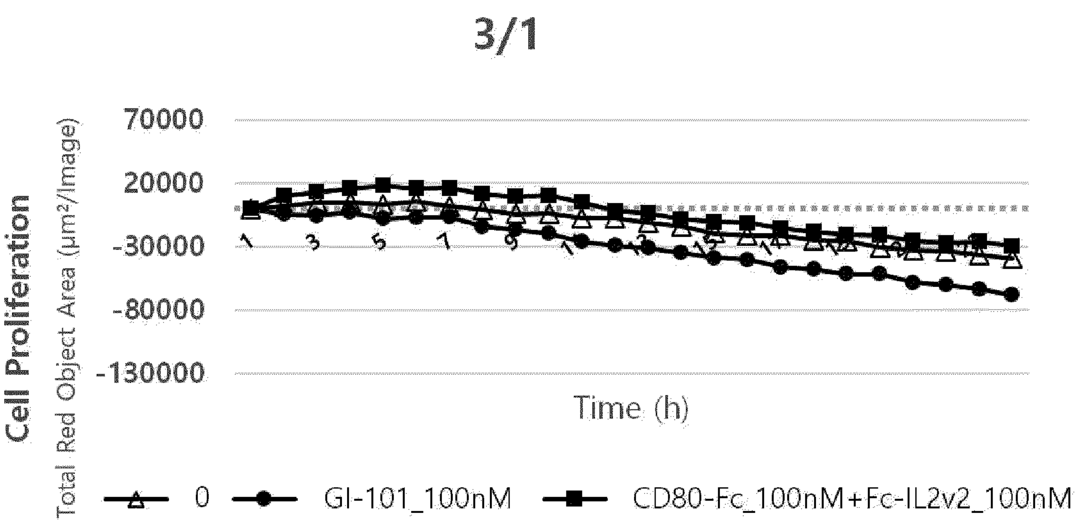
3/1
Cell Proliferation
Total Red Object Area (µm²/Image)
70000
20000
-30000
-80000
-130000
1
3
5
7
9
Time (h)
0
GI-101_100nM
CD80-Fc_100nM+Fc-IL2v2_100nM

FIG.19

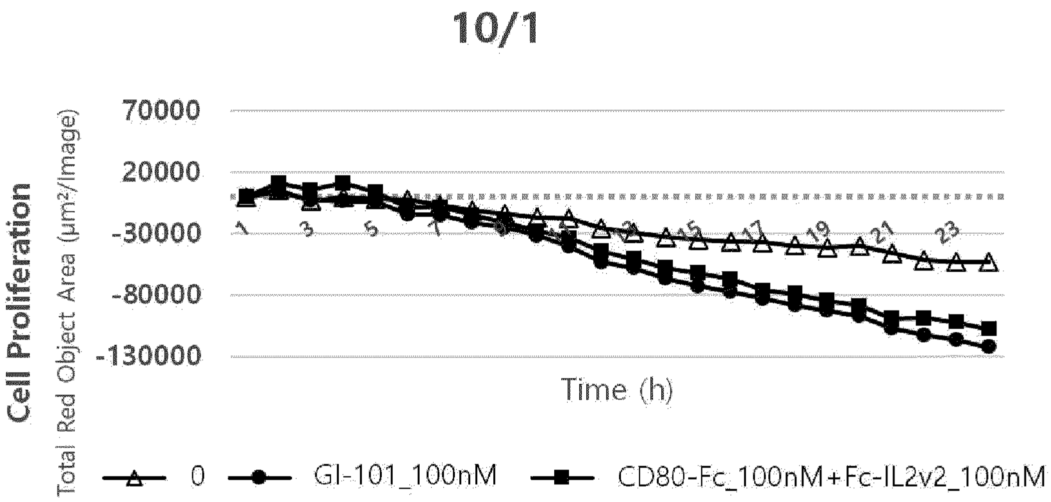
10/1
Cell Proliferation
Total Red Object Area (µm²/Image)
70000
20000
-30000
-80000
-130000
1
3
5
7
9
11
13
15
17
19
21
23
Time (h)
0
GI-101_100nM
CD80-Fc_100nM+Fc-IL2v2_100nM

FIG.20

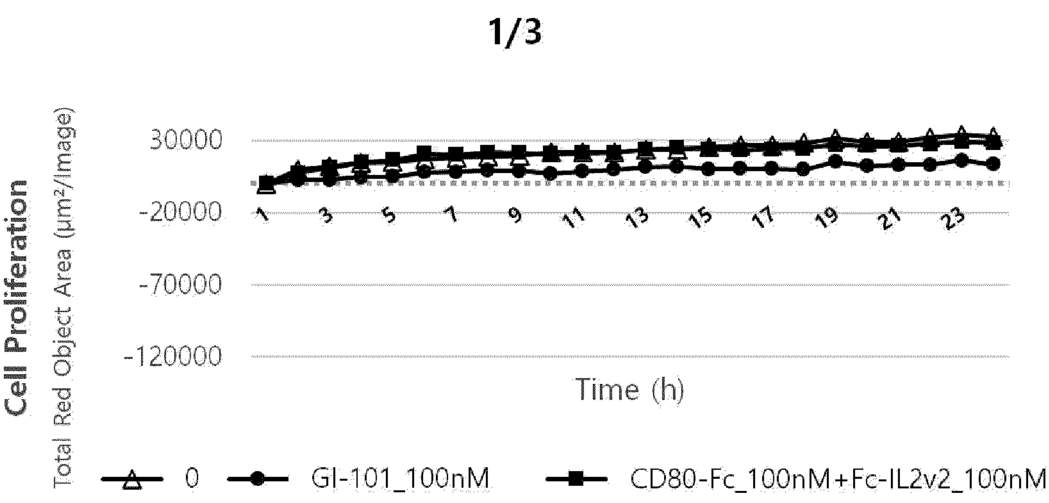
1/3
Cell Proliferation
Total Red Object Area (µm²/Image)
30000
-20000
-70000
-120000
1
3
5
7
9
11
13
15
17
19
21
23
Time (h)
0
GI-101_100nM
CD80-Fc_100nM+Fc-IL2v2_100nM

FIG.21

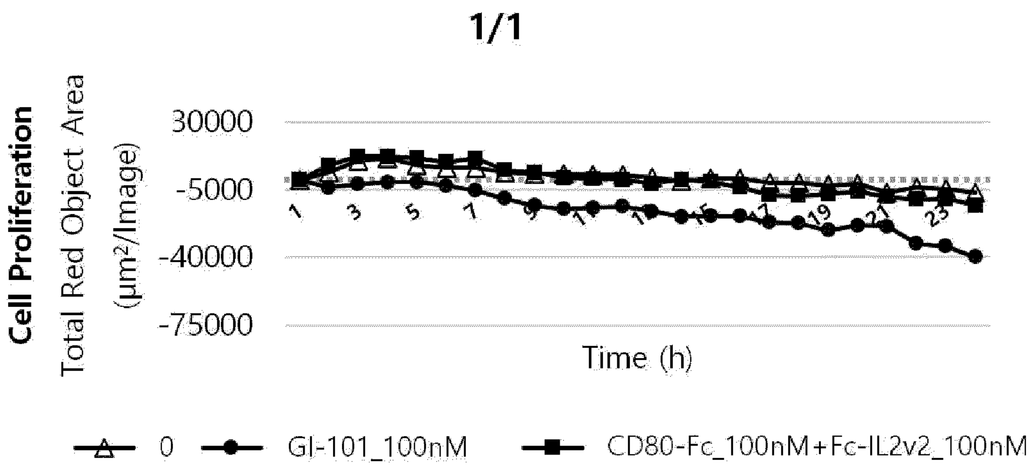
1/1
Cell Proliferation
Total Red Object Area
(μm²/Image)
30000
-5000
-40000
-75000
1
3
5
7
9
23
Time (h)
0
GI-101_100nM
CD80-Fc_100nM+Fc-IL2v2_100nM

FIG.22

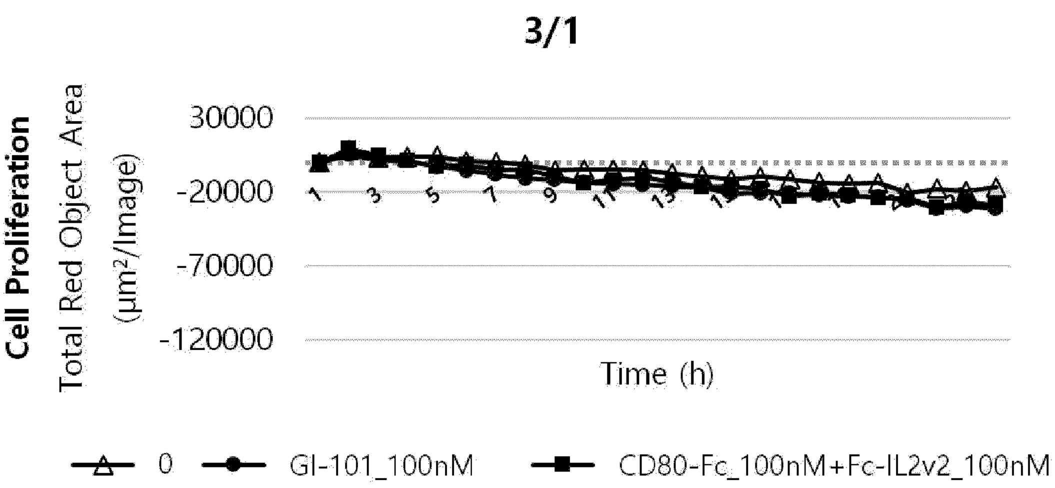
3/1
Cell Proliferation
Total Red Object Area
(μm²/Image)
30000
-20000
-70000
-120000
1
3
5
7
9
Time (h)
0
GI-101_100nM
CD80-Fc_100nM+Fc-IL2v2_100nM

FIG.23

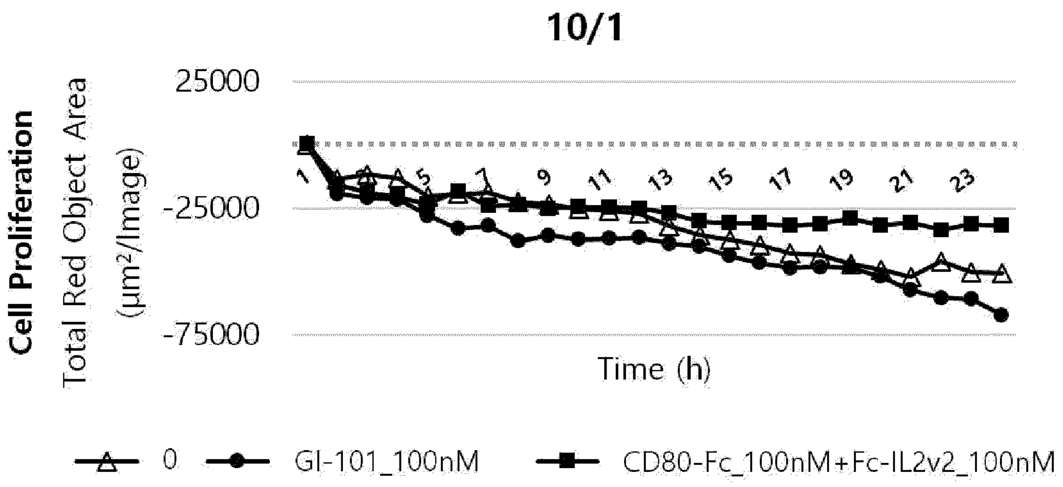
10/1
Cell Proliferation
Total Red Object Area
(μm²/Image)
25000
-25000
-75000
1
3
5
7
9
11
13
15
17
19
21
23
Time (h)
0
GI-101_100nM
CD80-Fc_100nM+Fc-IL2v2_100nM

FIG.24

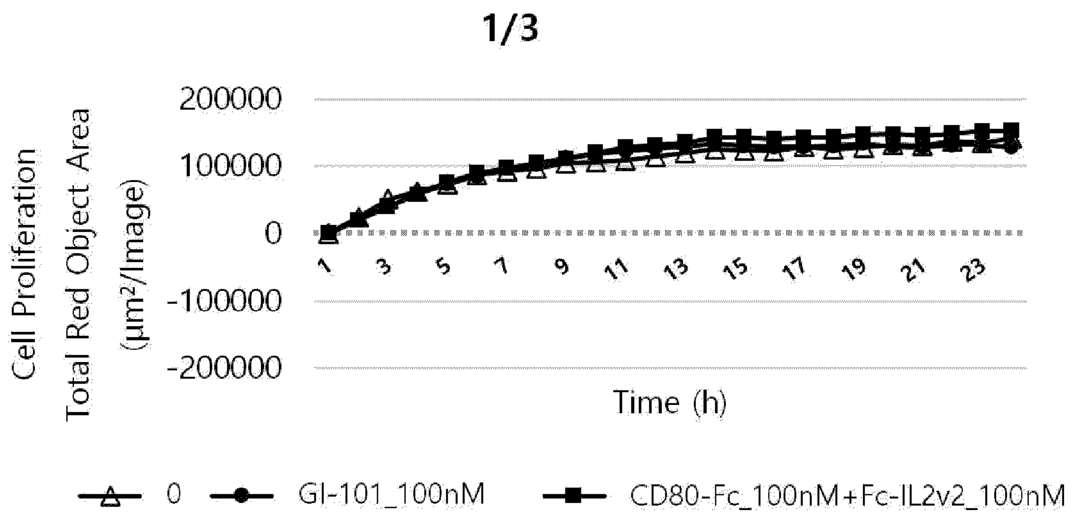
1/3
Cell Proliferation
Total Red Object Area
(μm²/Image)
200000
100000
0
-100000
-200000
1
3
5
7
9
11
13
15
17
19
21
23
Time (h)
0
GI-101_100nM
CD80-Fc_100nM+Fc-IL2v2_100nM

FIG.25

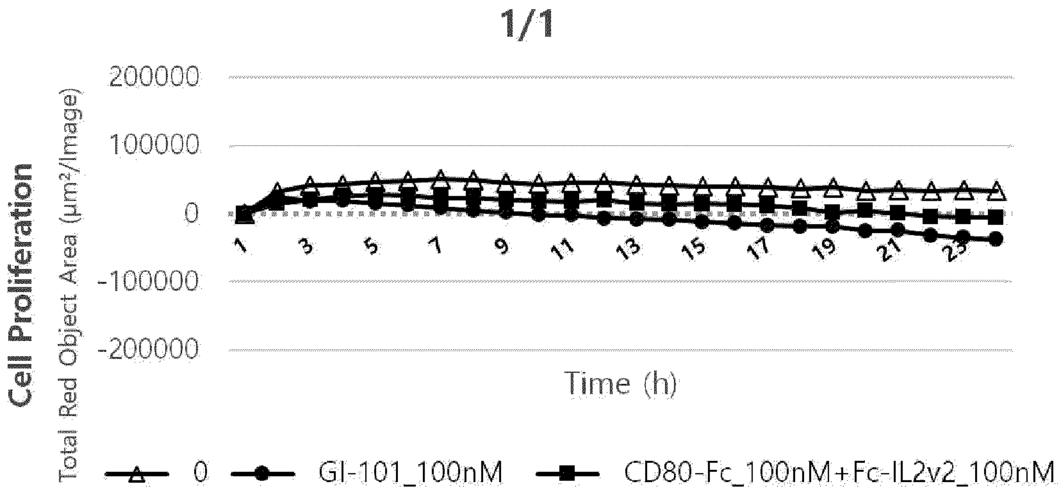
1/1
200000
100000
0
-100000
-200000
Cell Proliferation
Total Red Object Area (µm²/Image)
1
3
5
7
9
11
13
15
17
19
21
23
Time (h)
0
GI-101_100nM
CD80-Fc_100nM+Fc-IL2v2_100nM

FIG.26

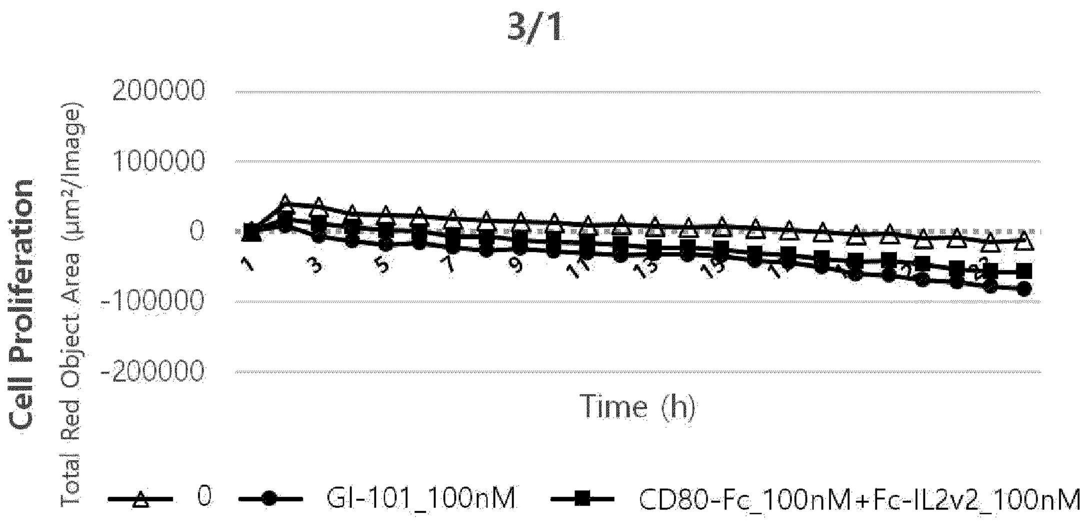
3/1
200000
100000
0
-100000
-200000
Cell Proliferation
Total Red Object Area (µm²/Image)
Time (h)
0
GI-101_100nM
CD80-Fc_100nM+Fc-IL2v2_100nM

FIG.27

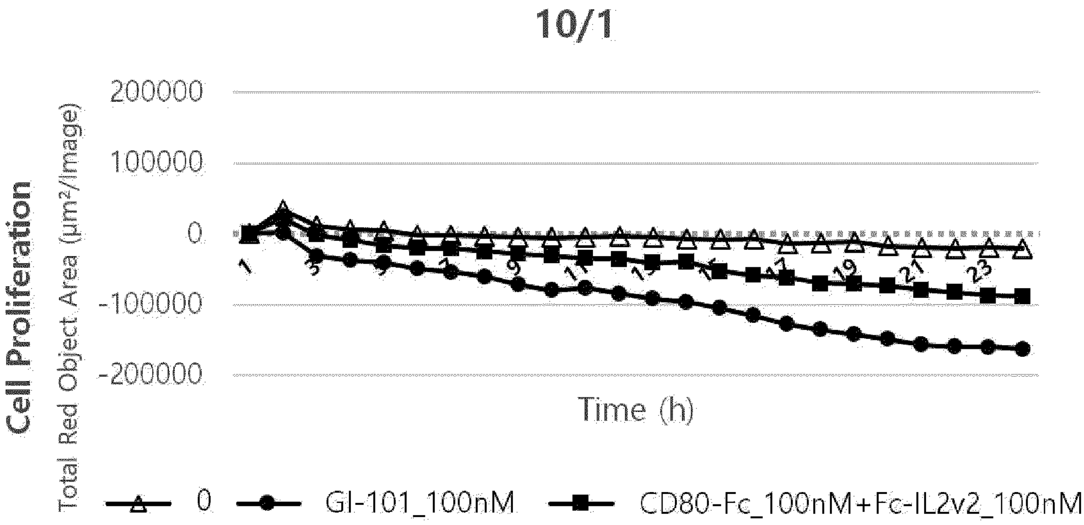
10/1
Cell Proliferation
Total Red Object Area (μm²/Image)
200000
100000
0
-100000
-200000
Time (h)
1
3
5
7
9
11
13
15
17
19
21
23
0
GI-101_100nM
CD80-Fc_100nM+Fc-IL2v2_100nM

FIG.28

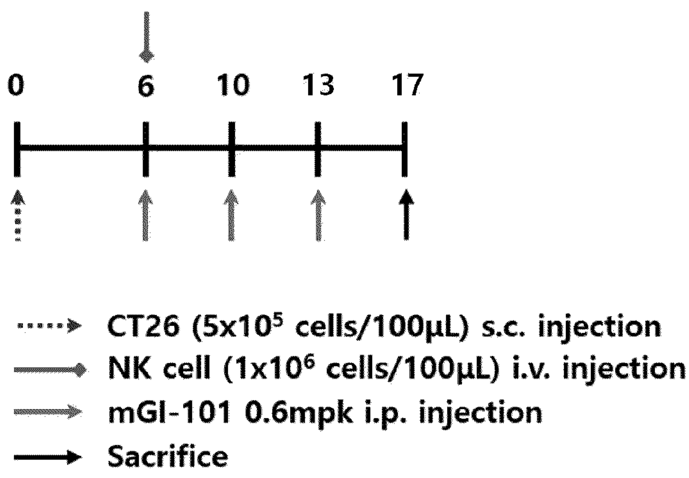
0
6
10
13
17
CT26 (5x10⁵ cells/100μL) s.c. injection
NK cell (1x10⁶ cells/100μL) i.v. injection
mGI-101 0.6mpk i.p. injection
Sacrifice

FIG.29

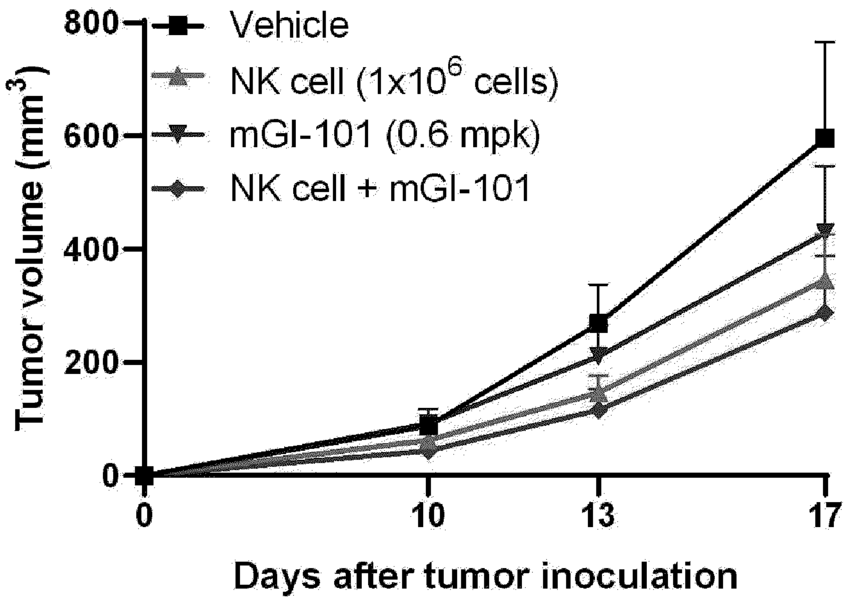
Vehicle
NK cell ($1x10^6$ cells)
mGI-101 (0.6 mpk)
NK cell + mGI-101
Tumor volume ($mm^3$)
800
600
400
200
0
0
10
13
17
Days after tumor inoculation

FIG.30

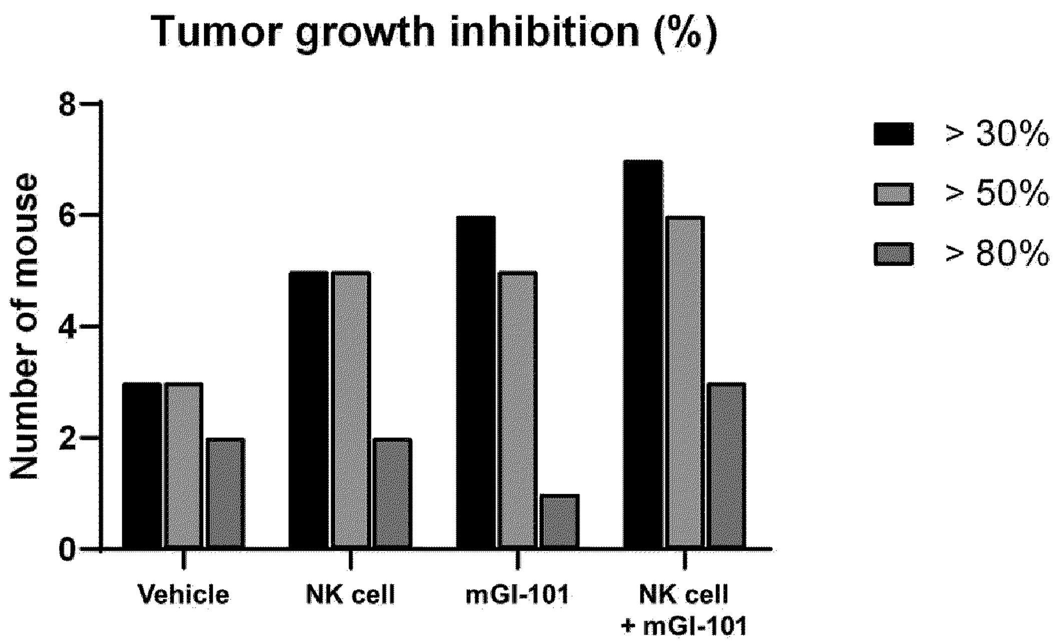
Tumor growth inhibition (%)
Number of mouse
8
6
4
2
0
Vehicle
NK cell
mGI-101
NK cell + mGI-101
> 30%
> 50%
> 80%

FIG.31

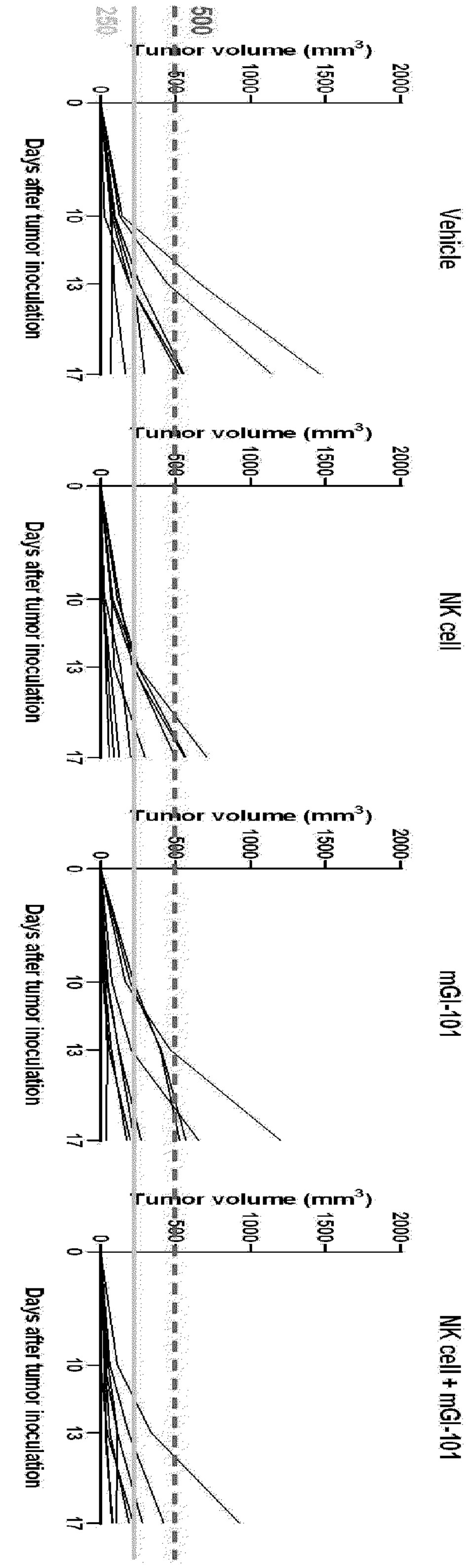
250
500
Vehicle
NK cell
mGI-101
NK cell + mGI-101
Tumor volume (mm3)
Days after tumor inoculation
0
10
13
17
500
1000
1500
2000

FIG.32

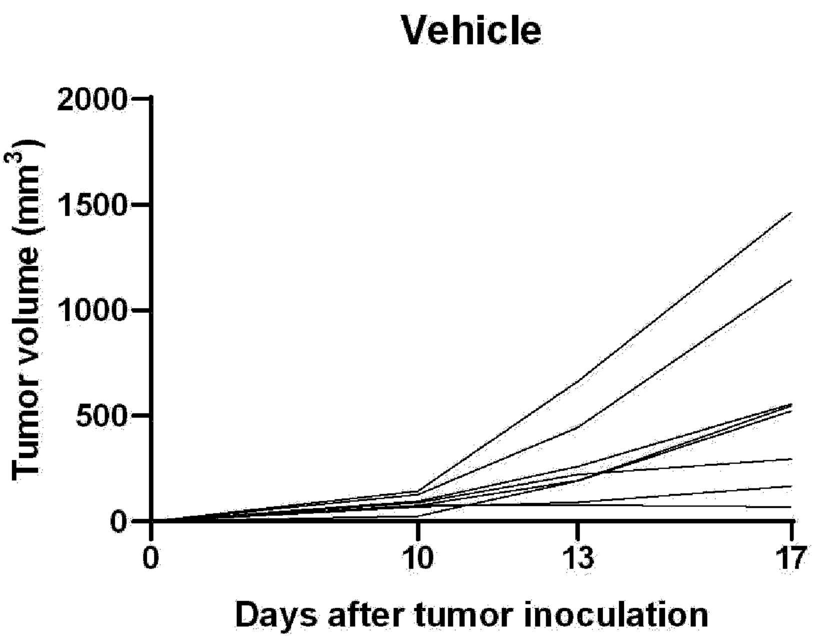
Vehicle
Tumor volume (mm$^3$)
2000
1500
1000
500
0
0
10
13
17
Days after tumor inoculation

FIG.33

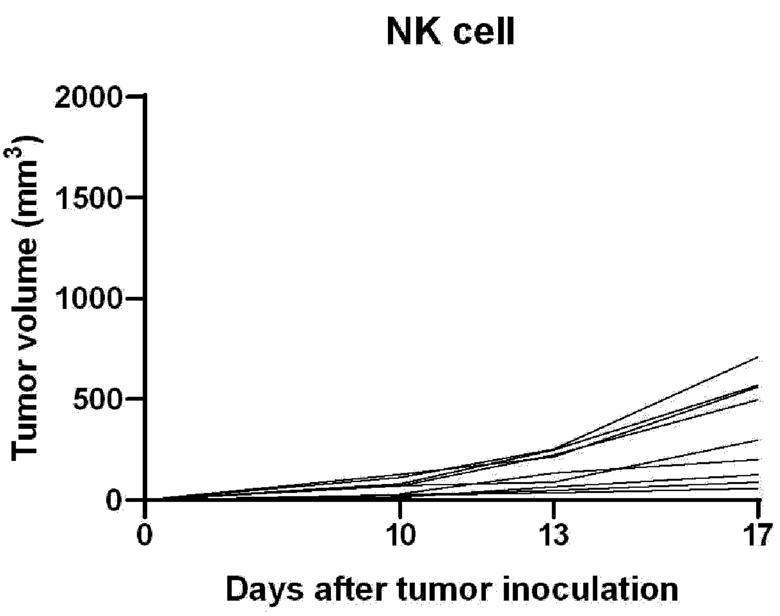
NK cell
Tumor volume (mm$^3$)
2000
1500
1000
500
0
0
10
13
17
Days after tumor inoculation

FIG.34

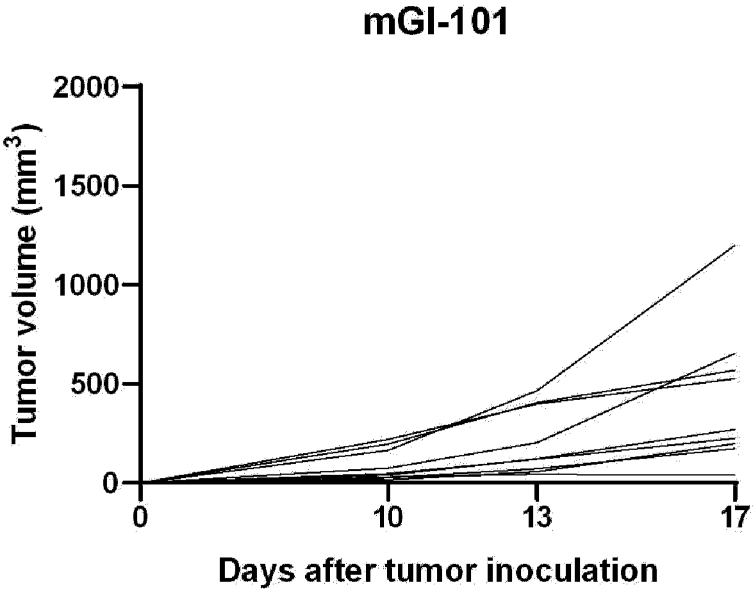
mGI-101
2000
1500
1000
500
0
Tumor volume (mm3)
0
10
13
17
Days after tumor inoculation

FIG.35

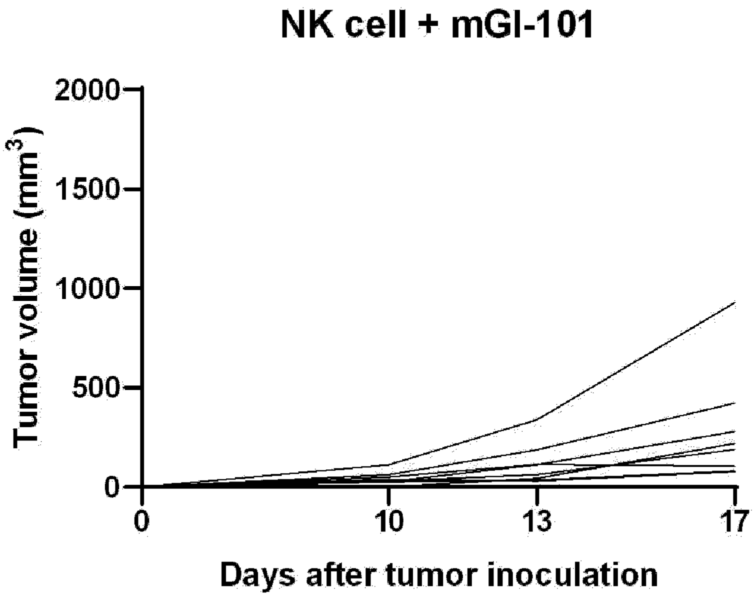
NK cell + mGI-101
2000
1500
1000
500
0
Tumor volume (mm3)
0
10
13
17
Days after tumor inoculation

## INTERNATIONAL SEARCH REPORT

International application No.
**PCT/KR2020/016949**

**A. CLASSIFICATION OF SUBJECT MATTER**

**C12N 5/0783**(2010.01)i; **C12N 15/63**(2006.01)i; **C07K 14/55**(2006.01)i; **C07K 14/705**(2006.01)i; **A61K 35/17**(2014.01)i; **A61P 35/00**(2006.01)i; **A61K 38/00**(2006.01)i; **A61K 38/20**(2006.01)i; **A61K 38/17**(2006.01)i

According to International Patent Classification (IPC) or to both national classification and IPC

**B. FIELDS SEARCHED**

Minimum documentation searched (classification system followed by classification symbols)

C12N 5/0783(2010.01); A61K 31/7088(2006.01); A61K 38/00(2006.01); A61K 38/20(2006.01); A61K 39/395(2006.01); C07K 14/55(2006.01); C07K 14/705(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: IL-2, CD80, 융합단백질(fusion protein), 변이(mutation), NK 세포(NK cell), 암 (cancer)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | CHAN, L. et al. IL-2/B7.1 (CD80) Fusagene Transduction of AML Blasts by a Self-Inactivating Lentiviral Vector Stimulates T Cell Responses in Vitro: a Strategy to Generate Whole Cell Vaccines for AML. MOLECULAR THERAPY. 2005, vol. 11, no. 1, pp. 120-131.<br>See abstract; pages 120-121; and figure 1. | 1,21-22<br>2-19<br>20 |
| Y | WO 2017-220989 A1 (KYMAB LIMITED) 28 December 2017 (2017-12-28)<br>See claims 1, 38-39, 41 and 45; and pages 6, 154 and 157. | 2-8,12-19 |
| Y | KR 10-2018-0069903 A (FIVE PRIME THERAPEUTICS, INC.) 25 June 2018 (2018-06-25)<br>See claims 1 and 25-27; paragraph [0042]; and SEQ ID NOs. 1 and 5. | 9-11 |
| Y | WO 2018-184964 A1 (F. HOFFMANN-LA ROCHE AG) 11 October 2018 (2018-10-11)<br>See claims 1-13, 17-18 and 23-32; pages 80-81 and 84; and the drawings. | 2-8,12-19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 April 2021** | **27 April 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No.
PCT/KR2020/016949

C. DOCUMENTS CONSIDERED TO BE RELEVANT

| Category* | Citation of document, with indication, where appropriate, of the relevant passages | Relevant to claim No. |
|---|---|---|
| A | US 2013-0149305 A1 (OSTRAND-ROSENBERG, S.) 13 June 2013 (2013-06-13) See entire document. | 1-22 |

Form PCT/ISA/210 (second sheet) (July 2019)

**INTERNATIONAL SEARCH REPORT**

International application No. **PCT/KR2020/016949**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**
**Information on patent family members**

International application No.
**PCT/KR2020/016949**

| Patent document cited in search report | Publication date (day/month/year) | Patent family member(s) | Publication date (day/month/year) |
|---|---|---|---|
| WO 2017-220989 A1 | 28 December 2017 | AU 2017-281830 A1 | 13 December 2018 |
| | | BR 112018076260 A2 | 26 March 2019 |
| | | CA 3026474 A1 | 28 December 2017 |
| | | CA 3026477 A1 | 28 December 2017 |
| | | CN 109475602 A | 15 March 2019 |
| | | CN 109475603 A | 15 March 2019 |
| | | EP 3292967 A1 | 14 March 2018 |
| | | EP 3292967 B1 | 28 October 2020 |
| | | EP 3471753 A1 | 24 April 2019 |
| | | EP 3471754 A1 | 24 April 2019 |
| | | EP 3497128 A2 | 19 June 2019 |
| | | EP 3559041 A1 | 30 October 2019 |
| | | JP 2019-528083 A | 10 October 2019 |
| | | JP 2019-534892 A | 05 December 2019 |
| | | JP 2020-502198 A | 23 January 2020 |
| | | JP 2020-511932 A | 23 April 2020 |
| | | KR 10-2019-0030208 A | 21 March 2019 |
| | | KR 10-2019-0032355 A | 27 March 2019 |
| | | KR 10-2019-0038618 A | 08 April 2019 |
| | | MX 2018016364 A | 28 November 2019 |
| | | RU 2018147413 A | 21 July 2020 |
| | | SG 11201810509 A | 28 December 2018 |
| | | TW 201803904 A | 01 February 2018 |
| | | TW 201803905 A | 01 February 2018 |
| | | TW 201811820 A | 01 April 2018 |
| | | TW 201811826 A | 01 April 2018 |
| | | TW 201900680 A | 01 January 2019 |
| | | TW I640536 B | 11 November 2018 |
| | | US 10604576 B2 | 31 March 2020 |
| | | US 2017-0362321 A1 | 21 December 2017 |
| | | US 2018-0066058 A1 | 08 March 2018 |
| | | US 2019-0202917 A1 | 04 July 2019 |
| | | US 2019-0330345 A1 | 31 October 2019 |
| | | US 2019-0330351 A1 | 31 October 2019 |
| | | US 2019-0338032 A1 | 07 November 2019 |
| | | US 2020-0190191 A1 | 18 June 2020 |
| | | US 9567399 B1 | 14 February 2017 |
| | | US 9617338 B1 | 11 April 2017 |
| | | US 9957323 B2 | 01 May 2018 |
| | | WO 2017-220988 A1 | 28 December 2017 |
| | | WO 2017-220990 A1 | 28 December 2017 |
| | | WO 2018-029474 A2 | 15 February 2018 |
| | | WO 2018-029474 A3 | 22 March 2018 |
| | | WO 2018-115859 A1 | 28 June 2018 |
| KR 10-2018-0069903 A | 25 June 2018 | AU 2016-350701 A1 | 31 May 2018 |
| | | BR 112018008865 A2 | 06 November 2018 |
| | | CA 3001131 A1 | 11 May 2017 |
| | | CL 2018001172 A1 | 21 September 2018 |
| | | CN 108884139 A | 23 November 2018 |
| | | CO 2018004132 A2 | 30 November 2018 |

Form PCT/ISA/210 (patent family annex) (July 2019)

**INTERNATIONAL SEARCH REPORT**
**Information on patent family members**

International application No.
**PCT/KR2020/016949**

| Patent document cited in search report | Publication date (day/month/year) | Patent family member(s) | Publication date (day/month/year) |
|---|---|---|---|
| | | CR 20180306 A | 16 October 2018 |
| | | EA 201891106 A1 | 28 December 2018 |
| | | EC SP18041501 A | 30 June 2018 |
| | | EP 3371208 A1 | 12 September 2018 |
| | | IL 258725 A | 28 June 2018 |
| | | JP 2018-535204 A | 29 November 2018 |
| | | MX 2018005517 A | 09 November 2018 |
| | | PE 13002018 A1 | 09 August 2018 |
| | | PE 20181300 A1 | 09 August 2018 |
| | | PH 12018500933 A1 | 19 November 2018 |
| | | SG 11201803009 A | 30 May 2018 |
| | | TW 201722985 A | 01 July 2017 |
| | | US 10273281 B2 | 30 April 2019 |
| | | US 2017-0145071 A1 | 25 May 2017 |
| | | US 2019-0194288 A1 | 27 June 2019 |
| | | US 2019-0202889 A1 | 04 July 2019 |
| | | WO 2017-079117 A1 | 11 May 2017 |
| WO 2018-184964 A1 | 11 October 2018 | AR 111400 A1 | 10 July 2019 |
| | | AU 2018-247765 A1 | 22 August 2019 |
| | | BR 112019017329 A2 | 14 April 2020 |
| | | CA 3053357 A1 | 11 October 2018 |
| | | CL 2019002346 A1 | 10 January 2020 |
| | | CN 110392692 A | 29 October 2019 |
| | | CO 2019009354 A2 | 09 September 2019 |
| | | CR 20190426 A | 01 November 2019 |
| | | EP 3606946 A1 | 12 February 2020 |
| | | JP 2020-515275 A | 28 May 2020 |
| | | KR 10-2019-0121816 A | 28 October 2019 |
| | | MX 2019011770 A | 09 January 2020 |
| | | PE 20191494 A1 | 21 October 2019 |
| | | SG 11201909205 A | 28 November 2019 |
| | | TW 201900220 A | 01 January 2019 |
| | | US 2018-0326010 A1 | 15 November 2018 |
| US 2013-0149305 A1 | 13 June 2013 | US 10377810 B2 | 13 August 2019 |
| | | US 2015-0232532 A1 | 20 August 2015 |
| | | US 2017-0226181 A1 | 10 August 2017 |
| | | US 8956619 B2 | 17 February 2015 |
| | | US 9650429 B2 | 16 May 2017 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5229109 A
- KR 1667096


### Non-patent literature cited in the description


- **LORIS ZAMAI.** *J. Immunol.,* 2007, vol. 178, 4011-4016
- Remington's Pharmaceutical Sciences. 1995